# EUROPEAN PATENT APPLICATION

(11) **EP 4 692 327 A1**
(43) Date of publication of application: **11.02.2026**
(21) Application number: 24780653.2
(22) Date of filing: 28.03.2024
(51) Int. Cl.: C12N 5/0783, A61K 35/17, A61P 1/04, A61P 1/16, A61P 1/18, A61P 3/10, A61P 7/06, A61P 9/00, A61P 9/12, A61P 13/12, A61P 15/00, A61P 17/00, A61P 17/06, A61P 19/02, A61P 19/08, A61P 21/04, A61P 25/00, A61P 25/28, A61P 27/06, A61P 29/00, A61P 31/00

(54) **HUMAN INDUCIBLE REGULATORY T CELLS AND METHOD FOR PRODUCING SAME, AND PHARMACEUTICAL COMPOSITION FOR TREATING OR PREVENTING T CELL-RELATED DISEASE**

(30) Priority: 29.03.2023 JP 2023053538
(71) Applicant: Regcell Co., Ltd., Kyoto-shi, Kyoto 603-8436 (JP); The University of Osaka, Osaka 565-0871 (JP)
(72) Inventor: MIKAMI, Norihisa, Kyoto-shi, Kyoto 603-8436 (JP); SAKAGUCHI, Shimon, Suita-shi, Osaka 565-0871 (JP)
(74) Representative: Vossius & Partner Patentanwälte Rechtsanwälte mbB
(86) International application number: PCT/JP2024/012680
(87) International publication number: WO 2024/204553

(57) **Abstract**

The present disclosure provides inducible regulatory T cells having high functionality and a stable immunosuppressive function. The present disclosure provides inducible regulatory human T cells having at least one characteristic selected from the group consisting of FoxP3-positive, CTLA4-positive, NT5E-positive, ITGAE (CD103)-positive, AREG-positive, CD172g-positive, and CD26-positive, and a cell population containing the same. The present disclosure also provides a composition and a pharmaceutical composition containing such cells, and methods using these compositions.

## Description

### [Technical Field]

The present disclosure relates to human inducible regulatory T cells having high functionality, a method for producing the same, and a pharmaceutical composition for treating or preventing T cell-related diseases, the pharmaceutical composition containing the cells.

### [Background Art]

As an important characteristic of CD25-positive CD4-positive regulatory T cells inherent in the immune system, the cells specifically express the transcription factor FoxP3, and a deficiency or mutation of FoxP3 may impair the development, differentiation, or suppressive function of the regulatory T cells. The regulatory T cells suppress the immune system by expression of various genes such as CTLA4 and IL-10 in addition to FoxP3. It is considered that epigenetic states such as DNA demethylation contribute to comprehensive gene expression regulation of regulatory T cells, including stable expression of FoxP3, and these states correlate with the functional phenotype of regulatory T cells.

### [Summary of Invention]

### [Solution to Problem]

The present inventors have found for the first time that when regulatory T cells are induced from human peripheral T cells, stable induced T cells are induced from the human peripheral T cells by stimulation using an anti-CD3 antibody, a resting culture is performed, a culture is performed again with anti-CD3 antibody stimulation, and then, a resting culture is performed again, such that regulatory T cells having high expression of suppressive molecules and a high suppressive function can be induced. In addition, the present inventors have also found that the inducible regulatory T cells thus obtained have sufficient immunosuppressive ability to function as a medicament.

Accordingly, the present disclosure provides the following.

### (Item X1)

An inducible regulatory human T cell having at least one characteristic selected from the group consisting of FoxP3-positive, CTLA4-positive, NT5E-positive, ITGAE (CD103)-positive, AREG-positive, CD172g-positive, and CD26-positive.

### (Item X1A)

An NT5E-positive inducible regulatory human T cell.

### (Item X1B)

An ITGAE (CD103)-positive inducible regulatory human T cell.

### (Item X1C)

An AREG-positive inducible regulatory human T cell.

### (Item X1D)

A CD172g-positive inducible regulatory human T cell.

### (Item X1E)

A CD26-positive inducible regulatory human T cell.

### (Item X1F)

A CTLA4-positive inducible regulatory human T cell.

### (Item X1G)

A stably FoxP3-positive inducible regulatory human T cell.

### (Item X1G1)

An inducible regulatory human T cell having at least two characteristics selected from the group consisting of CTLA4-positive, NT5E-positive, ITGAE (CD103)-positive, AREG-positive, CD172g-positive, and CD26-positive.

### (Item X1G2)

An inducible regulatory human T cell having at least three characteristics selected from the group consisting of CTLA4-positive, NT5E-positive, ITGAE (CD103)-positive, AREG-positive, CD172g-positive, and CD26-positive.

### (Item X1G3)

An inducible regulatory human T cell having at least four characteristics selected from the group consisting of CTLA4-positive, NT5E-positive, ITGAE (CD103)-positive, AREG-positive, CD172g-positive, and CD26-positive.

### (Item X1G4)

An inducible regulatory human T cell having at least five characteristics selected from the group consisting of CTLA4-positive, NT5E-positive, ITGAE (CD103)-positive, AREG-positive, CD172g-positive, and CD26-positive.

### (Item X1G5)

An inducible regulatory human T cell having all of CTLA4-positive, NT5E-positive, ITGAE (CD103)-positive, AREG-positive, CD172g-positive, and CD26-positive characteristics.

### (Item X2)

The inducible regulatory human T cell according to any one of the preceding items, in which the inducible regulatory human T cell is at least CTLA4-positive and FoxP3-positive.

### (Item X2a)

The inducible regulatory human T cell according to any one of the preceding items, in which the FoxP3 is stably expressed.

### (Item X2b)

The inducible regulatory human T cell according to any one of the preceding items, in which the FoxP3 is continuously expressed for at least about 48 hours or longer.

### (Item X2c)

The inducible regulatory human T cell according to any one of the preceding items, in which the FoxP3 is stably expressed at least from day 7 of culture.

### (Item X2d)

The inducible regulatory human T cell according to any one of the preceding items, in which the FoxP3 is expressed even after freeze-thawing of the cell.

### (Item X3)

The inducible regulatory human T cell according to any one of the preceding items, in which the inducible regulatory human T cell is at least CD172g-positive and/or CD26-positive.

### (Item X4)

The inducible regulatory human T cell according to any one of the preceding items, in which the CNS2 region of the FOXP3 gene is demethylated.

### (Item X5)

The inducible regulatory human T cell according to any one of the preceding items, in which the inducible regulatory human T cell is CD4-positive or CD8-positive.

### (Item X6)

The inducible regulatory human T cell according to any one of the preceding items, in which the inducible regulatory human T cell is obtained from or derived from a human peripheral blood T cell or a human tissue-derived T cell.

### (Item X7)

An inducible regulatory human T cell obtained by a method including:
a step (a) of stimulating CD4-positive T cells or CD8-positive T cells in human peripheral blood in a first basal medium for about 1 to about 5 days;
a step (b) of subjecting the cells obtained in the step (a) to a resting culture in a medium containing IL-2 for at least about 1 to about 3 days;
a step (c) of stimulating the cells obtained in the step (b) in a second basal medium for about 1 to about 5 days; and
a step (d) of subjecting the cells obtained in the step (c) to a resting culture in a medium containing IL-2 for at least about 1 to about 3 days.

### (Item X7a)

The inducible regulatory human T cell according to any one of the preceding items, in which the inducible regulatory human T cell is stably FoxP3-positive.

### (Item X8)

A cell population containing the inducible regulatory human T cells according to any one of the preceding items, in which the cell population has a proportion of about 50% or more of cells having at least one characteristic selected from the group consisting of FoxP3-positive, CTLA4-positive, NT5E-positive, ITGAE (CD103)-positive, AREG-positive, CD172g-positive, and CD26-positive.

### (Item X8A)

A cell population containing inducible regulatory human T cells, in which the cell population has a proportion of about 50% or more of NT5E-positive cells.

### (Item X8B)

A cell population containing inducible regulatory human T cells, in which the cell population has a proportion of about 50% or more of ITGAE (CD103)-positive cells.

### (Item X8C)

A cell population containing inducible regulatory human T cells, in which the cell population has a proportion of about 50% or more of AREG-positive cells.

### (Item X8D)

A cell population containing inducible regulatory human T cells, in which the cell population has a proportion of about 50% or more of CD172g-positive cells.

### (Item X8E)

A cell population containing inducible regulatory human T cells, in which the cell population has a proportion of about 50% or more of CD26-positive cells.

### (Item X8F)

A cell population containing inducible regulatory human T cells, in which the cell population has a proportion of about 50% or more of CTLA4-positive cells.

### (Item X8G)

A cell population containing inducible regulatory human T cells, in which the cell population has a proportion of about 50% or more of stably FoxP3-positive cells.

### (Item X8G1)

A cell population containing inducible regulatory human T cells, in which the cell population has a proportion of about 50% or more of cells each having at least two characteristics selected from the group consisting of CTLA4-positive, NT5E-positive, ITGAE (CD103)-positive, AREG-positive, CD172g-positive, and CD26-positive.

### (Item X8G2)

A cell population containing inducible regulatory human T cells, in which the cell population has a proportion of about 50% or more of cells each having at least three characteristics selected from the group consisting of CTLA4-positive, NT5E-positive, ITGAE (CD103)-positive, AREG-positive, CD172g-positive, and CD26-positive.

### (Item X8G3)

A cell population containing inducible regulatory human T cells, in which the cell population has a proportion of about 50% or more of cells each having at least four characteristics selected from the group consisting of CTLA4-positive, NT5E-positive, ITGAE (CD103)-positive, AREG-positive, CD172g-positive, and CD26-positive.

### (Item X8G4)

A cell population containing inducible regulatory human T cells, in which the cell population has a proportion of about 50% or more of cells each having at least five characteristics selected from the group consisting of CTLA4-positive, NT5E-positive, ITGAE (CD103)-positive, AREG-positive, CD172g-positive, and CD26-positive.

### (Item X8G5)

A cell population containing inducible regulatory human T cells, in which the cell population has a proportion of about 50% or more of cells having all of CTLA4-positive, NT5E-positive, ITGAE (CD103)-positive, AREG-positive, CD172g-positive, and CD26-positive characteristics.

### (Item X9)

The cell population according to any one of the preceding items, in which proportions of at least the CTLA4-positive cells and FoxP3-positive cells are each about 50% or more.

### (Item X9a)

The cell population according to any one of the preceding items, in which the FoxP3 is stably expressed.

### (Item X9b)

The cell population according to any one of the preceding items, in which the FoxP3 is continuously expressed for at least about 48 hours or longer.

### (Item X9c)

The cell population according to any one of the preceding items, in which the FoxP3 is stably expressed at least from day 7 of culture.

### (Item X9d)

The cell population according to any one of the preceding items, in which the FoxP3 is expressed even after freeze-thawing of the cell.

### (Item X10)

The cell population according to any one of the preceding items, in which proportions of at least the CD172g-positive cells and/or CD26-positive cells are each about 50% or more.

### (Item X11)

The cell population according to any one of the preceding items, in which a proportion of cells having the at least one characteristic in the cell population is about 60% or more.

### (Item X12)

The cell population according to any one of the preceding items, in which a proportion of cells having the at least one characteristic in the cell population is about 80% or more.

### (Item X12a)

The cell population according to any one of the preceding items, in which a proportion of FoxP3 strong-positive cells is about 50% or more.

### (Item X12b)

The cell population according to any one of the preceding items, in which a proportion of stably FoxP3 strong-positive cells is about 50% or more.

### (Item X13)

The cell population according to any one of the preceding items, in which the cell population contains about 90% or more T cells.

### (Item X14)

A pharmaceutical composition containing the inducible regulatory human T cell according to any one of the preceding items or the cell population according to any one of the preceding items.

### (Item X15)

A regenerative medical material or product containing the inducible regulatory human T cell according to any one of the preceding items or the cell population according to any one of the preceding items.

### (Item X16)

A method for producing inducible regulatory human T cells, the method including:
a step (a) of stimulating CD4-positive T cells or CD8-positive T cells in human peripheral blood in a first basal medium for about 1 to about 5 days;
a step (b) of subjecting the cells obtained in the step (a) to a resting culture in a medium containing IL-2 for at least about 1 to about 3 days;
a step (c) of stimulating the cells obtained in the step (b) in a second basal medium for about 1 to about 5 days; and
a step (d) of subjecting the cells obtained in the step (c) to a resting culture in a medium containing IL-2 for at least about 1 to about 3 days.

### (Item X17-1)

The method according to any one of the preceding items, in which the first basal medium contains an anti-CD3 antibody, TGF-β, IL-2, retinoic acid, and at least one selected from the group consisting of a CDK8 inhibitor, a CDK19 inhibitor, and a CDK8/19 inhibitor.

### (Item X17-2)

The method according to any one of the preceding items, in which the second basal medium contains an anti-CD3 antibody, TGF-β, IL-2, retinoic acid, ascorbic acid, and at least one selected from the group consisting of a CDK8 inhibitor, a CDK19 inhibitor, and a CDK8/19 inhibitor.

### (Item X17-1A)

The method according to any one of the preceding items, in which the first basal medium contains an anti-CD3 antibody, TGF-β, IL-2, retinoic acid, and a CDK8 inhibitor, a CDK19 inhibitor, or a CDK8/19 inhibitor.

### (Item X17-2A)

The method according to any one of the preceding items, in which the second basal medium contains an anti-CD3 antibody, TGF-β, IL-2, retinoic acid, ascorbic acid, and a CDK8 inhibitor, a CDK19 inhibitor, or a CDK8/19 inhibitor.

### (Item X17-3)

The method according to any one of the preceding items, in which at least one selected from the group consisting of a CDK8 inhibitor, a CDK19 inhibitor, and a CDK8/19 inhibitor contained in the first basal medium includes Senexin A or AS2863619.

### (Item X17-4)

The method according to any one of the preceding items, in which at least one selected from the group consisting of a CDK8 inhibitor, a CDK19 inhibitor, and a CDK8/19 inhibitor contained in the second basal medium includes Senexin A or AS2863619.

### (Item X17-5)

The method according to any one of the preceding items, in which TGF-β contained in the first basal medium includes TGF-β1 and/or TGF-β3.

### (Item X17-6)

The method according to any one of the preceding items, in which TGF-β contained in the second basal medium includes TGF-β1 and/or TGF-β3.

### (Item X17-7)

The method according to any one of the preceding items, in which TGF-β contained in the first basal medium includes TGF-β1.

### (Item X17-8)

The method according to any one of the preceding items, in which TGF-β contained in the second basal medium includes TGF-β1.

### (Item X17-9)

The method according to any one of the preceding items, in which the first basal medium contains an anti-CD3 antibody, TGF-β1, IL-2, retinoic acid, and Selexin A.

### (Item X17-10)

The method according to any one of the preceding items, in which the second basal medium contains an anti-CD3 antibody, TGF-β1, IL-2, retinoic acid, Selexin A, and ascorbic acid.

### (Item X17)

The method according to any one of the preceding items, in which the first basal medium contains at least one factor selected from the group consisting of an anti-CD3 antibody, TGF-β, IL-2, retinoic acid, a CDK8 inhibitor, a CDK19 inhibitor, a CDK8/19 inhibitor, and ascorbic acid.

### (Item X18)

The method according to any one of the preceding items, in which the first basal medium contains an anti-CD3 antibody, TGF-β1, IL-2, retinoic acid, a CDK8 inhibitor, a CDK19 inhibitor, a CDK8/19 inhibitor, and ascorbic acid.

### (Item X19)

The method according to any one of the preceding items, in which the second basal medium contains at least one factor selected from the group consisting of an anti-CD3 antibody, TGF-β1, IL-2, retinoic acid, a CDK8 inhibitor, a CDK19 inhibitor, and a CDK8/19 inhibitor.

### (Item X20)

The method according to any one of the preceding items, in which the second basal medium contains an anti-CD3 antibody, TGF-β1, IL-2, retinoic acid, a CDK8 inhibitor, a CDK19 inhibitor, and a CDK8/19 inhibitor.

### (Item X21)

The method according to any one of the preceding items, in which the step (a) includes stimulating the CD4-positive T cells or CD8-positive T cells in the first basal medium for about 3 days.

### (Item X22)

The method according to any one of the preceding items, in which the step (b) includes subjecting the cells obtained in the step (a) to a resting culture in a medium containing the IL-2 for at least about 2 days.

### (Item X23)

The method according to any one of the preceding items, in which the step (c) includes stimulating the cells obtained in the step (b) in the second basal medium for about 3 days.

### (Item X24)

The method according to any one of the preceding items, in which the step (d) includes subjecting the cells obtained in the step (c) to a resting culture in a medium containing the IL-2 for at least about 2 days.

### (Item X25)

An inducible regulatory human T cell or a cell population containing the inducible regulatory human T cells generated by the method according to any one of the preceding items.

In addition, the present disclosure also provides the following.

### (Item 1)

An inducible regulatory T cell having at least one characteristic selected from the group consisting of CTLA4-positive, NT5E-positive, ITGAE (CD103)-positive, and AREG-positive.

### (Item 2)

The inducible regulatory T cell according to the preceding item, in which the inducible regulatory T cell has at least two characteristics selected from the group consisting of CTLA4-positive, NT5E-positive, ITGAE (CD103)-positive, and AREG-positive.

### (Item 3)

The inducible regulatory T cell according to any one of the preceding items, in which the inducible regulatory T cell is at least CTLA4-positive.

### (Item 4)

The inducible regulatory T cell according to any one of the preceding items, in which the CNS2 region of the FOXP3 gene is demethylated.

### (Item 5)

The inducible regulatory T cell according to any one of the preceding items, in which the inducible regulatory T cell is CD4-positive or CD8-positive.

### (Item 6)

The inducible regulatory T cell according to any one of the preceding items, in which the inducible regulatory T cell is obtained from or derived from a human peripheral blood T cell or a human tissue-derived T cell.

### (Item 7)

An inducible regulatory T cell obtained by a method including:
a step (a) of stimulating CD4-positive T cells or CD8-positive T cells in peripheral blood in a first basal medium for about 1 to about 5 days;
a step (b) of subjecting the cells obtained in the step (a) to a resting culture in a medium containing IL-2 for at least about 1 to about 3 days;
a step (c) of stimulating the cells obtained in the step (b) in a second basal medium for about 1 to about 5 days; and
a step (d) of subjecting the cells obtained in the step (c) to a resting culture in a medium containing IL-2 for at least about 1 to about 3 days.

### (Item 8)

A cell population containing T cells, in which about 50% or more of the T cells in the cell population are the inducible regulatory T cells according to any one of the preceding items.

### (Item 9)

The cell population according to any one of the preceding items, in which about 80% or more of the T cells in the cell population are the inducible regulatory T cells according to any one of the preceding items.

### (Item 10)

The cell population according to any one of the preceding items, in which the T cells in the cell population are regulatory T cells.

### (Item 11)

The cell population according to any one of the preceding items, in which the cell population contains about 90% or more T cells.

### (Item 12)

A pharmaceutical composition containing the inducible regulatory T cell according to any one of the preceding items or the cell population according to any one of the preceding items.

### (Item 13)

A regenerative medical material or product containing the inducible regulatory T cell according to any one of the preceding items or the cell population according to any one of the preceding items.

### (Item A1)

A method for producing inducible regulatory T cells, the method including:
a step (a) of stimulating CD4-positive T cells or CD8-positive T cells in peripheral blood in a first basal medium for about 1 to about 5 days;
a step (b) of subjecting the cells obtained in the step (a) to a resting culture in a medium containing IL-2 for at least about 1 to about 3 days;
a step (c) of stimulating the cells obtained in the step (b) in a second basal medium for about 1 to about 5 days; and
a step (d) of subjecting the cells obtained in the step (c) to a resting culture in a medium containing IL-2 for at least about 1 to about 3 days.

### (Item A2-1)

The method according to any one of the preceding items, in which the first basal medium contains an anti-CD3 antibody, TGF-β, IL-2, retinoic acid, and at least one selected from the group consisting of a CDK8 inhibitor, a CDK19 inhibitor, and a CDK8/19 inhibitor.

### (Item A2-2)

The method according to any one of the preceding items, in which the second basal medium contains an anti-CD3 antibody, TGF-β, IL-2, retinoic acid, ascorbic acid, and at least one selected from the group consisting of a CDK8 inhibitor, a CDK19 inhibitor, and a CDK8/19 inhibitor.

### (Item A2-1A)

The method according to any one of the preceding items, in which the first basal medium contains an anti-CD3 antibody, TGF-β, IL-2, retinoic acid, and a CDK8 inhibitor, a CDK19 inhibitor, or a CDK8/19 inhibitor.

### (Item A2-2A)

The method according to any one of the preceding items, in which the second basal medium contains an anti-CD3 antibody, TGF-β, IL-2, retinoic acid, ascorbic acid, and a CDK8 inhibitor, a CDK19 inhibitor, or a CDK8/19 inhibitor.

### (Item A2-3)

The method according to any one of the preceding items, in which at least one selected from the group consisting of a CDK8 inhibitor, a CDK19 inhibitor, and a CDK8/19 inhibitor contained in the first basal medium includes Senexin A or AS2863619.

### (Item A2-4)

The method according to any one of the preceding items, in which at least one selected from the group consisting of a CDK8 inhibitor, a CDK19 inhibitor, and a CDK8/19 inhibitor contained in the second basal medium includes Senexin A or AS2863619.

### (Item A2-5)

The method according to any one of the preceding items, in which TGF-β contained in the first basal medium includes TGF-β1 and/or TGF-β3.

### (Item A2-6)

The method according to any one of the preceding items, in which TGF-β contained in the second basal medium includes TGF-β1 and/or TGF-β3.

### (Item A2-7)

The method according to any one of the preceding items, in which TGF-β contained in the first basal medium includes TGF-β1.

### (Item A2-8)

The method according to any one of the preceding items, in which TGF-β contained in the second basal medium includes TGF-β1.

### (Item A2-9)

The method according to any one of the preceding items, in which the first basal medium contains an anti-CD3 antibody, TGF-β1, IL-2, retinoic acid, and Selexin A.

### (Item A2-10)

The method according to any one of the preceding items, in which the second basal medium contains an anti-CD3 antibody, TGF-β1, IL-2, retinoic acid, Selexin A, and ascorbic acid.

### (Item A2)

The method according to the preceding item, in which the first basal medium contains at least one factor selected from the group consisting of an anti-CD3 antibody, TGF-β1, IL-2, retinoic acid, a CDK8 inhibitor, a CDK19 inhibitor, a CDK8/19 inhibitor, and ascorbic acid.

### (Item A3)

The method according to any one of the preceding items, in which the first basal medium contains an anti-CD3 antibody, TGF-β1, IL-2, retinoic acid, a CDK8 inhibitor, a CDK19 inhibitor, a CDK8/19 inhibitor, and ascorbic acid.

### (Item A4)

The method according to any one of the preceding items, in which the second basal medium contains at least one factor selected from the group consisting of an anti-CD3 antibody, TGF-β1, IL-2, retinoic acid, a CDK8 inhibitor, a CDK19 inhibitor, and a CDK8/19 inhibitor.

### (Item A5)

The method according to any one of the preceding items, in which the second basal medium contains an anti-CD3 antibody, TGF-β1, IL-2, retinoic acid, a CDK8 inhibitor, a CDK19 inhibitor, and a CDK8/19 inhibitor.

### (Item A6)

The method according to any one of the preceding items, in which the step (a) includes stimulating the CD4-positive T cells or CD8-positive T cells in the first basal medium for about 3 days.

### (Item A7)

The method according to any one of the preceding items, in which the step (b) includes subjecting the cells obtained in the step (a) to a resting culture in a medium containing the IL-2 for at least about 2 days.

### (Item A8)

The method according to any one of the preceding items, in which the step (c) includes stimulating the cells obtained in the step (b) in the second basal medium for about 3 days.

### (Item A9)

The method according to any one of the preceding items, in which the step (d) includes subjecting the cells obtained in the step (c) to a resting culture in a medium containing the IL-2 for at least about 2 days.

### (Item A10)

An inducible regulatory T cell or a cell population containing the inducible regulatory T cells generated by the method according to any one of the preceding items.

The present disclosure further provides the following.

### (Item CX1)

A pharmaceutical composition for treating or preventing a T cell-related disease, the pharmaceutical composition containing, as an active ingredient, an inducible regulatory human T cell having at least one characteristic selected from the group consisting of FoxP3-positive, CTLA4-positive, NT5E-positive, ITGAE (CD103)-positive, AREG-positive, CD172g-positive, and CD26-positive.

### (Item CX1A)

A pharmaceutical composition for treating or preventing a T cell-related disease, the pharmaceutical composition containing, as an active ingredient, an NT5E-positive inducible regulatory human T cell.

### (Item CX1B)

A pharmaceutical composition for treating or preventing a T cell-related disease, the pharmaceutical composition containing, as an active ingredient, an ITGAE (CD103)-positive inducible regulatory human T cell.

### (Item CX1C)

A pharmaceutical composition for treating or preventing a T cell-related disease, the pharmaceutical composition containing, as an active ingredient, an AREG-positive inducible regulatory human T cell.

### (Item CX1D)

A pharmaceutical composition for treating or preventing a T cell-related disease, the pharmaceutical composition containing, as an active ingredient, a CD172g-positive inducible regulatory human T cell.

### (Item CX1E)

A pharmaceutical composition for treating or preventing a T cell-related disease, the pharmaceutical composition containing, as an active ingredient, a CD26-positive inducible regulatory human T cell.

### (Item CX1F)

A pharmaceutical composition for treating or preventing a T cell-related disease, the pharmaceutical composition containing, as an active ingredient, a CTLA4-positive inducible regulatory human T cell.

### (Item CX1G)

A pharmaceutical composition for treating or preventing a T cell-related disease, the pharmaceutical composition containing, as an active ingredient, a stably FoxP3-positive inducible regulatory human T cell.

### (Item CX1G1)

A pharmaceutical composition for treating or preventing a T cell-related disease, the pharmaceutical composition containing, as an active ingredient, an inducible regulatory human T cell having at least two characteristics selected from the group consisting of CTLA4-positive, NT5E-positive, ITGAE (CD103)-positive, AREG-positive, CD172g-positive, and CD26-positive.

### (Item CX1G2)

A pharmaceutical composition for treating or preventing a T cell-related disease, the pharmaceutical composition containing, as an active ingredient, an inducible regulatory human T cell having at least three characteristics selected from the group consisting of CTLA4-positive, NT5E-positive, ITGAE (CD103)-positive, AREG-positive, CD172g-positive, and CD26-positive.

### (Item CX1G3)

A pharmaceutical composition for treating or preventing a T cell-related disease, the pharmaceutical composition containing, as an active ingredient, an inducible regulatory human T cell having at least four characteristics selected from the group consisting of CTLA4-positive, NT5E-positive, ITGAE (CD103)-positive, AREG-positive, CD172g-positive, and CD26-positive.

### (Item CX1G4)

A pharmaceutical composition for treating or preventing a T cell-related disease, the pharmaceutical composition containing, as an active ingredient, an inducible regulatory human T cell having at least five characteristics selected from the group consisting of CTLA4-positive, NT5E-positive, ITGAE (CD103)-positive, AREG-positive, CD172g-positive, and CD26-positive.

### (Item CX1G5)

A pharmaceutical composition for treating or preventing a T cell-related disease, the pharmaceutical composition containing, as an active ingredient, an inducible regulatory human T cell having all of CTLA4-positive, NT5E-positive, ITGAE (CD103)-positive, AREG-positive, CD172g-positive, and CD26-positive characteristics.

### (Item CX2)

The pharmaceutical composition according to any one of the preceding items, in which the inducible regulatory human T cell is at least CTLA4-positive and FoxP3-positive.

### (Item CX2a)

The pharmaceutical composition according to any one of the preceding items, in which the FoxP3 is stably expressed.

### (Item CX2b)

The pharmaceutical composition according to any one of the preceding items, in which the FoxP3 is continuously expressed for at least about 48 hours or longer.

### (Item CX2c)

The pharmaceutical composition according to any one of the preceding items, in which the FoxP3 is stably expressed at least from day 7 of culture.

### (Item CX2d)

The pharmaceutical composition according to any one of the preceding items, in which the FoxP3 is expressed even after freeze-thawing of the cell.

### (Item CX3)

The pharmaceutical composition according to any one of the preceding items, in which the inducible regulatory human T cell is at least CD172g-positive and/or CD26-positive.

### (Item CX4)

The pharmaceutical composition according to any one of the preceding items, in which the CNS2 region of the FOXP3 gene of the inducible regulatory human T cell is demethylated.

### (Item CX5)

The pharmaceutical composition according to any one of the preceding items, in which the inducible regulatory human T cell is CD4-positive or CD8-positive.

### (Item CX6)

The pharmaceutical composition according to any one of the preceding items, in which the inducible regulatory human T cell is obtained from or derived from a human peripheral blood T cell or a human tissue-derived T cell.

### (Item CX7)

The pharmaceutical composition according to any one of the preceding items, in which the inducible regulatory human T cell is obtained by a method including:
a step (a) of stimulating CD4-positive T cells or CD8-positive T cells in human peripheral blood in a first basal medium for about 1 to about 5 days;
a step (b) of subjecting the cells obtained in the step (a) to a resting culture in a medium containing IL-2 for at least about 1 to about 3 days;
a step (c) of stimulating the cells obtained in the step (b) in a second basal medium for about 1 to about 5 days; and
a step (d) of subjecting the cells obtained in the step (c) to a resting culture in a medium containing IL-2 for at least about 1 to about 3 days.

### (Item CX8)

The pharmaceutical composition according to any one of the preceding items, in which the pharmaceutical composition contains, as an active ingredient, a cell population containing the inducible regulatory human T cells, and the cell population has a proportion of about 50% or more of cells each having at least one characteristic selected from the group consisting of FoxP3-positive, CTLA4-positive, NT5E-positive, ITGAE (CD103)-positive, AREG-positive, CD172g-positive, and CD26-positive.

### (Item CX8A)

A pharmaceutical composition for treating or preventing a T cell-related disease, the pharmaceutical composition containing, as an active ingredient, a cell population containing inducible regulatory human T cells, in which the cell population has a proportion of about 50% or more of NT5E-positive cells.

### (Item CX8B)

A pharmaceutical composition for treating or preventing a T cell-related disease, the pharmaceutical composition containing, as an active ingredient, a cell population containing inducible regulatory human T cells, in which the cell population has a proportion of about 50% or more of ITGAE (CD103)-positive cells.

### (Item CX8C)

A pharmaceutical composition for treating or preventing a T cell-related disease, the pharmaceutical composition containing, as an active ingredient, a cell population containing inducible regulatory human T cells, in which the cell population has a proportion of about 50% or more of AREG-positive cells.

### (Item CX8D)

A pharmaceutical composition for treating or preventing a T cell-related disease, the pharmaceutical composition containing, as an active ingredient, a cell population containing inducible regulatory human T cells, in which the cell population has a proportion of about 50% or more of CD172g-positive cells.

### (Item CX8E)

A pharmaceutical composition for treating or preventing a T cell-related disease, the pharmaceutical composition containing, as an active ingredient, a cell population containing inducible regulatory human T cells, in which the cell population has a proportion of about 50% or more of CD26-positive cells.

### (Item CX8F)

A pharmaceutical composition for treating or preventing a T cell-related disease, the pharmaceutical composition containing, as an active ingredient, a cell population containing inducible regulatory human T cells, in which the cell population has a proportion of about 50% or more of CTLA4-positive cells.

### (Item CX8G)

A pharmaceutical composition for treating or preventing a T cell-related disease, the pharmaceutical composition containing, as an active ingredient, a cell population containing inducible regulatory human T cells, in which the cell population has a proportion of about 50% or more of stably FoxP3-positive cells.

### (Item CX8G1)

A pharmaceutical composition for treating or preventing a T cell-related disease, the pharmaceutical composition containing, as an active ingredient, a cell population containing inducible regulatory human T cells, in which the cell population has a proportion of about 50% or more of cells each having at least two characteristics selected from the group consisting of CTLA4-positive, NT5E-positive, ITGAE (CD103)-positive, AREG-positive, CD172g-positive, and CD26-positive.

### (Item CX8G2)

A pharmaceutical composition for treating or preventing a T cell-related disease, the pharmaceutical composition containing, as an active ingredient, a cell population containing inducible regulatory human T cells, in which the cell population has a proportion of about 50% or more of cells each having at least three characteristics selected from the group consisting of CTLA4-positive, NT5E-positive, ITGAE (CD103)-positive, AREG-positive, CD172g-positive, and CD26-positive.

### (Item CX8G3)

A pharmaceutical composition for treating or preventing a T cell-related disease, the pharmaceutical composition containing, as an active ingredient, a cell population containing inducible regulatory human T cells, in which the cell population has a proportion of about 50% or more of cells each having at least four characteristics selected from the group consisting of CTLA4-positive, NT5E-positive, ITGAE (CD103)-positive, AREG-positive, CD172g-positive, and CD26-positive.

### (Item CX8G4)

A pharmaceutical composition for treating or preventing a T cell-related disease, the pharmaceutical composition containing, as an active ingredient, a cell population containing inducible regulatory human T cells, in which the cell population has a proportion of about 50% or more of cells each having at least five characteristics selected from the group consisting of CTLA4-positive, NT5E-positive, ITGAE (CD103)-positive, AREG-positive, CD172g-positive, and CD26-positive.

### (Item CX8G5)

A pharmaceutical composition for treating or preventing a T cell-related disease, the pharmaceutical composition containing, as an active ingredient, a cell population containing inducible regulatory human T cells, in which the cell population has a proportion of about 50% or more of cells each having all of CTLA4-positive, NT5E-positive, ITGAE (CD103)-positive, AREG-positive, CD172g-positive, and CD26-positive characteristics.

### (Item CX9)

The pharmaceutical composition according to any one of the preceding items, in which proportions of at least the CTLA4-positive cells and FoxP3-positive cells in the cell population are each about 50% or more.

### (Item CX9a)

The pharmaceutical composition according to any one of the preceding items, in which the FoxP3 is stably expressed.

### (Item CX9b)

The pharmaceutical composition according to any one of the preceding items, in which the FoxP3 is continuously expressed for at least about 48 hours or longer.

### (Item CX9c)

The pharmaceutical composition according to any one of the preceding items, in which the FoxP3 is stably expressed at least from day 7 of culture.

### (Item CX9d)

The pharmaceutical composition according to any one of the preceding items, in which the FoxP3 is expressed even after freeze-thawing of the cell.

### (Item CX10)

The pharmaceutical composition according to any one of the preceding items, in which proportions of at least the CD172g-positive cells and/or CD26-positive cells in the cell population are each about 50% or more.

### (Item CX11)

The pharmaceutical composition according to any one of the preceding items, in which a proportion of cells having the at least one characteristic in the cell population is about 60% or more.

### (Item CX12)

The pharmaceutical composition according to any one of the preceding items, in which a proportion of cells having the at least one characteristic in the cell population is about 80% or more.

### (Item CX12a)

The pharmaceutical composition according to any one of the preceding items, in which a proportion of FoxP3 strong-positive cells in the cell population is about 50% or more.

### (Item CX12b)

The pharmaceutical composition according to any one of the preceding items, in which a proportion of stably FoxP3 strong-positive cells in the cell population is about 50% or more.

### (Item CX13)

The pharmaceutical composition according to any one of the preceding items, in which the cell population contains about 90% or more T cells.

### (Item CX14)

The pharmaceutical composition according to any one of the preceding items, in which the inducible regulatory human T cells are contained so as to be administered at about 10⁸ to about 10⁹ cells per dose or about 10⁷ cells/kg.

### (Item CX15)

The pharmaceutical composition according to any one of the preceding items, in which the T cell-related disease includes an autoimmune disease, an infectious disease, cancer, an allergy, an inflammatory disease, and ALS, which are treatable by an immunosuppressive action.

### (Item CX16)

The pharmaceutical composition according to any one of the preceding items, in which the autoimmune disease is selected from the group consisting of Addison's disease, alopecia areata, ankylosing spondylitis, autoimmune parotitis, Crohn's disease, diabetes (Type I), dystrophic epidermolysis bullosa, epididymitis, glomerulonephritis, Graves' disease, Guillain-Barré syndrome, Hashimoto's disease, hemolytic anemia, systemic lupus erythematosus, multiple sclerosis, myasthenia gravis, psoriasis, rheumatic fever, rheumatoid arthritis, sarcoidosis, scleroderma, Sjogren's syndrome, spondyloarthritis, thyroiditis, vasculitis, vitiligo, myxedema, pernicious anemia, cardiomyopathy, dilated cardiomyopathy (DCM), peripartum cardiomyopathy (PPCM), idiopathic cardiomyopathy, Chagas cardiomyopathy, Chagas megacolon, Chagas megaesophagus, Chagas neuropathy, benign prostatic hyperplasia, scleroderma, Raynaud's syndrome, preeclampsia, myocarditis, glaucoma, hypertension, pulmonary hypertension, malignant hypertension, pemphigus, Alzheimer's disease, systemic sclerosis, polymyositis, mixed connective tissue disease, antiphospholipid antibody syndrome, microscopic polyangiitis, granulomatosis with polyangiitis, eosinophilic granulomatosis with polyangiitis, crescentic glomerulonephritis, organ-specific autoimmune disease, Basedow's disease, autoimmune liver disease, primary sclerosing cholangitis (PSC), autoimmune hepatitis (AIH), primary biliary cholangitis, autoimmune pancreatitis, Goodpasture's syndrome, autoimmune hemolytic anemia, megaloblastic anemia, idiopathic thrombocytopenic purpura, primary sclerosing cholangitis, polyarteritis nodosa, Takayasu's arteritis, giant cell arteritis, polymyalgia rheumatica, adult-onset Still's disease, Behçet's disease, and ulcerative colitis.

### (Item CX17)

The pharmaceutical composition according to any one of the preceding items, in which the pharmaceutical composition is administered by injection.

### (Item CX18)

The pharmaceutical composition according to any one of the preceding items, in which when the pharmaceutical composition is administered to a patient and is ineffective or insufficient, the pharmaceutical composition is additionally administered to the patient.

### (Item CX19)

The pharmaceutical composition according to any one of the preceding items, in which the pharmaceutical composition is additionally administered at least about 2 weeks after the initial administration.

In addition, the present disclosure also provides the following.

### (Item C1)

A pharmaceutical composition for treating or preventing a T cell-related disease, the pharmaceutical composition containing, as an active ingredient, an inducible regulatory T cell having at least one characteristic selected from the group consisting of CTLA4-positive, NT5E-positive, ITGAE (CD103)-positive, and AREG-positive.

### (Item C2)

The pharmaceutical composition according to the preceding item, in which the inducible regulatory T cell has at least two characteristics selected from the group consisting of CTLA4-positive, NT5E-positive, ITGAE (CD103)-positive, and AREG-positive.

### (Item C3)

The pharmaceutical composition according to any one of the preceding items, in which the inducible regulatory T cell is at least CTLA4-positive.

### (Item C4)

The pharmaceutical composition according to any one of the preceding items, in which the CNS2 region of the FOXP3 gene of the inducible regulatory T cell is demethylated.

### (Item C5)

The pharmaceutical composition according to any one of the preceding items, in which the inducible regulatory T cell is CD4-positive or CD8-positive.

### (Item C6)

The pharmaceutical composition according to any one of the preceding items, in which the inducible regulatory T cell is obtained from or derived from a human peripheral blood T cell or a human tissue-derived T cell.

### (Item C7)

The pharmaceutical composition according to any one of the preceding items, in which the inducible regulatory T cell is obtained by a method including:
a step (a) of stimulating CD4-positive T cells or CD8-positive T cells in peripheral blood in a first basal medium for about 1 to about 5 days;
a step (b) of subjecting the cells obtained in the step (a) to a resting culture in a medium containing IL-2 for at least about 1 to about 3 days;
a step (c) of stimulating the cells obtained in the step (b) in a second basal medium for about 1 to about 5 days; and
a step (d) of subjecting the cells obtained in the step (c) to a resting culture in a medium containing IL-2 for at least about 1 to about 3 days.

### (Item C8)

The pharmaceutical composition according to any one of the preceding items, in which the pharmaceutical composition contains a T cell population in which about 50% or more of the cells are the inducible regulatory T cells.

### (Item C9)

The pharmaceutical composition according to any one of the preceding items, in which the pharmaceutical composition contains a T cell population in which about 80% or more of the cells are the inducible regulatory T cells.

### (Item C10)

The pharmaceutical composition according to any one of the preceding items, in which the T cell population is a regulatory T cell population.

### (Item C11)

The pharmaceutical composition according to any one of the preceding items, in which the cell population contains about 90% or more T cells.

### (Item C12)

The pharmaceutical composition according to any one of the preceding items, in which the inducible regulatory T cells are contained so as to be administered at about 10⁸ to about 10⁹ cells per dose or about 10⁷ cells/kg.

### (Item C13)

The pharmaceutical composition according to any one of the preceding items, in which the T cell-related disease includes an autoimmune disease, an infectious disease, cancer, an allergy, an inflammatory disease, and ALS, which are treatable by an immunosuppressive action.

### (Item C14)

The pharmaceutical composition according to any one of the preceding items, in which the autoimmune disease is selected from the group consisting of Addison's disease, alopecia areata, ankylosing spondylitis, autoimmune parotitis, Crohn's disease, diabetes (Type I), dystrophic epidermolysis bullosa, epididymitis, glomerulonephritis, Graves' disease, Guillain-Barré syndrome, Hashimoto's disease, hemolytic anemia, systemic lupus erythematosus, multiple sclerosis, myasthenia gravis, psoriasis, rheumatic fever, rheumatoid arthritis, sarcoidosis, scleroderma, Sjogren's syndrome, spondyloarthritis, thyroiditis, vasculitis, vitiligo, myxedema, pernicious anemia, cardiomyopathy, dilated cardiomyopathy (DCM), peripartum cardiomyopathy (PPCM), idiopathic cardiomyopathy, Chagas cardiomyopathy, Chagas megacolon, Chagas megaesophagus, Chagas neuropathy, benign prostatic hyperplasia, scleroderma, Raynaud's syndrome, preeclampsia, myocarditis, glaucoma, hypertension, pulmonary hypertension, malignant hypertension, pemphigus, Alzheimer's disease, systemic sclerosis, polymyositis, mixed connective tissue disease, antiphospholipid antibody syndrome, microscopic polyangiitis, granulomatosis with polyangiitis, eosinophilic granulomatosis with polyangiitis, crescentic glomerulonephritis, organ-specific autoimmune disease, Basedow's disease, autoimmune liver disease, primary sclerosing cholangitis (PSC), autoimmune hepatitis (AIH), primary biliary cholangitis, autoimmune pancreatitis, Goodpasture's syndrome, autoimmune hemolytic anemia, megaloblastic anemia, idiopathic thrombocytopenic purpura, primary sclerosing cholangitis, polyarteritis nodosa, Takayasu's arteritis, giant cell arteritis, polymyalgia rheumatica, adult-onset Still's disease, Behçet's disease, and ulcerative colitis.

### (Item C15)

The pharmaceutical composition according to any one of the preceding items, in which the pharmaceutical composition is administered by injection.

### (Item C16)

The pharmaceutical composition according to any one of the preceding items, in which when the pharmaceutical composition is administered to a patient and is ineffective or insufficient, the pharmaceutical composition is additionally administered to the patient.

### (Item C17)

The pharmaceutical composition according to any one of the preceding items, in which the pharmaceutical composition is additionally administered at least about 2 weeks after the initial administration.

The present disclosure further provides the following.

### (Item AX14)

The inducible regulatory human T cell according to any one of the preceding items, or the cell population according to any one of the preceding items, for use as a medicament.

### (Item AX15)

The inducible regulatory human T cell according to any one of the preceding items, or the cell population according to any one of the preceding items, for use as a regenerative medical material or product.

### (Item ACX1)

An inducible regulatory human T cell for use in the treatment or prevention of a T cell-related disease, the inducible regulatory human T cell having at least one characteristic selected from the group consisting of FoxP3-positive, CTLA4-positive, NT5E-positive, ITGAE (CD103)-positive, AREG-positive, CD172g-positive, and CD26-positive.

### (Item ACX1A)

An NT5E-positive inducible regulatory human T cell for use in the treatment or prevention of a T cell-related disease.

### (Item ACX1B)

An ITGAE (CD103)-positive inducible regulatory human T cell for use in the treatment or prevention of a T cell-related disease.

### (Item ACX1C)

An AREG-positive inducible regulatory human T cell for use in the treatment or prevention of a T cell-related disease.

### (Item ACX1D)

A CD172g-positive inducible regulatory human T cell for use in the treatment or prevention of a T cell-related disease.

### (Item ACX1E)

A CD26-positive inducible regulatory human T cell for use in the treatment or prevention of a T cell-related disease.

### (Item ACX1F)

A CTLA4-positive inducible regulatory human T cell for use in the treatment or prevention of a T cell-related disease.

### (Item ACX1G)

A stably FoxP3-positive inducible regulatory human T cell for use in the treatment or prevention of a T cell-related disease.

### (Item ACX1G1)

An inducible regulatory human T cell for use in the treatment or prevention of a T cell-related disease, the inducible regulatory human T cell having at least two characteristics selected from the group consisting of CTLA4-positive, NT5E-positive, ITGAE (CD103)-positive, AREG-positive, CD172g-positive, and CD26-positive.

### (Item ACX1G2)

An inducible regulatory human T cell for use in the treatment or prevention of a T cell-related disease as an active ingredient, the inducible regulatory human T cell having at least three characteristics selected from the group consisting of CTLA4-positive, NT5E-positive, ITGAE (CD103)-positive, AREG-positive, CD172g-positive, and CD26-positive.

### (Item ACX1G3)

An inducible regulatory human T cell for use in the treatment or prevention of a T cell-related disease, the inducible regulatory human T cell having at least four characteristics selected from the group consisting of CTLA4-positive, NT5E-positive, ITGAE (CD103)-positive, AREG-positive, CD172g-positive, and CD26-positive.

### (Item ACX1G4)

An inducible regulatory human T cell for use in the treatment or prevention of a T cell-related disease, the inducible regulatory human T cell having at least five characteristics selected from the group consisting of CTLA4-positive, NT5E-positive, ITGAE (CD103)-positive, AREG-positive, CD172g-positive, and CD26-positive.

### (Item ACX1G5)

An inducible regulatory human T cell for use in the treatment or prevention of a T cell-related disease, the inducible regulatory human T cell having all of CTLA4-positive, NT5E-positive, ITGAE (CD103)-positive, AREG-positive, CD172g-positive, and CD26-positive characteristics.

### (Item ACX2)

The inducible regulatory human T cell for use according to any one of the preceding items, in which the inducible regulatory human T cell is at least CTLA4-positive and FoxP3-positive.

### (Item ACX2a)

The inducible regulatory human T cell for use according to any one of the preceding items, in which the FoxP3 is stably expressed.

### (Item ACX2b)

The inducible regulatory human T cell for use according to any one of the preceding items, in which the FoxP3 is continuously expressed for at least about 48 hours or longer.

### (Item ACX2c)

The inducible regulatory human T cell for use according to any one of the preceding items, in which the FoxP3 is stably expressed at least from day 7 of culture.

### (Item ACX2d)

The inducible regulatory human T cell for use according to any one of the preceding items, in which the FoxP3 is expressed even after freeze-thawing of the cell.

### (Item ACX3)

The inducible regulatory human T cell for use according to any one of the preceding items, in which the inducible regulatory human T cell is at least CD172g-positive and/or CD26-positive.

### (Item ACX4)

The inducible regulatory human T cell for use according to any one of the preceding items, in which the CNS2 region of the FOXP3 gene of the inducible regulatory human T cell is demethylated.

### (Item ACX5)

The inducible regulatory human T cell for use according to any one of the preceding items, in which the inducible regulatory human T cell is CD4-positive or CD8-positive.

### (Item ACX6)

The inducible regulatory human T cell for use according to any one of the preceding items, in which the inducible regulatory human T cell is obtained from or derived from a human peripheral blood T cell or a human tissue-derived T cell.

### (Item ACX7)

The inducible regulatory human T cell for use according to any one of the preceding items, in which the inducible regulatory human T cell is obtained by a method including:
a step (a) of stimulating CD4-positive T cells or CD8-positive T cells in human peripheral blood in a first basal medium for about 1 to about 5 days;
a step (b) of subjecting the cells obtained in the step (a) to a resting culture in a medium containing IL-2 for at least about 1 to about 3 days;
a step (c) of stimulating the cells obtained in the step (b) in a second basal medium for about 1 to about 5 days; and
a step (d) of subjecting the cells obtained in the step (c) to a resting culture in a medium containing IL-2 for at least about 1 to about 3 days.

### (Item ACX8)

A cell population containing the inducible regulatory human T cells for use according to any one of the preceding items, in which a cell population containing the inducible regulatory human T cells is contained as an active ingredient, and the cell population has a proportion of about 50% or more of cells each having at least one characteristic selected from the group consisting of FoxP3-positive, CTLA4-positive, NT5E-positive, ITGAE (CD103)-positive, AREG-positive, CD172g-positive, and CD26-positive.

### (Item ACX8A)

A cell population for use in the treatment or prevention of a T cell-related disease, the cell population containing inducible regulatory human T cells, in which the cell population has a proportion of about 50% or more of NT5E-positive cells.

### (Item ACX8B)

A cell population for use in the treatment or prevention of a T cell-related disease, the cell population containing inducible regulatory human T cells, in which the cell population has a proportion of about 50% or more of ITGAE (CD103)-positive cells.

### (Item ACX8C)

A cell population for use in the treatment or prevention of a T cell-related disease, the cell population containing inducible regulatory human T cells, in which the cell population has a proportion of about 50% or more of AREG-positive cells.

### (Item ACX8D)

A cell population for use in the treatment or prevention of a T cell-related disease, the cell population containing inducible regulatory human T cells, in which the cell population has a proportion of about 50% or more of CD172g-positive cells.

### (Item ACX8E)

A cell population for use in the treatment or prevention of a T cell-related disease, the cell population containing inducible regulatory human T cells, in which the cell population has a proportion of about 50% or more of CD26-positive cells.

### (Item ACX8F)

A cell population for use in the treatment or prevention of a T cell-related disease, the cell population containing inducible regulatory human T cells, in which the cell population has a proportion of about 50% or more of CTLA4-positive cells.

### (Item ACX8G)

A cell population for use in the treatment or prevention of a T cell-related disease, the cell population containing inducible regulatory human T cells, in which the cell population has a proportion of about 50% or more of stably FoxP3-positive cells.

### (Item ACX8G1)

A cell population for use in the treatment or prevention of a T cell-related disease, the cell population containing inducible regulatory human T cells, in which the cell population has a proportion of about 50% or more of cells each having at least two characteristics selected from the group consisting of CTLA4-positive, NT5E-positive, ITGAE (CD103)-positive, AREG-positive, CD172g-positive, and CD26-positive.

### (Item ACX8G2)

A cell population for use in the treatment or prevention of a T cell-related disease, the cell population containing inducible regulatory human T cells, in which the cell population has a proportion of about 50% or more of cells each having at least three characteristics selected from the group consisting of CTLA4-positive, NT5E-positive, ITGAE (CD103)-positive, AREG-positive, CD172g-positive, and CD26-positive.

### (Item ACX8G3)

A cell population for use in the treatment or prevention of a T cell-related disease, the cell population containing inducible regulatory human T cells, in which the cell population has a proportion of about 50% or more of cells each having at least four characteristics selected from the group consisting of CTLA4-positive, NT5E-positive, ITGAE (CD103)-positive, AREG-positive, CD172g-positive, and CD26-positive.

### (Item ACX8G4)

A cell population for use in the treatment or prevention of a T cell-related disease, the cell population containing inducible regulatory human T cells, in which the cell population has a proportion of about 50% or more of cells each having at least five characteristics selected from the group consisting of CTLA4-positive, NT5E-positive, ITGAE (CD103)-positive, AREG-positive, CD172g-positive, and CD26-positive.

### (Item ACX8G5)

A cell population for use in the treatment or prevention of a T cell-related disease, the cell population containing inducible regulatory human T cells, in which the cell population has a proportion of about 50% or more of cells each having all of CTLA4-positive, NT5E-positive, ITGAE (CD103)-positive, AREG-positive, CD172g-positive, and CD26-positive characteristics.

### (Item ACX9)

The cell population for use according to any one of the preceding items, in which proportions of at least the CTLA4-positive cells and FoxP3-positive cells in the cell population are each about 50% or more.

### (Item ACX9a)

The cell population for use according to any one of the preceding items, in which the FoxP3 is stably expressed.

### (Item ACX9b)

The cell population for use according to any one of the preceding items, in which the FoxP3 is continuously expressed for at least about 48 hours or longer.

### (Item ACX9c)

The cell population for use according to any one of the preceding items, in which the FoxP3 is stably expressed at least from day 7 of culture.

### (Item ACX9d)

The cell population for use according to any one of the preceding items, in which the FoxP3 is expressed even after freeze-thawing of the cell.

### (Item ACX10)

The cell population for use according to any one of the preceding items, in which proportions of at least the CD172g-positive cells and/or CD26-positive cells in the cell population are each about 50% or more.

### (Item ACX11)

The cell population for use according to any one of the preceding items, in which a proportion of cells having the at least one characteristic in the cell population is about 60% or more.

### (Item ACX12)

The cell population for use according to any one of the preceding items, in which a proportion of cells having the at least one characteristic in the cell population is about 80% or more.

### (Item ACX12a)

The cell population for use according to any one of the preceding items, in which a proportion of FoxP3 strong-positive cells in the cell population is about 50% or more.

### (Item ACX12b)

The cell population for use according to any one of the preceding items, in which a proportion of stably FoxP3 strong-positive cells in the cell population is about 50% or more.

### (Item ACX13)

The cell population for use according to any one of the preceding items, in which the cell population contains about 90% or more T cells.

### (Item ACX14)

The cell population for use according to any one of the preceding items, in which the inducible regulatory human T cells are contained so as to be administered at about 10⁸ to about 10⁹ cells per dose or about 10⁷ cells/kg.

### (Item ACX15)

The cell population for use according to any one of the preceding items, in which the T cell-related disease includes an autoimmune disease, an infectious disease, cancer, an allergy, an inflammatory disease, and ALS, which are treatable by an immunosuppressive action.

### (Item ACX16)

The cell population for use according to any one of the preceding items, in which the autoimmune disease is selected from the group consisting of Addison's disease, alopecia areata, ankylosing spondylitis, autoimmune parotitis, Crohn's disease, diabetes (Type I), dystrophic epidermolysis bullosa, epididymitis, glomerulonephritis, Graves' disease, Guillain-Barré syndrome, Hashimoto's disease, hemolytic anemia, systemic lupus erythematosus, multiple sclerosis, myasthenia gravis, psoriasis, rheumatic fever, rheumatoid arthritis, sarcoidosis, scleroderma, Sjogren's syndrome, spondyloarthritis, thyroiditis, vasculitis, vitiligo, myxedema, pernicious anemia, cardiomyopathy, dilated cardiomyopathy (DCM), peripartum cardiomyopathy (PPCM), idiopathic cardiomyopathy, Chagas cardiomyopathy, Chagas megacolon, Chagas megaesophagus, Chagas neuropathy, benign prostatic hyperplasia, scleroderma, Raynaud's syndrome, preeclampsia, myocarditis, glaucoma, hypertension, pulmonary hypertension, malignant hypertension, pemphigus, Alzheimer's disease, systemic sclerosis, polymyositis, mixed connective tissue disease, antiphospholipid antibody syndrome, microscopic polyangiitis, granulomatosis with polyangiitis, eosinophilic granulomatosis with polyangiitis, crescentic glomerulonephritis, organ-specific autoimmune disease, Basedow's disease, autoimmune liver disease, primary sclerosing cholangitis (PSC), autoimmune hepatitis (AIH), primary biliary cholangitis, autoimmune pancreatitis, Goodpasture's syndrome, autoimmune hemolytic anemia, megaloblastic anemia, idiopathic thrombocytopenic purpura, primary sclerosing cholangitis, polyarteritis nodosa, Takayasu's arteritis, giant cell arteritis, polymyalgia rheumatica, adult-onset Still's disease, Behçet's disease, and ulcerative colitis.

### (Item ACX17)

The cell population for use according to any one of the preceding items, in which the cell population is administered by injection.

### (Item ACX18)

The cell population for use according to any one of the preceding items, in which when the cell population is administered to a patient and is ineffective or insufficient, the pharmaceutical composition is additionally administered to the patient.

### (Item ACX19)

The cell population for use according to any one of the preceding items, in which the cell population is additionally administered at least about 2 weeks after the initial administration.

In addition, the present disclosure also provides the following.

### (Item AC1)

An inducible regulatory T cell for use in the treatment or prevention of a T cell-related disease, the inducible regulatory T cell having at least one characteristic selected from the group consisting of CTLA4-positive, NT5E-positive, ITGAE (CD103)-positive, and AREG-positive.

### (Item AC2)

The inducible regulatory T cell for use according to the preceding item, in which the inducible regulatory T cell has at least two characteristics selected from the group consisting of CTLA4-positive, NT5E-positive, ITGAE (CD103)-positive, and AREG-positive.

### (Item AC3)

The inducible regulatory T cell for use according to any one of the preceding items, in which the inducible regulatory T cell is at least CTLA4-positive.

### (Item AC4)

The inducible regulatory T cell for use according to any one of the preceding items, in which the CNS2 region of the FOXP3 gene of the inducible regulatory T cell is demethylated.

### (Item AC5)

The inducible regulatory T cell for use according to any one of the preceding items, in which the inducible regulatory T cell is CD4-positive or CD8-positive.

### (Item AC6)

The inducible regulatory T cell for use according to any one of the preceding items, in which the inducible regulatory T cell is obtained from or derived from a human peripheral blood T cell or a human tissue-derived T cell.

### (Item AC7)

The inducible regulatory T cell for use according to any one of the preceding items, in which the inducible regulatory T cell is obtained by a method including:
a step (a) of stimulating CD4-positive T cells or CD8-positive T cells in peripheral blood in a first basal medium for about 1 to about 5 days;
a step (b) of subjecting the cells obtained in the step (a) to a resting culture in a medium containing IL-2 for at least about 1 to about 3 days;
a step (c) of stimulating the cells obtained in the step (b) in a second basal medium for about 1 to about 5 days; and
a step (d) of subjecting the cells obtained in the step (c) to a resting culture in a medium containing IL-2 for at least about 1 to about 3 days.

### (Item AC8)

The cell population for use according to any one of the preceding items, in which the cell population includes a T cell population in which about 50% or more of the cells are the inducible regulatory T cells.

### (Item AC9)

The cell population for use according to any one of the preceding items, in which the cell population includes a T cell population in which about 80% or more of the cells are the inducible regulatory T cells.

### (Item AC10)

The cell population for use according to any one of the preceding items, in which the T cell population is a regulatory T cell population.

### (Item AC11)

The cell population for use according to any one of the preceding items, in which the cell population contains about 90% or more T cells.

### (Item AC12)

The cell population for use according to any one of the preceding items, in which the inducible regulatory T cells are contained so as to be administered at about 10⁸ to about 10⁹ cells per dose or about 10⁷ cells/kg.

### (Item AC13)

The cell population for use according to any one of the preceding items, in which the T cell-related disease includes an autoimmune disease, an infectious disease, cancer, an allergy, an inflammatory disease, and ALS, which are treatable by an immunosuppressive action.

### (Item AC14)

The cell population for use according to any one of the preceding items, in which the autoimmune disease is selected from the group consisting of Addison's disease, alopecia areata, ankylosing spondylitis, autoimmune parotitis, Crohn's disease, diabetes (Type I), dystrophic epidermolysis bullosa, epididymitis, glomerulonephritis, Graves' disease, Guillain-Barré syndrome, Hashimoto's disease, hemolytic anemia, systemic lupus erythematosus, multiple sclerosis, myasthenia gravis, psoriasis, rheumatic fever, rheumatoid arthritis, sarcoidosis, scleroderma, Sjogren's syndrome, spondyloarthritis, thyroiditis, vasculitis, vitiligo, myxedema, pernicious anemia, cardiomyopathy, dilated cardiomyopathy (DCM), peripartum cardiomyopathy (PPCM), idiopathic cardiomyopathy, Chagas cardiomyopathy, Chagas megacolon, Chagas megaesophagus, Chagas neuropathy, benign prostatic hyperplasia, scleroderma, Raynaud's syndrome, preeclampsia, myocarditis, glaucoma, hypertension, pulmonary hypertension, malignant hypertension, pemphigus, Alzheimer's disease, systemic sclerosis, polymyositis, mixed connective tissue disease, antiphospholipid antibody syndrome, microscopic polyangiitis, granulomatosis with polyangiitis, eosinophilic granulomatosis with polyangiitis, crescentic glomerulonephritis, organ-specific autoimmune disease, Basedow's disease, autoimmune liver disease, primary sclerosing cholangitis (PSC), autoimmune hepatitis (AIH), primary biliary cholangitis, autoimmune pancreatitis, Goodpasture's syndrome, autoimmune hemolytic anemia, megaloblastic anemia, idiopathic thrombocytopenic purpura, primary sclerosing cholangitis, polyarteritis nodosa, Takayasu's arteritis, giant cell arteritis, polymyalgia rheumatica, adult-onset Still's disease, Behçet's disease, and ulcerative colitis.

### (Item AC15)

The inducible regulatory T cell or cell population for use according to any one of the preceding items, in which the inducible regulatory T cell or cell population is administered by injection.

### (Item AC16)

The inducible regulatory T cell or cell population for use according to any one of the preceding items, in which when the inducible regulatory T cell or cell population is administered to a patient and is ineffective or insufficient, the pharmaceutical composition is additionally administered to the patient.

### (Item AC17)

The inducible regulatory T cell or cell population for use according to any one of the preceding items, in which the inducible regulatory T cell or cell population is additionally administered at least about 2 weeks after the initial administration.

The present disclosure also provides the following.

### (Item BX14)

A method for treatment or prevention, the method including a step of administering, to a subject, an effective amount of the inducible regulatory human T cell according to any one of the preceding items or the cell population according to any one of the preceding items as a medicament.

### (Item BX15)

A method for treatment or prevention, the method including a step of administering, to a subject, an effective amount of the inducible regulatory human T cell according to any one of the preceding items or the cell population according to any one of the preceding items as a regenerative medical material or product.

### (Item BCX1)

A method for treating or preventing a T cell-related disease, the method including a step of administering, to a subject, an effective amount of inducible regulatory human T cells having at least one characteristic selected from the group consisting of FoxP3-positive, CTLA4-positive, NT5E-positive, ITGAE (CD103)-positive, AREG-positive, CD172g-positive, and CD26-positive.

### (Item BCX1A)

A method for treating or preventing a T cell-related disease, the method including a step of administering, to a subject, an effective amount of NT5E-positive inducible regulatory human T cells.

### (Item BCX1B)

A method for treating or preventing a T cell-related disease, the method including a step of administering, to a subject, an effective amount of ITGAE (CD103)-positive inducible regulatory human T cells.

### (Item BCX1C)

A method for treating or preventing a T cell-related disease, the method including a step of administering, to a subject, an effective amount of AREG-positive inducible regulatory human T cells.

### (Item BCX1D)

A method for treating or preventing a T cell-related disease, the method including a step of administering, to a subject, an effective amount of CD172g-positive inducible regulatory human T cells.

### (Item BCX1E)

A method for treating or preventing a T cell-related disease, the method including a step of administering, to a subject, an effective amount of CD26-positive inducible regulatory human T cells.

### (Item BCX1F)

A method for treating or preventing a T cell-related disease, the method including a step of administering, to a subject, an effective amount of CTLA4-positive inducible regulatory human T cells.

### (Item BCX1G)

A method for treating or preventing a T cell-related disease, the method including a step of administering, to a subject, an effective amount of stably FoxP3-positive inducible regulatory human T cells.

### (Item BCX1G1)

A method for treating or preventing a T cell-related disease, the method including a step of administering, to a subject, an effective amount of inducible regulatory human T cells having at least two characteristics selected from the group consisting of CTLA4-positive, NT5E-positive, ITGAE (CD103)-positive, AREG-positive, CD172g-positive, and CD26-positive.

### (Item BCX1G2)

A method for treating or preventing a T cell-related disease, the method including a step of administering, to a subject, an effective amount of inducible regulatory human T cells having at least three characteristics selected from the group consisting of CTLA4-positive, NT5E-positive, ITGAE (CD103)-positive, AREG-positive, CD172g-positive, and CD26-positive.

### (Item BCX1G3)

A method for treating or preventing a T cell-related disease, the method including a step of administering, to a subject, an effective amount of inducible regulatory human T cells having at least four characteristics selected from the group consisting of CTLA4-positive, NT5E-positive, ITGAE (CD103)-positive, AREG-positive, CD172g-positive, and CD26-positive.

### (Item BCX1G4)

A method for treating or preventing a T cell-related disease, the method including a step of administering, to a subject, an effective amount of inducible regulatory human T cells having at least five characteristics selected from the group consisting of CTLA4-positive, NT5E-positive, ITGAE (CD103)-positive, AREG-positive, CD172g-positive, and CD26-positive.

### (Item BCX1G5)

A method for treating or preventing a T cell-related disease, the method including a step of administering, to a subject, an effective amount of inducible regulatory human T cells having all of CTLA4-positive, NT5E-positive, ITGAE (CD103)-positive, AREG-positive, CD172g-positive, and CD26-positive characteristics.

### (Item BCX2)

The method according to any one of the preceding items, in which the inducible regulatory human T cells are at least CTLA4-positive and FoxP3-positive.

### (Item BCX2a)

The method according to any one of the preceding items, in which the FoxP3 is stably expressed.

### (Item BCX2b)

The method according to any one of the preceding items, in which the FoxP3 is continuously expressed for at least about 48 hours or longer.

### (Item BCX2c)

The method according to any one of the preceding items, in which the FoxP3 is stably expressed at least from day 7 of culture.

### (Item BCX2d)

The method according to any one of the preceding items, in which the FoxP3 is expressed even after freeze-thawing of the cell.

### (Item BCX3)

The method according to any one of the preceding items, in which the inducible regulatory human T cells are at least CD172g-positive and/or CD26-positive.

### (Item BCX4)

The method according to any one of the preceding items, in which the CNS2 region of the FOXP3 gene of the inducible regulatory human T cell is demethylated.

### (Item BCX5)

The method according to any one of the preceding items, in which the inducible regulatory human T cells are CD4-positive or CD8-positive.

### (Item BCX6)

The method according to any one of the preceding items, in which the inducible regulatory human T cells are obtained from or derived from human peripheral blood T cells or human tissue-derived T cells.

### (Item BCX7)

The method according to any one of the preceding items, in which the inducible regulatory human T cells are obtained by a method including:
a step (a) of stimulating CD4-positive T cells or CD8-positive T cells in human peripheral blood in a first basal medium for about 1 to about 5 days;
a step (b) of subjecting the cells obtained in the step (a) to a resting culture in a medium containing IL-2 for at least about 1 to about 3 days;
a step (c) of stimulating the cells obtained in the step (b) in a second basal medium for about 1 to about 5 days; and
a step (d) of subjecting the cells obtained in the step (c) to a resting culture in a medium containing IL-2 for at least about 1 to about 3 days.

### (Item BCX8)

The method according to any one of the preceding items, the method including a step of administering, to the subject, an effective amount of a cell population containing the inducible regulatory human T cells, in which the cell population has a proportion of about 50% or more of cells each having at least one characteristic selected from the group consisting of FoxP3-positive, CTLA4-positive, NT5E-positive, ITGAE (CD103)-positive, AREG-positive, CD172g-positive, and CD26-positive.

### (Item BCX8A)

A method for treating or preventing a T cell-related disease, the method including a step of administering, to a subject, an effective amount of a cell population containing inducible regulatory human T cells, in which the cell population has a proportion of about 50% or more of NT5E-positive cells.

### (Item BCX8B)

A method for treating or preventing a T cell-related disease, the method including a step of administering, to a subject, an effective amount of a cell population containing inducible regulatory human T cells, in which the cell population has a proportion of about 50% or more of ITGAE (CD103)-positive cells.

### (Item BCX8C)

A method for treating or preventing a T cell-related disease, the method including a step of administering, to a subject, an effective amount of a cell population containing inducible regulatory human T cells, in which the cell population has a proportion of about 50% or more of AREG-positive cells.

### (Item BCX8D)

A method for treating or preventing a T cell-related disease, the method including a step of administering, to a subject, an effective amount of a cell population containing inducible regulatory human T cells, in which the cell population has a proportion of about 50% or more of CD172g-positive cells.

### (Item BCX8E)

A method for treating or preventing a T cell-related disease, the method including a step of administering, to a subject, an effective amount of a cell population containing inducible regulatory human T cells, in which the cell population has a proportion of about 50% or more of CD26-positive cells.

### (Item BCX8F)

A method for treating or preventing a T cell-related disease, the method including a step of administering, to a subject, an effective amount of a cell population containing inducible regulatory human T cells, in which the cell population has a proportion of about 50% or more of CTLA4-positive cells.

### (Item BCX8G)

A method for treating or preventing a T cell-related disease, the method including a step of administering, to a subject, an effective amount of a cell population containing inducible regulatory human T cells, in which the cell population has a proportion of about 50% or more of stably FoxP3-positive cells.

### (Item BCX8G1)

A method for treating or preventing a T cell-related disease, the method including a step of administering, to a subject, an effective amount of a cell population containing inducible regulatory human T cells, in which the cell population has a proportion of about 50% or more of cells each having at least two characteristics selected from the group consisting of CTLA4-positive, NT5E-positive, ITGAE (CD103)-positive, AREG-positive, CD172g-positive, and CD26-positive.

### (Item BCX8G2)

A method for treating or preventing a T cell-related disease, the method including a step of administering, to a subject, an effective amount of a cell population containing inducible regulatory human T cells, in which the cell population has a proportion of about 50% or more of cells each having at least three characteristics selected from the group consisting of CTLA4-positive, NT5E-positive, ITGAE (CD103)-positive, AREG-positive, CD172g-positive, and CD26-positive.

### (Item BCX8G3)

A method for treating or preventing a T cell-related disease, the method including a step of administering, to a subject, an effective amount of a cell population containing inducible regulatory human T cells, in which the cell population has a proportion of about 50% or more of cells each having at least four characteristics selected from the group consisting of CTLA4-positive, NT5E-positive, ITGAE (CD103)-positive, AREG-positive, CD172g-positive, and CD26-positive.

### (Item BCX8G4)

A method for treating or preventing a T cell-related disease, the method including a step of administering, to a subject, an effective amount of a cell population containing inducible regulatory human T cells, in which the cell population has a proportion of about 50% or more of cells each having at least five characteristics selected from the group consisting of CTLA4-positive, NT5E-positive, ITGAE (CD103)-positive, AREG-positive, CD172g-positive, and CD26-positive.

### (Item BCX8G5)

A method for treating or preventing a T cell-related disease, the method including a step of administering, to a subject, an effective amount of a cell population containing inducible regulatory human T cells, in which the cell population has a proportion of about 50% or more of cells each having all of CTLA4-positive, NT5E-positive, ITGAE (CD103)-positive, AREG-positive, CD172g-positive, and CD26-positive characteristics.

### (Item BCX9)

The method according to any one of the preceding items, in which proportions of at least the CTLA4-positive cells and FoxP3-positive cells in the cell population are each about 50% or more.

### (Item BCX9a)

The method according to any one of the preceding items, in which the FoxP3 is stably expressed in the cell population.

### (Item BCX9b)

The method according to any one of the preceding items, in which the FoxP3 is continuously expressed in the cell population for at least about 48 hours or longer.

### (Item BCX9c)

The method according to any one of the preceding items, in which the FoxP3 is stably expressed in the cell population at least from day 7 of culture.

### (Item BCX9d)

The method according to any one of the preceding items, in which the FoxP3 is expressed in the cell population even after freeze-thawing of the cell.

### (Item BCX10)

The method according to any one of the preceding items, in which proportions of at least the CD172g-positive cells and/or CD26-positive cells in the cell population are each about 50% or more.

### (Item BCX11)

The method according to any one of the preceding items, in which a proportion of cells having the at least one characteristic in the cell population is about 60% or more.

### (Item BCX12)

The method according to any one of the preceding items, in which a proportion of cells having the at least one characteristic in the cell population is about 80% or more.

### (Item BCX12a)

The method according to any one of the preceding items, in which a proportion of FoxP3 strong-positive cells in the cell population is about 50% or more.

### (Item BCX12b)

The method according to any one of the preceding items, in which a proportion of stably FoxP3 strong-positive cells in the cell population is about 50% or more.

### (Item BCX13)

The method according to any one of the preceding items, in which the cell population contains about 90% or more T cells.

### (Item BCX14)

The method according to any one of the preceding items, in which the inducible regulatory human T cells are administered at about 10⁸ to about 10⁹ cells per dose or about 10⁷ cells/kg.

### (Item BCX15)

The method according to any one of the preceding items, in which the T cell-related disease includes an autoimmune disease, an infectious disease, cancer, an allergy, an inflammatory disease, and ALS, which are treatable by an immunosuppressive action.

### (Item BCX16)

The method according to any one of the preceding items, in which the autoimmune disease is selected from the group consisting of Addison's disease, alopecia areata, ankylosing spondylitis, autoimmune parotitis, Crohn's disease, diabetes (Type I), dystrophic epidermolysis bullosa, epididymitis, glomerulonephritis, Graves' disease, Guillain-Barré syndrome, Hashimoto's disease, hemolytic anemia, systemic lupus erythematosus, multiple sclerosis, myasthenia gravis, psoriasis, rheumatic fever, rheumatoid arthritis, sarcoidosis, scleroderma, Sjogren's syndrome, spondyloarthritis, thyroiditis, vasculitis, vitiligo, myxedema, pernicious anemia, cardiomyopathy, dilated cardiomyopathy (DCM), peripartum cardiomyopathy (PPCM), idiopathic cardiomyopathy, Chagas cardiomyopathy, Chagas megacolon, Chagas megaesophagus, Chagas neuropathy, benign prostatic hyperplasia, scleroderma, Raynaud's syndrome, preeclampsia, myocarditis, glaucoma, hypertension, pulmonary hypertension, malignant hypertension, pemphigus, Alzheimer's disease, systemic sclerosis, polymyositis, mixed connective tissue disease, antiphospholipid antibody syndrome, microscopic polyangiitis, granulomatosis with polyangiitis, eosinophilic granulomatosis with polyangiitis, crescentic glomerulonephritis, organ-specific autoimmune disease, Basedow's disease, autoimmune liver disease, primary sclerosing cholangitis (PSC), autoimmune hepatitis (AIH), primary biliary cholangitis, autoimmune pancreatitis, Goodpasture's syndrome, autoimmune hemolytic anemia, megaloblastic anemia, idiopathic thrombocytopenic purpura, primary sclerosing cholangitis, polyarteritis nodosa, Takayasu's arteritis, giant cell arteritis, polymyalgia rheumatica, adult-onset Still's disease, Behçet's disease, and ulcerative colitis.

### (Item BCX17)

The method according to any one of the preceding items, in which the inducible regulatory human T cell or cell population is administered by injection.

### (Item BCX18)

The method according to any one of the preceding items, in which when the inducible regulatory human T cell or cell population is administered to a patient and is ineffective or insufficient, the inducible regulatory human T cell or cell population is additionally administered to the patient.

### (Item BCX19)

The method according to any one of the preceding items, in which the inducible regulatory human T cell or cell population is additionally administered at least about 2 weeks after the initial administration.

In addition, the present disclosure also provides the following.

### (Item BC1)

A method for treating or preventing a T cell-related disease, the method including a step of administering, to a subject, an effective amount of inducible regulatory T cells having at least one characteristic selected from the group consisting of CTLA4-positive, NT5E-positive, ITGAE (CD103)-positive, and AREG-positive.

### (Item BC2)

The method according to the preceding item, in which the inducible regulatory T cells have at least two characteristics selected from the group consisting of CTLA4-positive, NT5E-positive, ITGAE (CD103)-positive, and AREG-positive.

### (Item BC3)

The method according to any one of the preceding items, in which the inducible regulatory T cells are at least CTLA4-positive.

### (Item BC4)

The method according to any one of the preceding items, in which the CNS2 region of the FOXP3 gene of the inducible regulatory T cell is demethylated.

### (Item BC5)

The method according to any one of the preceding items, in which the inducible regulatory T cells are CD4-positive or CD8-positive.

### (Item BC6)

The method according to any one of the preceding items, in which the inducible regulatory T cells are obtained from or derived from human peripheral blood T cells or human tissue-derived T cells.

### (Item BC7)

The method according to any one of the preceding items, in which the inducible regulatory T cells are obtained by a method including:
a step (a) of stimulating CD4-positive T cells or CD8-positive T cells in peripheral blood in a first basal medium for about 1 to about 5 days;
a step (b) of subjecting the cells obtained in the step (a) to a resting culture in a medium containing IL-2 for at least about 1 to about 3 days;
a step (c) of stimulating the cells obtained in the step (b) in a second basal medium for about 1 to about 5 days; and
a step (d) of subjecting the cells obtained in the step (c) to a resting culture in a medium containing IL-2 for at least about 1 to about 3 days.

### (Item BC8)

The method according to any one of the preceding items, in which a T cell population in which about 50% or more of the cells are the inducible regulatory T cells is contained.

### (Item BC9)

The method according to any one of the preceding items, in which a T cell population in which about 80% or more of the cells are the inducible regulatory T cells is contained.

### (Item BC10)

The method according to any one of the preceding items, in which the T cell population is a regulatory T cell population.

### (Item BC11)

The method according to any one of the preceding items, in which the cell population contains about 90% or more T cells.

### (Item BC12)

The method according to any one of the preceding items, in which the inducible regulatory T cells are contained so as to be administered at about 10⁸ to about 10⁹ cells per dose or about 10⁷ cells/kg.

### (Item BC13)

The method according to any one of the preceding items, in which the T cell-related disease includes an autoimmune disease, an infectious disease, cancer, an allergy, an inflammatory disease, and ALS, which are treatable by an immunosuppressive action.

### (Item BC14)

The method according to any one of the preceding items, in which the autoimmune disease is selected from the group consisting of Addison's disease, alopecia areata, ankylosing spondylitis, autoimmune parotitis, Crohn's disease, diabetes (Type I), dystrophic epidermolysis bullosa, epididymitis, glomerulonephritis, Graves' disease, Guillain-Barré syndrome, Hashimoto's disease, hemolytic anemia, systemic lupus erythematosus, multiple sclerosis, myasthenia gravis, psoriasis, rheumatic fever, rheumatoid arthritis, sarcoidosis, scleroderma, Sjogren's syndrome, spondyloarthritis, thyroiditis, vasculitis, vitiligo, myxedema, pernicious anemia, cardiomyopathy, dilated cardiomyopathy (DCM), peripartum cardiomyopathy (PPCM), idiopathic cardiomyopathy, Chagas cardiomyopathy, Chagas megacolon, Chagas megaesophagus, Chagas neuropathy, benign prostatic hyperplasia, scleroderma, Raynaud's syndrome, preeclampsia, myocarditis, glaucoma, hypertension, pulmonary hypertension, malignant hypertension, pemphigus, Alzheimer's disease, systemic sclerosis, polymyositis, mixed connective tissue disease, antiphospholipid antibody syndrome, microscopic polyangiitis, granulomatosis with polyangiitis, eosinophilic granulomatosis with polyangiitis, crescentic glomerulonephritis, organ-specific autoimmune disease, Basedow's disease, autoimmune liver disease, primary sclerosing cholangitis (PSC), autoimmune hepatitis (AIH), primary biliary cholangitis, autoimmune pancreatitis, Goodpasture's syndrome, autoimmune hemolytic anemia, megaloblastic anemia, idiopathic thrombocytopenic purpura, primary sclerosing cholangitis, polyarteritis nodosa, Takayasu's arteritis, giant cell arteritis, polymyalgia rheumatica, adult-onset Still's disease, Behçet's disease, and ulcerative colitis.

### (Item BC15)

The method according to any one of the preceding items, in which the inducible regulatory T cell or cell population is administered by injection.

### (Item BC16)

The method according to any one of the preceding items, in which when the inducible regulatory T cell or cell population is administered to a patient and is ineffective or insufficient, an additional administration is performed on the patient by the method.

### (Item BC17)

The method according to any one of the preceding items, in which the inducible regulatory T cell or cell population is additionally administered at least about 2 weeks after the initial administration.

The present disclosure also provides the following.

### (Item CX14)

Use of the inducible regulatory human T cell according to any one of the preceding items or the cell population according to any one of the preceding items for the manufacture of a medicament.

### (Item CX15)

Use of the inducible regulatory human T cell according to any one of the preceding items or the cell population according to any one of the preceding items for the manufacture of a regenerative medical material or product.

### (Item CCX1)

Use of an inducible regulatory human T cell having at least one characteristic selected from the group consisting of FoxP3-positive, CTLA4-positive, NT5E-positive, ITGAE (CD103)-positive, AREG-positive, CD172g-positive, and CD26-positive for the manufacture of a medicament for treating or preventing a T cell-related disease.

### (Item CCX1A)

Use of an NT5E-positive inducible regulatory human T cell for the manufacture of a medicament for treating or preventing a T cell-related disease.

### (Item CCX1B)

Use of an ITGAE (CD103)-positive inducible regulatory human T cell for the manufacture of a medicament for treating or preventing a T cell-related disease.

### (Item CCX1C)

Use of an AREG-positive inducible regulatory human T cell for the manufacture of a medicament for treating or preventing a T cell-related disease.

### (Item CCX1D)

Use of a CD172g-positive inducible regulatory human T cell for the manufacture of a medicament for treating or preventing a T cell-related disease.

### (Item CCX1E)

Use of a CD26-positive inducible regulatory human T cell for the manufacture of a medicament for treating or preventing a T cell-related disease.

### (Item CCX1F)

Use of a CTLA4-positive inducible regulatory human T cell for the manufacture of a medicament for treating or preventing a T cell-related disease.

### (Item CCX1G)

Use of a stably FoxP3-positive inducible regulatory human T cell for the manufacture of a medicament for treating or preventing a T cell-related disease.

### (Item CCX1G1)

Use of an inducible regulatory human T cell having at least two characteristics selected from the group consisting of CTLA4-positive, NT5E-positive, ITGAE (CD103)-positive, AREG-positive, CD172g-positive, and CD26-positive for the manufacture of a medicament for treating or preventing a T cell-related disease.

### (Item CCX1G2)

Use of an inducible regulatory human T cell having at least three characteristics selected from the group consisting of CTLA4-positive, NT5E-positive, ITGAE (CD103)-positive, AREG-positive, CD172g-positive, and CD26-positive for the manufacture of a medicament for treating or preventing a T cell-related disease.

### (Item CCX1G3)

Use of an inducible regulatory human T cell having at least four characteristics selected from the group consisting of CTLA4-positive, NT5E-positive, ITGAE (CD103)-positive, AREG-positive, CD172g-positive, and CD26-positive for the manufacture of a medicament for treating or preventing a T cell-related disease.

### (Item CCX1G4)

Use of an inducible regulatory human T cell having at least five characteristics selected from the group consisting of CTLA4-positive, NT5E-positive, ITGAE (CD103)-positive, AREG-positive, CD172g-positive, and CD26-positive for the manufacture of a medicament for treating or preventing a T cell-related disease.

### (Item CCX1G5)

Use of an inducible regulatory human T cell having all of CTLA4-positive, NT5E-positive, ITGAE (CD103)-positive, AREG-positive, CD172g-positive, and CD26-positive characteristics for the manufacture of a medicament for treating or preventing a T cell-related disease.

### (Item CCX2)

The use according to any one of the preceding items, in which the inducible regulatory human T cell is at least CTLA4-positive and FoxP3-positive.

### (Item CCX2a)

The use according to any one of the preceding items, in which the FoxP3 is stably expressed.

### (Item CCX2b)

The use according to any one of the preceding items, in which the FoxP3 is continuously expressed for at least about 48 hours or longer.

### (Item CCX2c)

The use according to any one of the preceding items, in which the FoxP3 is stably expressed at least from day 7 of culture.

### (Item CCX2d)

The use according to any one of the preceding items, in which the FoxP3 is expressed even after freeze-thawing of the cell.

### (Item CCX3)

The use according to any one of the preceding items, in which the inducible regulatory human T cell is at least CD172g-positive and/or CD26-positive.

### (Item CCX4)

The use according to any one of the preceding items, in which the CNS2 region of the FOXP3 gene of the inducible regulatory human T cell is demethylated.

### (Item CCX5)

The use according to any one of the preceding items, in which the inducible regulatory human T cells are CD4-positive or CD8-positive.

### (Item CCX6)

The use according to any one of the preceding items, in which the inducible regulatory human T cell is obtained from or derived from a human peripheral blood T cell or a human tissue-derived T cell.

### (Item CCX7)

The use according to any one of the preceding items, in which the inducible regulatory human T cell is obtained by a method including:
a step (a) of stimulating CD4-positive T cells or CD8-positive T cells in human peripheral blood in a first basal medium for about 1 to about 5 days;
a step (b) of subjecting the cells obtained in the step (a) to a resting culture in a medium containing IL-2 for at least about 1 to about 3 days;
a step (c) of stimulating the cells obtained in the step (b) in a second basal medium for about 1 to about 5 days; and
a step (d) of subjecting the cells obtained in the step (c) to a resting culture in a medium containing IL-2 for at least about 1 to about 3 days.

### (Item CCX8)

Use according to any one of the preceding items, in which the use is of a cell population containing the inducible regulatory human T cells for the manufacture of a medicament for treating or preventing a T cell-related disease, and the cell population has a proportion of about 50% or more of cells each having at least one characteristic selected from the group consisting of FoxP3-positive, CTLA4-positive, NT5E-positive, ITGAE (CD103)-positive, AREG-positive, CD172g-positive, and CD26-positive.

### (Item CCX8A)

Use of a cell population containing inducible regulatory human T cells for the manufacture of a medicament for treating or preventing a T cell-related disease, in which the cell population has a proportion of about 50% or more of NT5E-positive cells.

### (Item CCX8B)

Use of a cell population containing inducible regulatory human T cells for the manufacture of a medicament for treating or preventing a T cell-related disease, in which the cell population has a proportion of about 50% or more of ITGAE (CD103)-positive cells.

### (Item CCX8C)

Use of a cell population containing inducible regulatory human T cells for the manufacture of a medicament for treating or preventing a T cell-related disease, in which the cell population has a proportion of about 50% or more of AREG-positive cells.

### (Item CCX8D)

Use of a cell population containing inducible regulatory human T cells for the manufacture of a medicament for treating or preventing a T cell-related disease, in which the cell population has a proportion of about 50% or more of CD172g-positive cells.

### (Item CCX8E)

Use of a cell population containing inducible regulatory human T cells for the manufacture of a medicament for treating or preventing a T cell-related disease, in which the cell population has a proportion of about 50% or more of CD26-positive cells.

### (Item CCX8F)

A pharmaceutical composition containing a cell population containing inducible regulatory human T cells for use in the manufacture of a medicament for treating or preventing a T cell-related disease, in which the cell population has a proportion of about 50% or more of CTLA4-positive cells.

### (Item CCX8G)

Use of a cell population containing inducible regulatory human T cells for the manufacture of a medicament for treating or preventing a T cell-related disease, in which the cell population has a proportion of about 50% or more of stably FoxP3-positive cells.

### (Item CCX8G1)

Use of a cell population containing inducible regulatory human T cells for the manufacture of a medicament for treating or preventing a T cell-related disease, in which the cell population has a proportion of about 50% or more of cells each having at least two characteristics selected from the group consisting of CTLA4-positive, NT5E-positive, ITGAE (CD103)-positive, AREG-positive, CD172g-positive, and CD26-positive.

### (Item CCX8G2)

Use of a cell population containing inducible regulatory human T cells for the manufacture of a medicament for treating or preventing a T cell-related disease, in which the cell population has a proportion of about 50% or more of cells each having at least three characteristics selected from the group consisting of CTLA4-positive, NT5E-positive, ITGAE (CD103)-positive, AREG-positive, CD172g-positive, and CD26-positive.

### (Item CCX8G3)

Use of a cell population containing inducible regulatory human T cells for the manufacture of a medicament for treating or preventing a T cell-related disease, in which the cell population has a proportion of about 50% or more of cells each having at least four characteristics selected from the group consisting of CTLA4-positive, NT5E-positive, ITGAE (CD103)-positive, AREG-positive, CD172g-positive, and CD26-positive.

### (Item CCX8G4)

Use of a cell population containing inducible regulatory human T cells for the manufacture of a medicament for treating or preventing a T cell-related disease, in which the cell population has a proportion of about 50% or more of cells each having at least five characteristics selected from the group consisting of CTLA4-positive, NT5E-positive, ITGAE (CD103)-positive, AREG-positive, CD172g-positive, and CD26-positive.

### (Item CCX8G5)

Use of a cell population containing inducible regulatory human T cells for the manufacture of a medicament for treating or preventing a T cell-related disease, in which the cell population has a proportion of about 50% or more of cells each having all of CTLA4-positive, NT5E-positive, ITGAE (CD103)-positive, AREG-positive, CD172g-positive, and CD26-positive characteristics.

### (Item CCX9)

The use according to any one of the preceding items, in which proportions of at least the CTLA4-positive cells and FoxP3-positive cells in the cell population are each about 50% or more.

### (Item CCX9a)

The use according to any one of the preceding items, in which the FoxP3 is stably expressed.

### (Item CCX9b)

The use according to any one of the preceding items, in which the FoxP3 is continuously expressed for at least about 48 hours or longer.

### (Item CCX9c)

The use according to any one of the preceding items, in which the FoxP3 is stably expressed at least from day 7 of culture.

### (Item CCX9d)

The use according to any one of the preceding items, in which the FoxP3 is expressed even after freeze-thawing of the cell.

### (Item CCX10)

The use according to any one of the preceding items, in which proportions of at least the CD172g-positive cells and/or CD26-positive cells in the cell population are each about 50% or more.

### (Item CCX11)

The use according to any one of the preceding items, in which a proportion of cells having the at least one characteristic in the cell population is about 60% or more.

### (Item CCX12)

The use according to any one of the preceding items, in which a proportion of cells having the at least one characteristic in the cell population is about 80% or more.

### (Item CCX12a)

The use according to any one of the preceding items, in which a proportion of FoxP3 strong-positive cells in the cell population is about 50% or more.

### (Item CCX12b)

The use according to any one of the preceding items, in which a proportion of stably FoxP3 strong-positive cells in the cell population is about 50% or more.

### (Item CCX13)

The use according to any one of the preceding items, in which the cell population contains about 90% or more T cells.

### (Item CCX14)

The use according to any one of the preceding items, in which the inducible regulatory human T cells are contained so as to be administered at about 10⁸ to about 10⁹ cells per dose or about 10⁷ cells/kg.

### (Item CCX15)

The use according to any one of the preceding items, in which the T cell-related disease includes an autoimmune disease, an infectious disease, cancer, an allergy, an inflammatory disease, and ALS, which are treatable by an immunosuppressive action.

### (Item CCX16)

The use according to any one of the preceding items, in which the autoimmune disease is selected from the group consisting of Addison's disease, alopecia areata, ankylosing spondylitis, autoimmune parotitis, Crohn's disease, diabetes (Type I), dystrophic epidermolysis bullosa, epididymitis, glomerulonephritis, Graves' disease, Guillain-Barré syndrome, Hashimoto's disease, hemolytic anemia, systemic lupus erythematosus, multiple sclerosis, myasthenia gravis, psoriasis, rheumatic fever, rheumatoid arthritis, sarcoidosis, scleroderma, Sjogren's syndrome, spondyloarthritis, thyroiditis, vasculitis, vitiligo, myxedema, pernicious anemia, cardiomyopathy, dilated cardiomyopathy (DCM), peripartum cardiomyopathy (PPCM), idiopathic cardiomyopathy, Chagas cardiomyopathy, Chagas megacolon, Chagas megaesophagus, Chagas neuropathy, benign prostatic hyperplasia, scleroderma, Raynaud's syndrome, preeclampsia, myocarditis, glaucoma, hypertension, pulmonary hypertension, malignant hypertension, pemphigus, Alzheimer's disease, systemic sclerosis, polymyositis, mixed connective tissue disease, antiphospholipid antibody syndrome, microscopic polyangiitis, granulomatosis with polyangiitis, eosinophilic granulomatosis with polyangiitis, crescentic glomerulonephritis, organ-specific autoimmune disease, Basedow's disease, autoimmune liver disease, primary sclerosing cholangitis (PSC), autoimmune hepatitis (AIH), primary biliary cholangitis, autoimmune pancreatitis, Goodpasture's syndrome, autoimmune hemolytic anemia, megaloblastic anemia, idiopathic thrombocytopenic purpura, primary sclerosing cholangitis, polyarteritis nodosa, Takayasu's arteritis, giant cell arteritis, polymyalgia rheumatica, adult-onset Still's disease, Behçet's disease, and ulcerative colitis.

### (Item CCX17)

The use according to any one of the preceding items, in which the inducible regulatory human T cell or cell population is administered by injection.

### (Item CCX18)

The use according to any one of the preceding items, in which when the inducible regulatory human T cell or cell population is administered to a patient and is ineffective or insufficient, the inducible regulatory human T cell or cell population is additionally administered to the patient.

### (Item CCX19)

The use according to any one of the preceding items, in which the inducible regulatory human T cell or cell population is additionally administered at least about 2 weeks after the initial administration.

In addition, the present disclosure also provides the following.

### (Item CC1)

Use of an inducible regulatory T cell having at least one characteristic selected from the group consisting of CTLA4-positive, NT5E-positive, ITGAE (CD103)-positive, and AREG-positive for the manufacture of a medicament for treating or preventing a T cell-related disease.

### (Item CC2)

The use according to the preceding item, in which the inducible regulatory T cell has at least two characteristics selected from the group consisting of CTLA4-positive, NT5E-positive, ITGAE (CD103)-positive, and AREG-positive.

### (Item CC3)

The use according to any one of the preceding items, in which the inducible regulatory T cell is at least CTLA4-positive.

### (Item CC4)

The use according to any one of the preceding items, in which the CNS2 region of the FOXP3 gene of the inducible regulatory T cell is demethylated.

### (Item CC5)

The use according to any one of the preceding items, in which the inducible regulatory T cell is CD4-positive or CD8-positive.

### (Item CC6)

The use according to any one of the preceding items, in which the inducible regulatory T cell is obtained from or derived from a human peripheral blood T cell or a human tissue-derived T cell.

### (Item CC7)

The use according to any one of the preceding items, in which the inducible regulatory T cell is obtained by a method including:
a step (a) of stimulating CD4-positive T cells or CD8-positive T cells in peripheral blood in a first basal medium for about 1 to about 5 days;
a step (b) of subjecting the cells obtained in the step (a) to a resting culture in a medium containing IL-2 for at least about 1 to about 3 days;
a step (c) of stimulating the cells obtained in the step (b) in a second basal medium for about 1 to about 5 days; and
a step (d) of subjecting the cells obtained in the step (c) to a resting culture in a medium containing IL-2 for at least about 1 to about 3 days.

### (Item CC8)

The use according to any one of the preceding items, in which a T cell population in which about 50% or more of the cells are the inducible regulatory T cells is contained.

### (Item CC9)

The use according to any one of the preceding items, in which a T cell population in which about 80% or more of the cells are the inducible regulatory T cells is contained.

### (Item CC10)

The use according to any one of the preceding items, in which the T cell population is a regulatory T cell population.

### (Item CC11)

The use according to any one of the preceding items, in which the cell population contains about 90% or more T cells.

### (Item CC12)

The use according to any one of the preceding items, in which the inducible regulatory T cells are contained so as to be administered at about 10⁸ to about 10⁹ cells per dose or about 10⁷ cells/kg.

### (Item CC13)

The use according to any one of the preceding items, in which the T cell-related disease includes an autoimmune disease, an infectious disease, cancer, an allergy, an inflammatory disease, and ALS, which are treatable by an immunosuppressive action.

### (Item CC14)

The use according to any one of the preceding items, in which the autoimmune disease is selected from the group consisting of Addison's disease, alopecia areata, ankylosing spondylitis, autoimmune parotitis, Crohn's disease, diabetes (Type I), dystrophic epidermolysis bullosa, epididymitis, glomerulonephritis, Graves' disease, Guillain-Barré syndrome, Hashimoto's disease, hemolytic anemia, systemic lupus erythematosus, multiple sclerosis, myasthenia gravis, psoriasis, rheumatic fever, rheumatoid arthritis, sarcoidosis, scleroderma, Sjogren's syndrome, spondyloarthritis, thyroiditis, vasculitis, vitiligo, myxedema, pernicious anemia, cardiomyopathy, dilated cardiomyopathy (DCM), peripartum cardiomyopathy (PPCM), idiopathic cardiomyopathy, Chagas cardiomyopathy, Chagas megacolon, Chagas megaesophagus, Chagas neuropathy, benign prostatic hyperplasia, scleroderma, Raynaud's syndrome, preeclampsia, myocarditis, glaucoma, hypertension, pulmonary hypertension, malignant hypertension, pemphigus, Alzheimer's disease, systemic sclerosis, polymyositis, mixed connective tissue disease, antiphospholipid antibody syndrome, microscopic polyangiitis, granulomatosis with polyangiitis, eosinophilic granulomatosis with polyangiitis, crescentic glomerulonephritis, organ-specific autoimmune disease, Basedow's disease, autoimmune liver disease, primary sclerosing cholangitis (PSC), autoimmune hepatitis (AIH), primary biliary cholangitis, autoimmune pancreatitis, Goodpasture's syndrome, autoimmune hemolytic anemia, megaloblastic anemia, idiopathic thrombocytopenic purpura, primary sclerosing cholangitis, polyarteritis nodosa, Takayasu's arteritis, giant cell arteritis, polymyalgia rheumatica, adult-onset Still's disease, Behçet's disease, and ulcerative colitis.

### (Item CC15)

The use according to any one of the preceding items, in which the use involves administration by injection.

### (Item CC16)

The use according to any one of the preceding items, in which when the use involves administration to a patient and is ineffective or insufficient, the use additionally involves administration to the patient.

### (Item CC17)

The use according to any one of the preceding items, in which the use additionally involves administration at least about 2 weeks after the initial administration.

In the present disclosure, it is intended that the one or the plurality of features can be provided in further combination in addition to the specified combination. Note that still further embodiments and advantages of the present disclosure will be appreciated by those skilled in the art upon reading and understanding the following detailed description, if necessary.

Note that features and remarkable operations and effects of the present disclosure other than those described above will be apparent to those skilled in the art with reference to the following embodiments and drawings of the invention.

### Advantageous Effects of Invention

The present disclosure can provide a method for inducing regulatory T cells having high functionality from human peripheral T cells in vitro. The regulatory T cells induced by the method of the present disclosure highly express genes associated with regulatory T cells and have a strong suppressive ability. Therefore, the regulatory T cells can be used as regulatory T cells for treatment and prevention of various immune diseases, autoimmune diseases, and other inflammatory disorders.

Functional regulatory T cells can be induced in vitro from human peripheral T cells by the method of the present disclosure. The regulatory T cells obtained by the method of the present disclosure have functionality (for example, having a high suppressive function) and are stable as regulatory T cells. That is, by the method of the present disclosure, it is possible to induce regulatory T cells from human peripheral T cells, the regulatory T cells stably expressing FoxP3, which is a master gene of regulatory T cells, and having high functionality.

### [Brief Description of Drawings]

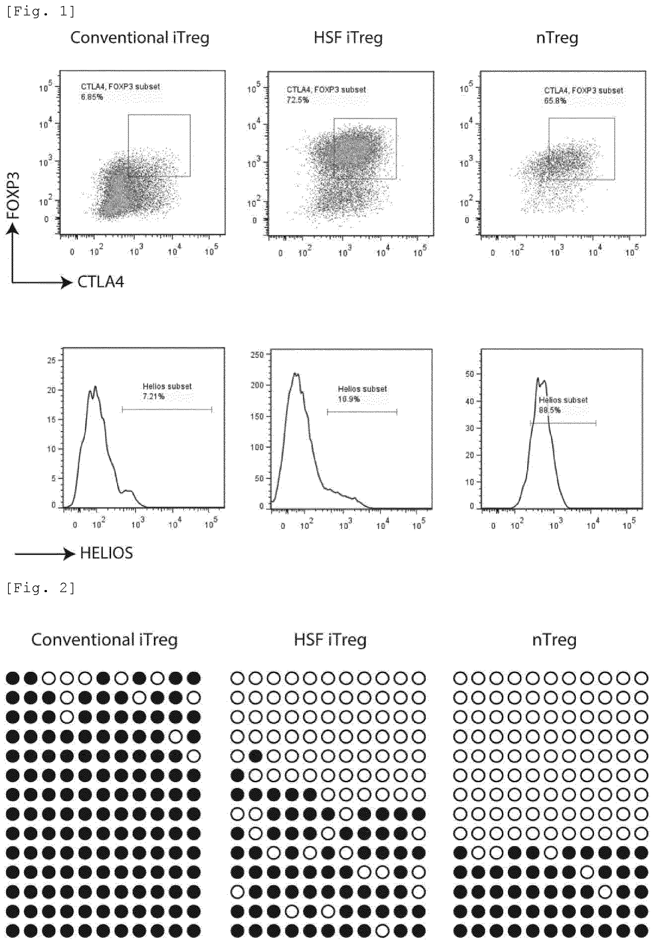

[FIG. 1] FIG. 1 is a diagram showing a phenotypic analysis (flow cytometry) of an embodiment of the inducible regulatory T cells of the present disclosure (also referred to as human highly functional and stable iTreg (HSF iTreg) cells). The inducible regulatory T cells of the present disclosure (HSF-iTregs in the drawing) were produced from human CD4-positive T cells, and the expression of FOXP3, CTLA4, and Helios was analyzed by flow cytometry. Cells obtained by stimulating conventional nTregs (CD4-positive CD25-positive T cells) and conventional iTregs (CD4-positive T cells stimulated with CD3/CD28 in the presence of IL-2 and TGF-β1 for 3 days) were compared.
[FIG. 2] FIG. 2 is a diagram showing an epigenomic analysis (bisulfite method) of an embodiment of the iTreg (HSF iTreg) cells of the present disclosure. The demethylation state of the FOXP3 CNS2 region in each cell shown in FIG. 1 was analyzed by the bisulfite method.

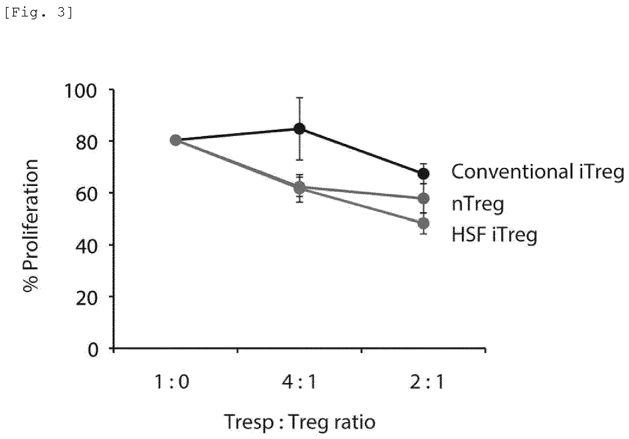

[FIG. 3] FIG. 3 is a diagram showing a suppressive ability analysis (in vitro suppression assay) of an embodiment of the iTreg (HSF iTreg) cells of the present disclosure. The in vitro suppressive ability of each cell shown in FIG. 1 was analyzed. Human CD4-positive T cells were stained with Cell Trace Violet reagent and co-cultured for 3 days at a constant ratio with each cell shown in FIG. 1 in the presence of Treg Suppression Inspector (Miltenyi Biotec) (5 µL/well), and the Cell Trace Violet intensity was analyzed by flow cytometry.

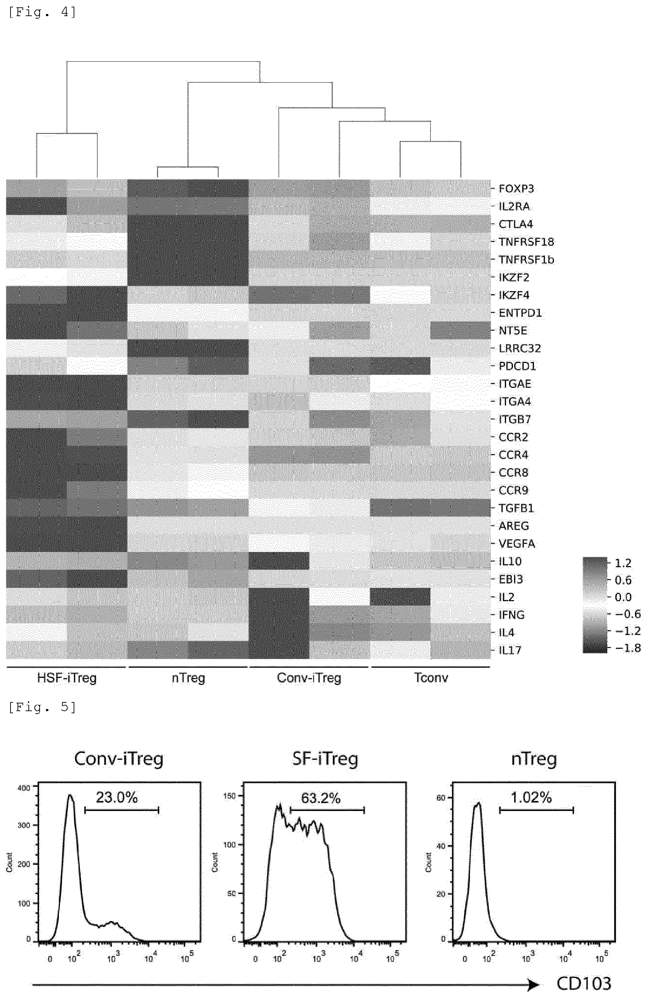

[FIG. 4] FIG. 4 is a diagram showing a gene expression pattern analysis (RAN-seq method) of an embodiment of the iTreg (HSF iTreg) cells of the present disclosure. Comprehensive gene expression patterns of each cell shown in FIG. 1 and FOXP3-negative cells (Tconv) stimulated from CD4-positive T cells in the presence of IL-2 were analyzed by RNA sequencing.
[FIG. 5] FIG. 5 is a diagram showing CD103 expression (flow cytometry) of an embodiment of the iTreg (HSF iTreg) cells of the present disclosure. CD103 expression in FOXP3-positive cells of each cell shown in FIG. 1 was analyzed by flow cytometry.

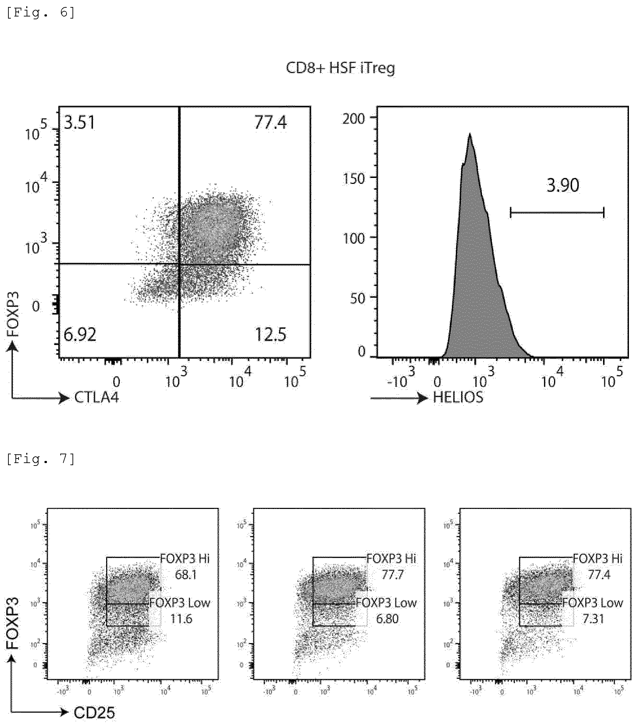

[FIG. 6] FIG. 6 is a diagram showing a phenotypic analysis (flow cytometry) of an embodiment of the iTreg (HSF iTreg) cells of the present disclosure produced from human CD8-positive T cells. The regulatory T cells of the present disclosure were produced from human CD8-positive T cells, and the expression of FOXP3, CTLA4, and Helios was analyzed by flow cytometry.
[FIG. 7] FIG. 7 is a diagram showing CD25 expression and FOXP3 expression (flow cytometry) of an embodiment of the iTreg (HSF iTreg) cells of the present disclosure. The expression intensity of each of CD25 and FOXP3 was analyzed by flow cytometry using a BD FACSLyric flow cytometer.

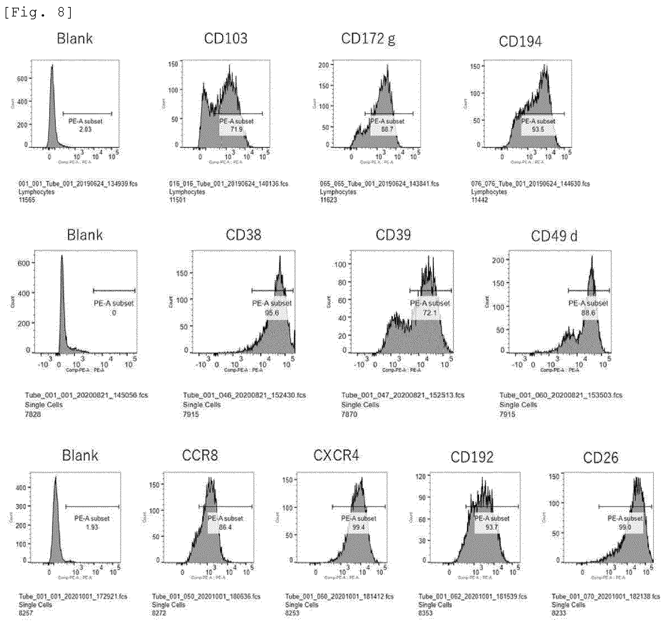

[FIG. 8] FIG. 8 is a diagram showing a phenotypic analysis of an embodiment of the iTreg (HSF iTreg) cells of the present disclosure. The inducible regulatory T cells (HSF-iTregs) of the present disclosure were produced, and the expression of CD172g, CD26, and the like was analyzed.

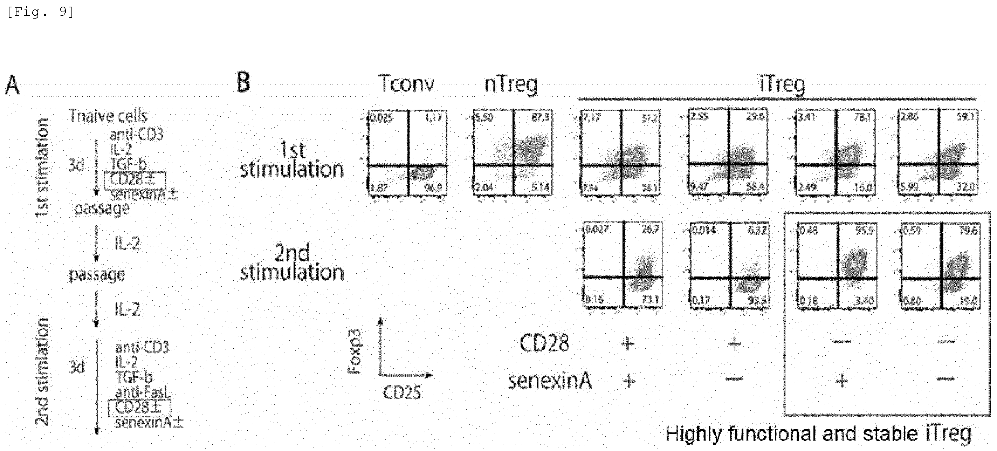

[FIG. 9] FIG. 9 shows an outline of a production method of an embodiment of mouse-inducible regulatory T cells (also referred to as mouse highly functional and stable iTreg (HSF iTreg) cells), and flow cytometry results of the inducible regulatory T cells. FIG. 9A shows a protocol outline when iTreg cells are induced from mouse CD4-positive naive T cells by various stimulation methods. FIG. 9B shows the results of flow cytometric analysis of FoxP3 and CD25 in iTreg cells produced in FIG. 9A, activated T cells, and activated nTreg cells.

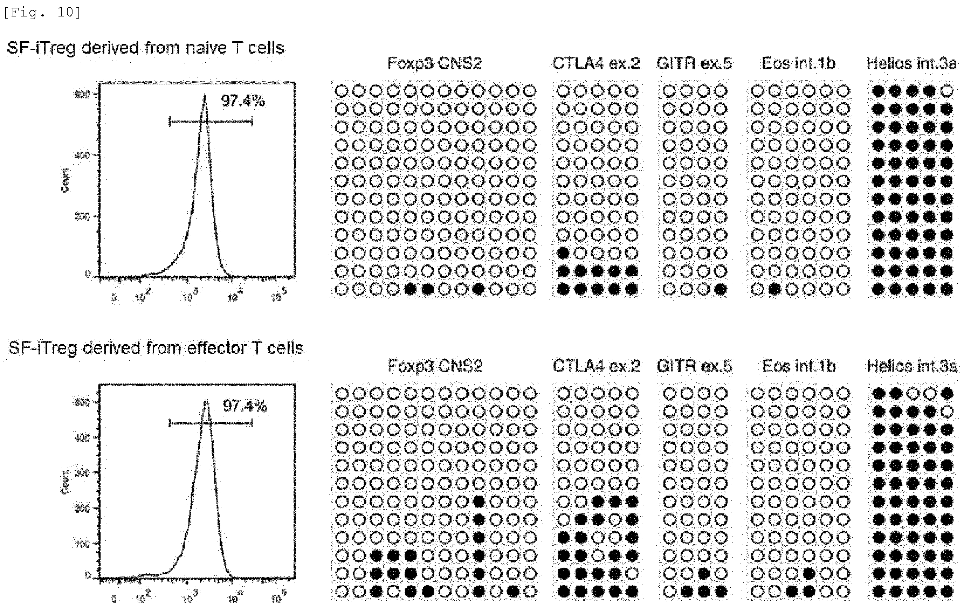

[FIG. 10] FIG. 10 is a diagram showing the results of respectively analyzing FoxP3 expression when SF-iTregs were produced from mouse CD4-positive naive T cells or effector T cells by flow cytometry and the demethylation state of the Treg-specific demethylated region by a bisulfite method on the basis of the production method according to an embodiment of iTregs (in the drawing, SF-iTregs) of the present disclosure in FIG. 9.

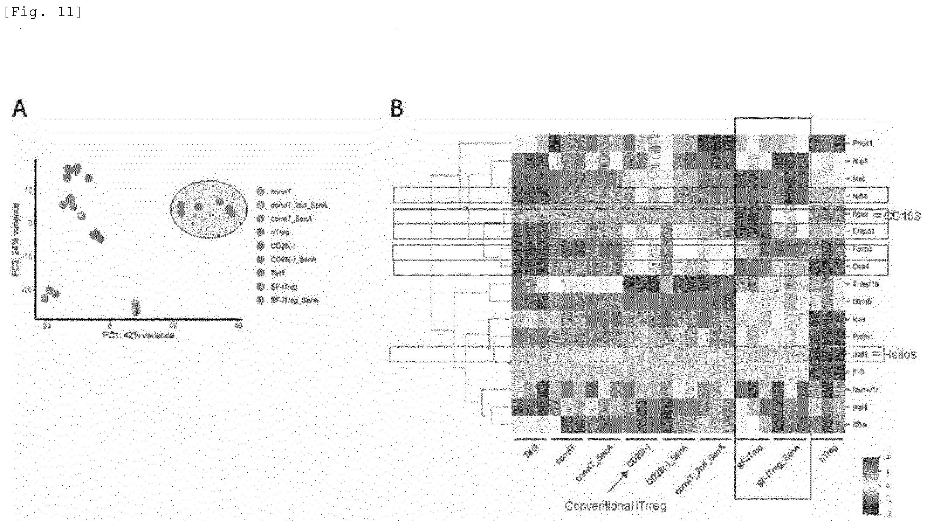

[FIG. 11] FIG. 11 shows the results of an embodiment in which comprehensive gene expression patterns of each cell in FIG. 9 were analyzed by RNA sequencing. FIG. 11A shows a PCA analysis plot, and FIG. 11B shows a heat map of Treg-related genes.

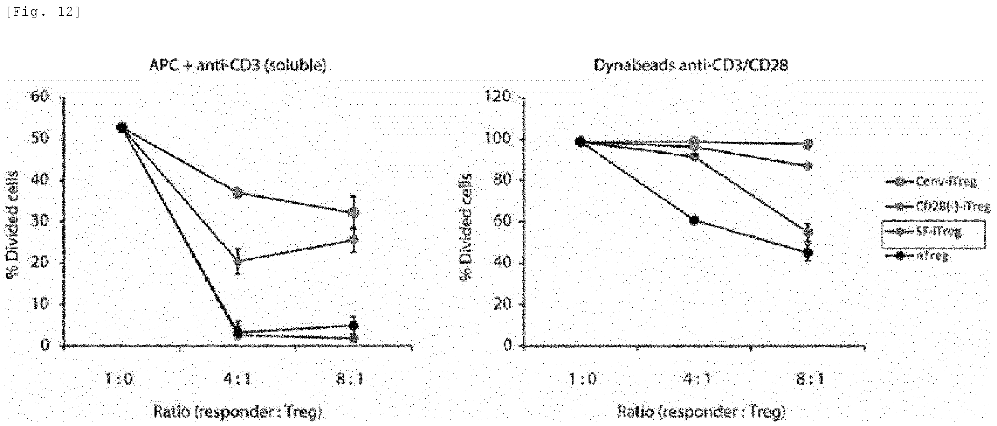

[FIG. 12] FIG. 12 shows the results of an embodiment in which the in vitro suppressive ability of each cell in FIG. 9 was analyzed. Mouse CD4-positive naive T cells were stained with Cell Trace Violet reagent and co-cultured for 3 days at a constant ratio with each cell shown in FIG. 9 in the presence of either anti-mouse CD3 antibody + MHC class II-positive cells or Dynabeads T-activator (Veritas) (5µL/well), and the Cell Trace Violet intensity was analyzed by flow cytometry.

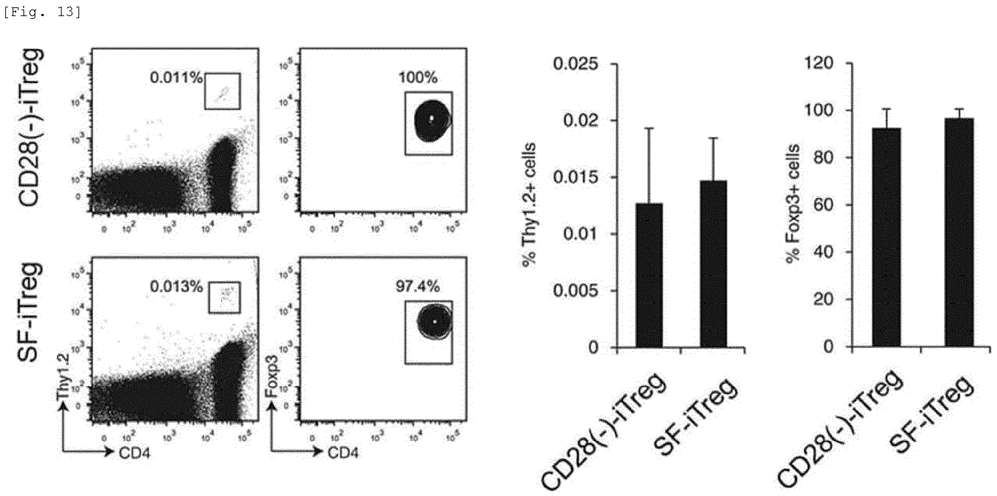

[FIG. 13] FIG. 13 shows the results of an embodiment in which regulatory T cells from the single-stimulation CD28 antibody-untreated group in FIG. 9 and the iTregs of the present disclosure were administered to wild-type mice, and the expression of Foxp3 in the transferred regulatory T cells was analyzed 2 weeks later.

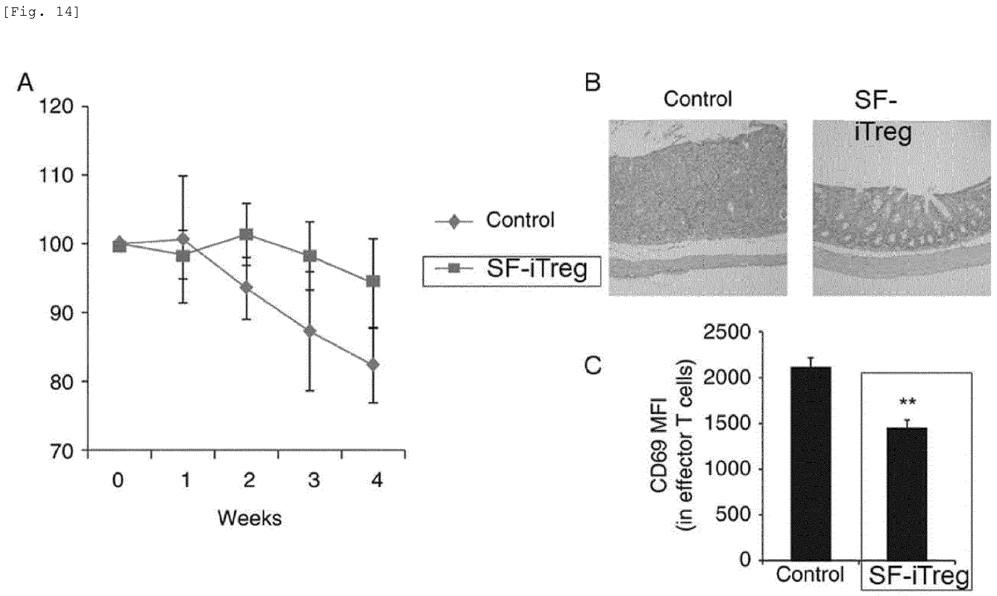

[FIG. 14] FIG. 14 shows the results of an embodiment in which a colitis model was established by transferring mouse CD4-positive naive T cells into RAG2-deficient mice, and therapeutic effects were analyzed after administration of the iTreg cells of the present disclosure. FIG. 14A shows changes in body weight (g), FIG. 14B shows HE-stained images of colon tissue, and FIG. 14C shows flow cytometry analysis results of CD69 expression in lymph node T cells.

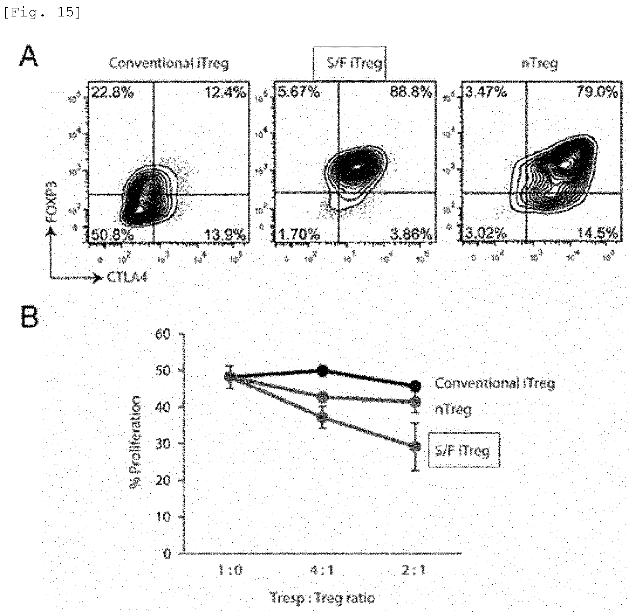

[FIG. 15] FIG. 15 is a diagram showing analysis results of an embodiment of the iTregs of the present disclosure derived from a patient with human Crohn's disease. FIG. 15A shows the results of flow cytometric analysis of FOXP3, CTLA4, and Helios expression in the inducible regulatory T cells of the present disclosure (referred to as HSF-iTregs in the drawing) produced from CD4-positive T cells derived from a patient with human Crohn's disease. Cells obtained by stimulating conventional nTregs (CD4-positive CD25-positive T cells) and conventional iTregs (CD4-positive T cells stimulated with CD3/CD28 in the presence of IL-2 and TGF-β1 for 3 days) were compared. FIG. 15B shows the results of in vitro suppressive ability analysis of each cell shown in FIG. 15A. Human CD4-positive T cells were stained with Cell Trace Violet reagent and co-cultured for 3 days at a constant ratio with each cell shown in FIG. 9 in the presence of Treg Suppression Inspector (Miltenyi Biotec) (5 µL/well), and the Cell Trace Violet intensity was analyzed by flow cytometry.

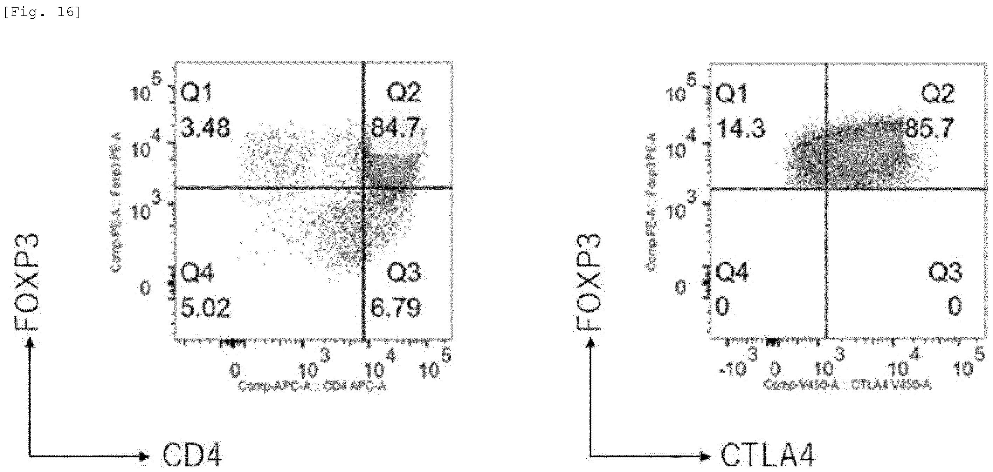

[FIG. 16] FIG. 16 is a diagram showing a phenotypic analysis (flow cytometry) of an embodiment of the iTreg (HSF iTreg) cells of the present disclosure produced from CD4-positive T cells derived from a patient with SLE. Highly functional inducible regulatory T cells were produced from CD4-positive T cells derived from a patient with SLE, and the expression of FoxP3, CD4, and CTLA4 was analyzed by flow cytometry.

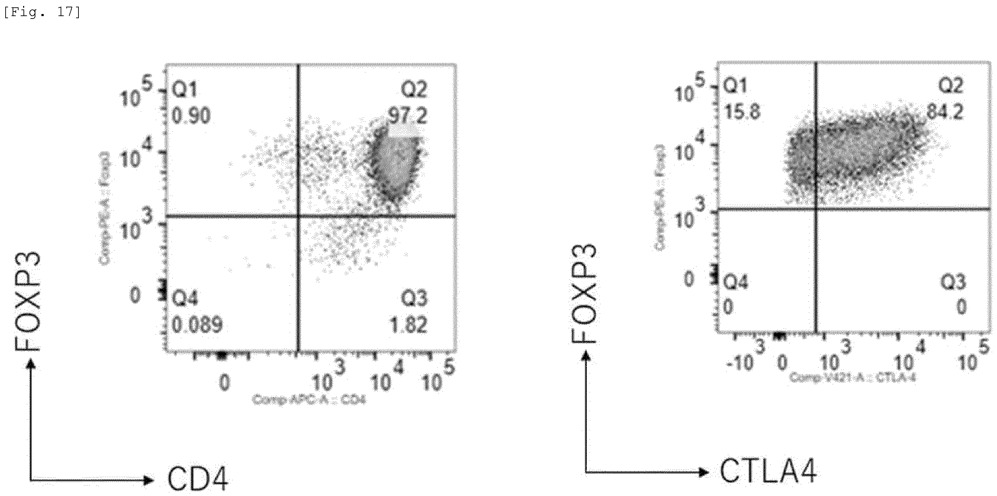

[FIG. 17] FIG. 17 is a diagram showing a phenotypic analysis (flow cytometry) of an embodiment of the iTreg (HSF iTreg) cells of the present disclosure produced from CD4-positive T cells derived from a patient with rheumatoid arthritis. Highly functional inducible regulatory T cells were produced from CD4-positive T cells derived from a patient with rheumatoid arthritis, and the expression of FoxP3, CD4, and CTLA4 was analyzed by flow cytometry.

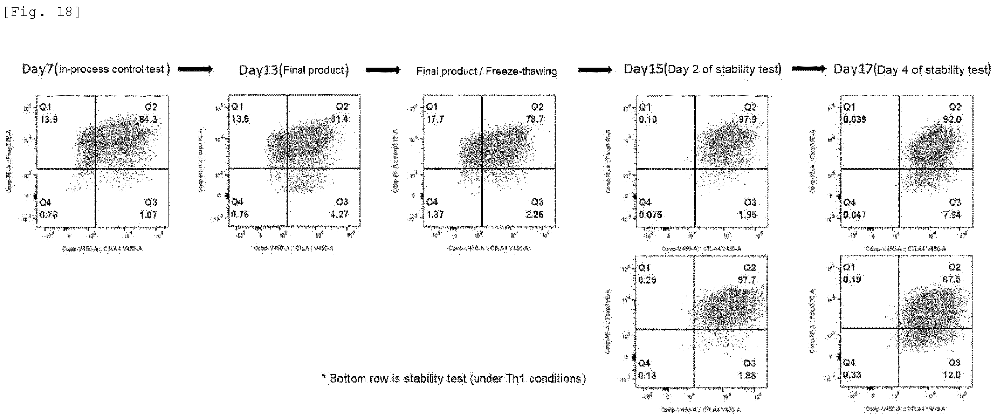

[FIG. 18] FIG. 18 shows that, in an embodiment of the present disclosure, the inducible regulatory human T cells in the cell population of the present disclosure stably express FOXP3 at all stages of the stability test, including the final product, after freeze-thawing, and after re-stimulation.

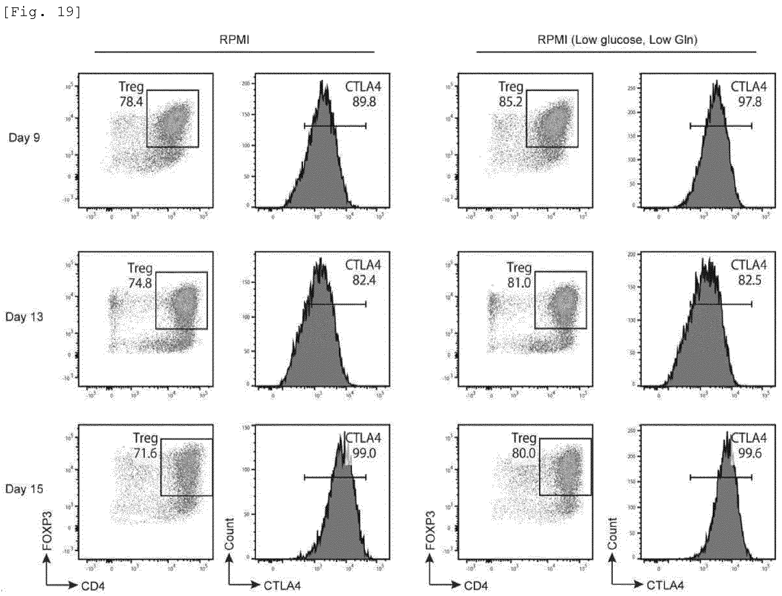

[FIG. 19] FIG. 19 is a diagram showing the results of an embodiment of the present disclosure in which the stability of FOXP3 expression was analyzed on day 9 (after the second stimulation), day 13 (final product), and day 15 (re-stimulation of the final product; example of a stability test), with respect to the medium conditions during the production of the inducible regulatory T cells in the cell population of the present disclosure when RPMI was used, when glucose was reduced to half in RPMI, and when glutamine was reduced to 10 to 50% in RPMI.

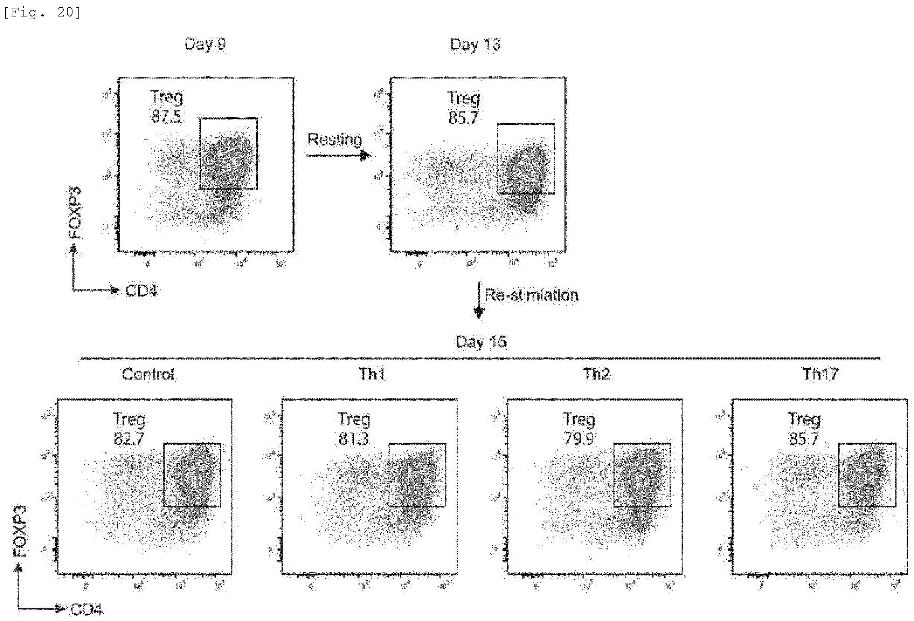

[FIG. 20] FIG. 20 is a diagram showing the results of an embodiment of the present disclosure in which the stability of FOXP3 expression in the inducible regulatory T cells of the present disclosure was analyzed after re-stimulation of the final product of the inducible regulatory T cells of the present disclosure when re-stimulated under a conventional re-stimulation condition (CD3/CD28 stimulation in RPMI + IL-2), or when re-stimulated with the addition of Th1 cytokines (IL-12 and IFN-γ), Th2 cytokines (IL-4 and IL-5), or Th17 cytokines (TGF-β, IL-6, IL-1β, and IL-23).

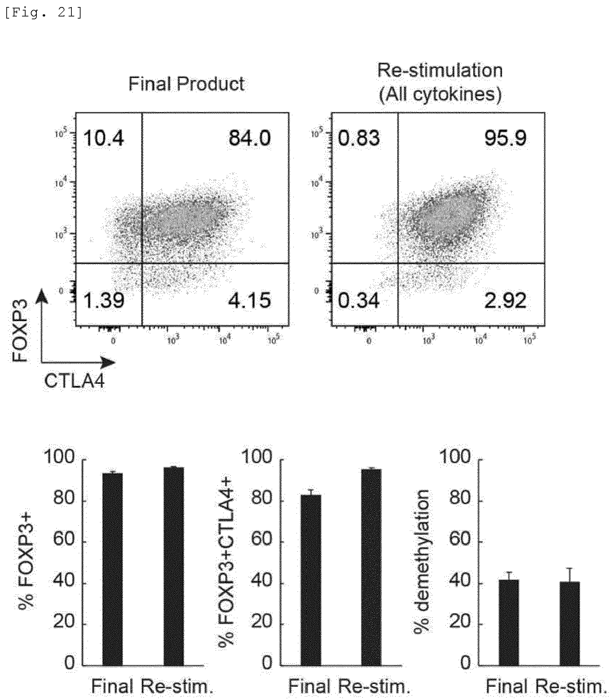

[FIG. 21] FIG. 21 is a diagram showing the results of an embodiment of the present disclosure in which, with respect to the final product of the inducible regulatory T cells of the present disclosure derived from female T cells, the expression of FoxP3 and CTLA4 in the inducible regulatory T cells of the present disclosure was analyzed by flow cytometry before re-stimulation, or after re-stimulation under a conventional re-stimulation condition (CD3/CD28 stimulation in RPMI + IL-2) with the addition of Th1 cytokines (IL-12 and IFN-γ), Th2 cytokines (IL-4 and IL-5), Th17 cytokines (TGF-β, IL-6, IL-1β, and IL-23), and TNF-α, the demethylation state was confirmed by bisulfite sequencing, and the stability of FOXP3 expression was analyzed.

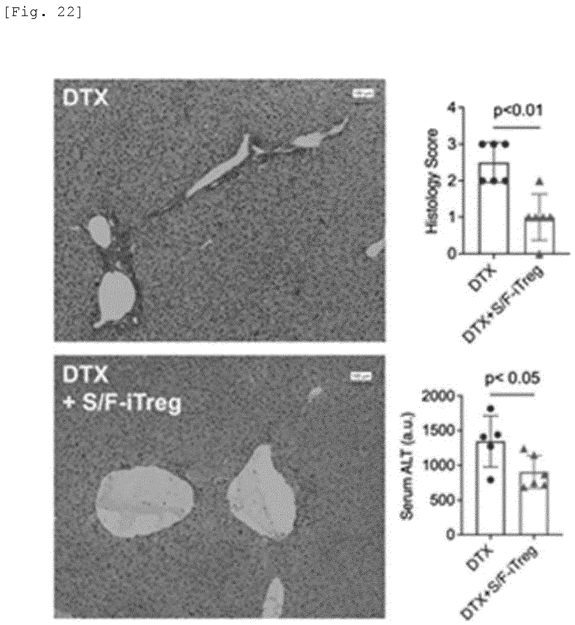

[FIG. 22] FIG. 22 shows an analysis using an autoimmune hepatitis model induced by Treg depletion through DTX administration. S/F-iTregs were administered simultaneously with induction, and liver H&E-stained images and serum ALT levels were analyzed 12 days later.

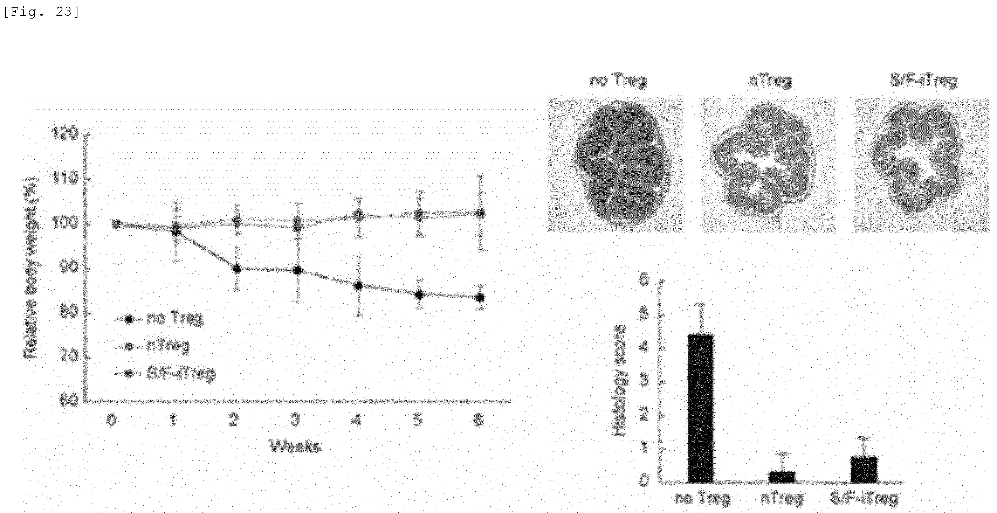

[FIG. 23] FIG. 23 shows the results using an autoimmune colitis model induced by administration of naive T cells into RAG-deficient mice. S/F-iTregs were administered simultaneously with induction, and changes in body weight for 6 weeks and colon H&E-stained images at the endpoint were analyzed.

### [Description of Embodiments]

Hereinafter, the present disclosure will be described while showing the best mode. Throughout herein, the expression of singular forms is to be understood as including the concept of plural forms unless otherwise stated. Therefore, the singular article (for example, "a", "an", "the", or the like in English) should be understood to include the concept of plural forms unless otherwise stated. In addition, the terms used herein are to be understood as being used in the sense commonly used in the art unless otherwise stated. Therefore, unless defined otherwise, all technical and scientific terms used herein have the same meaning as commonly understood by those skilled in the art to which the present disclosure pertains. In the case of conflict, the present specification (including definitions) will control.

Hereinafter, the definitions of the terms and/or basic technical concepts that are particularly used herein will be described as appropriate.

As used herein, the term "about" means ±10% of the subsequent numerical value. For example, "about 20" is intended to include "18 to 22". The range of numerical values includes all values between the two endpoints and the values of the endpoints. The "about" in relation to a range applies to both endpoints of the range. Accordingly, for example, "about 20 to 30" includes "18 to 33".

As used herein, when a gene name and a product thereof are indicated, they may refer to both a gene and a protein when expressed in all capital letters, unlike normal usage. For example, FOXP3 gene and FOXP3 protein may be referred to separately, and when expressed as FoxP3, it indicates both the concept and the actual entity (as a whole) of the gene or the protein.

As used herein, the term "regulatory T cell" refers to a T cell that is positive for FoxP3 expression. As used herein, it may also be referred to as "Treg". Tregs include endogenous regulatory T cells (naturally occurring regulatory T cells: nTregs), inducible regulatory T cells (iTregs), and the like. Regulatory T cells usually can have various functions (for example, immunosuppressive functions).

As used herein, the term "inducible regulatory T cell (iTreg)" refers to a regulatory T cell that is negative for IKZF2 (Helios) expression. iTregs are usually obtained by inducing differentiation from naive CD4-positive T cells or the like.

As used herein, the term "naturally occurring regulatory T cell (nTreg)" refers to a regulatory T cell that is positive for the expression of IKZF2 (Helios) and CTLA4. It is usually a cell present in vivo.

As used herein, the term "peripheral T cell" refers to a T cell present outside the thymus, which can be obtained from peripheral blood, lymph nodes, or other tissues. As used herein, the term "peripheral T cell" means that the cell population only needs to contain peripheral T cells, and it is not necessary for the T cells to be isolated. In addition to T cells, a cell fraction containing various lymphocytes, such as peripheral blood mononuclear cells (PBMCs), may be used.

As used herein, the term "flow cytometry" refers to a technique for measuring the number of particles and the individual physical, chemical, and biological properties of cells, organisms, and other biological particles suspended in a liquid. An apparatus that uses this technique is referred to as a "flow cytometer". In the present disclosure, "positive" or "negative" of cell markers (for example, FoxP3, CTLA4, Helios, CD103, and the like) is determined by flow cytometry, as commonly used in the art. More specifically, in flow cytometry, cells are aligned in a single row and flowed, and the number of cells is counted using a spectroscopic method. For example, the number of target cells is counted by irradiating cells labeled with fluorescent or luminescent enzymes with laser light and detecting fluorescence or luminescent signals emitted from the cells by a detector such as a photodiode. In addition, the detection results from the detector can be input into a computer to generate and display a two-dimensional plot. Therefore, the presence or absence of target cells, the number thereof, and the like can be easily determined.

As used herein, "demethylation" refers to removal of methyl modifications of adenine (for example, at the 6-position; m6A, or at the 1-position; m1A) or methylated cytosine (for example, at the 5-position; m5C, or at the 3-position; m3C). Demethylation can be specified using a technique known in the art, and can be measured using, for example, a bisulfite method.

As used herein, the "cell population" is a group containing two or more cells, and for example, may be in a state where cells are gathered in a planar manner, or may be a cell mass formed by cells adhering to each other in a three-dimensional manner. In addition, the "cell population" may be formed of a single type of cell or may contain a plurality of types of cells.

As used herein, the term "stimulation" means stimulation via a TCR. For example, stimulation of T cells includes stimulation using an anti-CD3 antibody and a complex containing the same, stimulation using a peptide that binds to a TCR and a complex containing the peptide, stimulation using an antigen-presenting cell, stimulation using an anti-CD3 antibody and an antigen-presenting cell simultaneously, stimulation using a peptide or a protein and an antigen-presenting cell simultaneously, and the like.

As used herein, the term "resting culture" refers to culturing cells in a state without the stimulation described above.

As used herein, the terms "stable", "stably expressed", or "stably" expressed refer to a state in which a cell marker (for example, FoxP3) is maintained in an expressed state for at least 48 hours at any time point under normal culture conditions (including after re-stimulation and freeze-thawing), in at least about 50% or more of the cells in a cell population, and preferably, continuously expressed at least from day 7 of culture. Whether the cell marker is stably expressed can be determined by a technique well known in the art such as flow cytometry.

### (Preferred embodiments)

Preferred embodiments of the present disclosure will be described below. The embodiments provided below are provided for a better understanding of the present disclosure, and the scope of the present disclosure should not be limited to the following description. Therefore, it is apparent that those skilled in the art can appropriately make modifications within the scope of the present disclosure in view of the description herein. In addition, the following embodiments of the present disclosure can be used alone or in combination.

### (Inducible regulatory T cells)

The present disclosure relates to a highly functional and stable inducible regulatory T cell (also referred to as a highly functional and stable iTreg or HSF iTreg) and use thereof. In addition, the present disclosure relates to a pharmaceutical application of a highly functional and stable inducible regulatory T cell (also referred to as a highly functional and stable iTreg or HSF iTreg).

In an aspect of the present disclosure, there is provided an inducible regulatory human T cell having at least one characteristic selected from the group consisting of FoxP3-positive, CTLA4-positive, NT5E-positive, ITGAE (CD103)-positive, AREG-positive, CD172g-positive, and CD26-positive. In an aspect of the present disclosure, there is provided a pharmaceutical composition for treating or preventing a T cell-related disease, the pharmaceutical composition containing, as an active ingredient, an inducible regulatory human T cell having at least one characteristic selected from the group consisting of FoxP3-positive, CTLA4-positive, NT5E-positive, ITGAE (CD103)-positive, AREG-positive, CD172g-positive, and CD26-positive. In an embodiment of the present disclosure, the inducible regulatory T cell of the present disclosure can have at least two characteristics selected from the group consisting of CTLA4-positive, NT5E-positive, ITGAE (CD103)-positive, and AREG-positive. In addition, in an embodiment of the present disclosure, the inducible regulatory T cell of the present disclosure may be at least CTLA4-positive. Without intending to be limiting, the inducible regulatory T cell of the present disclosure may be CD4-positive or CD8-positive.

In an embodiment of the present disclosure, the inducible regulatory T cell of the present disclosure may be at least CTLA4-positive and FoxP3-positive. In an embodiment of the present disclosure, the inducible regulatory T cell of the present disclosure may be at least CD172g-positive and/or CD26-positive.

The present disclosure provides a CD172g-positive inducible regulatory human T cell. In addition, the present disclosure provides a pharmaceutical composition for treating or preventing a T cell-related disease, the pharmaceutical composition containing, as an active ingredient, a CD172g-positive inducible regulatory human T cell. Since CD172g is a tyrosine kinase-associated protein involved in cell adhesion of neurons and the like (adhesion of cerebellar neurons, neurite outgrowth, and glial cell attachment), a CD172g-positive inducible regulatory human T cell is expected to have activated functions related to adhesion and to have a high effect on various diseases related thereto.

The present disclosure provides a CD26-positive inducible regulatory human T cell. In addition, the present disclosure provides a pharmaceutical composition for treating or preventing a T cell-related disease, the pharmaceutical composition containing, as an active ingredient, a CD26-positive inducible regulatory human T cell. Since CD26 is a gene also known as DPP4 and is associated with glucose metabolism, insulin metabolism, and immune function (remarkable also in infectious diseases), a CD26-positive inducible regulatory human T cell is expected to have a high effect on various diseases related thereto.

The present disclosure provides an NT5E-positive inducible regulatory human T cell. In addition, the present disclosure provides a pharmaceutical composition for treating or preventing a T cell-related disease, the pharmaceutical composition containing, as an active ingredient, an NT5E-positive inducible regulatory human T cell. NT5E (CD73) is a cell membrane protein that has a catalytic activity to convert extracellular nucleotides into membrane-permeable nucleosides. The encoded protein is used as a determinant of lymphocyte differentiation. Since a defect in this gene may cause calcification of joints or arteries, an NT5E-positive inducible regulatory human T cell is expected to be highly effective against various diseases related thereto.

The present disclosure provides an ITGAE (CD103)-positive inducible regulatory human T cell. In addition, the present disclosure provides a pharmaceutical composition for treating or preventing a T cell-related disease, the pharmaceutical composition containing, as an active ingredient, an ITGAE(CD103)-positive inducible regulatory human T cell. ITGAE (CD103) encodes an α-integrin with an I domain and undergoes post-translational cleavage in the extracellular domain to produce a disulfide-linked heavy chain and light chain. This protein binds to β7 integrin to form an E-cadherin-binding integrin known as human mucosal lymphocyte-1 antigen. This protein is preferentially expressed on human intestinal intraepithelial lymphocytes (IELs), and may function as an accessory molecule for IEL activation, in addition to its role of adhesion. Therefore, an ITGAE (CD103)-positive inducible regulatory human T cell is expected to have a high effect on various diseases related thereto.

The present disclosure provides an AREG-positive inducible regulatory human T cell. In addition, the present disclosure provides a pharmaceutical composition for treating or preventing a T cell-related disease, the pharmaceutical composition containing, as an active ingredient, an AREG-positive inducible regulatory human T cell. AREG is a member of the epidermal growth factor family and is associated with epidermal growth factor (EGF) and transforming growth factor-α (TGF-α). This protein interacts with the EGF/TGF-α receptor to promote the growth of normal epithelial cells and inhibit the growth of certain aggressive cancer cell lines. In addition, it also functions in the development of mammary glands, ovum, and bone tissue. This gene is associated with a skin phenotype similar to psoriasis and is also associated with other pathological diseases including various types of cancers and inflammatory states. Therefore, an AREG-positive inducible regulatory human T cell is expected to have a high effect on various diseases related thereto.

The present disclosure provides a CTLA4-positive inducible regulatory human T cell. In addition, the present disclosure provides a pharmaceutical composition for treating or preventing a T cell-related disease, the pharmaceutical composition containing, as an active ingredient, a CTLA4-positive inducible regulatory human T cell. CTLA4 is a protein that belongs to the immunoglobulin superfamily and transmits a suppressive signal to T cells. Membrane-bound CTLA4 functions as a homodimer linked by a disulfide bond, and soluble CTLA4 functions as a monomer. Mutations in this gene are said to be associated with insulin-dependent diabetes mellitus, Graves' disease, Hashimoto's thyroiditis, celiac disease, systemic lupus erythematosus, thyroid-associated orbitopathy, and other autoimmune diseases. Therefore, a CTLA4-positive inducible regulatory human T cell is expected to have a high effect on various diseases related thereto. In addition, CTLA4-positive inducible regulatory human T cells are expected to have a high effect on autoimmune diseases such as pemphigus, autoimmune liver disease (primary sclerosing cholangitis (PSC), autoimmune hepatitis (AIH), and the like), primary biliary cholangitis, and primary sclerosing cholangitis.

The expression of FoxP3 in the regulatory T cells can be induced in a test tube by culturing CD4-positive T cells in a medium containing IL2 and TGFβ in the presence of an anti-CD3 antibody and an anti-CD28 antibody, and then culturing the cells in a medium containing IL2 and TGFβ. In addition, although several methods for inducing regulatory T cells from peripheral T cells are known, expression of FoxP3 in inducible regulatory T cells is unstable, and the expression of many functional molecules other than FoxP3 has not been confirmed.

In recent years, a culture method for inducing DNA demethylation by a method in which ascorbic acid is added to a medium in inducible regulatory T cells (Kasahara et al., Int. Immunol. (2017) 29(10):457-469) and a method in which CD28 antibody stimulation is not used (Mikami et al., Proc Natl Acad Sci U S A. (2020) 117(22):12258-12268) has been developed. However, even in the inducible regulatory T cells produced by these methods, the expression of functional molecules has not been confirmed, and sufficient immunosuppressive activity has not been obtained. Also in clinical trials, one clinical trial using inducible regulatory T cells has been conducted for GVHD, but the proportion of regulatory T cells in the cells used was low, and efficacy such as prevention of GVHD has not been recognized at present (MacMillan et al., Blood Adv. (2021) 5(5):1425 - 1436).

In the present disclosure, it is possible to provide an inducible regulatory T cell in which the expression of FoxP3 is stable, and advantageously, an immunosuppressive action is stably maintained, and/or a pharmaceutical composition containing such an inducible regulatory T cell as an active ingredient, and such an inducible regulatory T cell can be produced, for example, by a method for producing inducible regulatory T cells as described elsewhere herein. For example, the present disclosure can provide an inducible regulatory T cell, in which the inducible regulatory T cell is obtained by a method including: a step (a) of stimulating CD4-positive T cells or CD8-positive T cells in human peripheral blood in a first basal medium for about 1 to about 5 days; a step (b) of subjecting the cells obtained in the step (a) to a resting culture in a medium containing IL-2 for at least about 1 to about 3 days; a step (c) of stimulating the cells obtained in the step (b) in a second basal medium for about 1 to about 5 days; and a step (d) of subjecting the cells obtained in the step (c) to a resting culture in a medium containing IL-2 for at least about 1 to about 3 days.

The inducible regulatory T cell produced in the present disclosure has an inducible regulatory T cell-specific demethylation state. The fact that the obtained regulatory T cells are in a regulatory T cell-specific demethylation state can be confirmed, for example, by the fact that the CNS2 region of the FoxP3 gene (FOXP3 (all in italics)) is demethylated. Since such a demethylation state can serve as an index of stability, the inducible regulatory T cell of the present disclosure can be shown to be a stable inducible regulatory T cell by confirming the demethylation state.

As used herein, the immunosuppressive activity or immunosuppressive action of the inducible regulatory T cell of the present disclosure can be confirmed, for example, by measuring Cell Trace Violet intensity in responder T cells. The inducible regulatory T cell of the present disclosure can stably provide an immunosuppressive activity or an immunosuppressive action, and, for example, the inducible regulatory T cell of the present disclosure can provide an immunosuppressive activity or an immunosuppressive action for at least about 2 weeks. As shown in Examples below, the inducible regulatory T cell of the present disclosure can have a higher immunosuppressive action than a conventional regulatory T cell (including both induced and naturally occurring regulatory T cells). Therefore, the inducible regulatory T cell of the present disclosure can also be referred to as a functional or highly functional inducible regulatory T cell.

In an embodiment, the inducible regulatory T cell of the present disclosure is capable of stably expressing FoxP3. Therefore, the inducible regulatory T cell of the present disclosure can also be referred to as a stable inducible regulatory T cell. The inducible regulatory T cell of the present disclosure is, in an aspect, highly functional and stable, can be referred to as a highly functional and stable inducible regulatory T cell (HSF iTreg).

In an embodiment of the present disclosure, whether or not markers in the inducible regulatory T cells of the present disclosure are positive can be determined by measuring a positivity rate using flow cytometry. For example, it is possible to determine whether or not the cells are positive based on a proportion of cells showing an antigen expression level equal to or higher than a reference standard by analyzing the cells with a flow cytometer. It can also be classified into negative, weakly positive, moderately positive, and strongly positive (weakly positive, moderately positive, and strongly positive collectively referred to as "positive") depending on the expression intensity of the cell surface markers. For example, depending on the setting of the instrument, a value of a median fluorescence intensity of each marker to a median fluorescence intensity of a negative control (stained with an isotype control antibody) can be classified as negative when less than 5, weakly positive when 5 or more and less than 10, moderately positive when 10 or more and less than 30, and strongly positive when 30 or more. Such determination can be made as exemplified in (Positivity Rate Measurement by Flow Cytometry), but the present disclosure is not limited thereto.

In an embodiment of the present disclosure, the expression intensity of cell surface markers in the inducible regulatory T cell of the present disclosure may be classified such that 10² or more is weakly positive, 10³ or more is moderately positive, and 10⁴ or more is strongly positive, 10³ or more is strongly positive and less than 10³ is weakly positive, or 10² or more is strongly positive and less than 10² is weakly positive, the expression intensity being determined, for example, based on analysis results obtained using a BD FACSLyric flow cytometer (BD Biosciences) using a sample prepared as described in Examples of the present application. Such determination can be appropriately set depending on experimental conditions, experimental purposes, the type of cells, instrument setting conditions, and the like, and the present disclosure is not limited thereto. In addition, even when analysis is performed using a flow cytometer other than the BD FACSLyric flow cytometer, it is possible to calculate the expression intensity on another instrument corresponding to the expression intensity measured by the BD FACSLyric flow cytometer, and the value indicating the expression intensity can be appropriately determined according to the instrument used.

In an embodiment of the present disclosure, the inducible regulatory T cell produced in the present disclosure can be derived from any cell, and preferably can be derived from a human peripheral blood T cell or a human tissue-derived T cell.

In an embodiment of the present disclosure, the inducible regulatory T cells of the present disclosure or the cell population thereof can target human cells, and can be inducible regulatory human T cells or a cell population thereof induced by a predetermined technique using T cells obtained from human peripheral blood as a material. The properties of human cells and mouse cells are clearly different, and even when the same cell surface markers are present, the properties of the cells cannot be considered to be the same. For example, in the field of inducible regulatory T cells, the techniques that can be applied to mice and humans are different, and in the case of mice, most lymphocytes are antigen-unprimed, whereas in the case of humans, T cells in peripheral blood exhibit diverse degrees of antigen sensitization and activation. Therefore, in mice, it is possible to directly produce highly functional and stable inducible regulatory T cells from T cells collected from mouse lymphoid tissue; however, in humans, this is difficult, and cells cannot be regarded as equivalent based solely on the presence of cell surface markers.

In an aspect of the present disclosure, there is provided a cell population including a cell population containing the inducible regulatory human T cells as described above, in which the cell population has a proportion of about 50% or more of cells having at least one characteristic selected from the group consisting of FoxP3-positive, CTLA4-positive, NT5E-positive, ITGAE (CD103)-positive, AREG-positive, CD172g-positive, and CD26-positive.

In another aspect of the present disclosure, there is provided a cell population containing inducible regulatory human T cells, in which the cell population has a proportion of about 50% or more of CTLA4-positive cells and FoxP3-positive cells. In another aspect of the present disclosure, there is provided a pharmaceutical composition for treating or preventing a T cell-related disease, the pharmaceutical composition containing, as an active ingredient, a cell population containing inducible regulatory human T cells, in which the cell population has proportions of about 50% or more of CTLA4-positive cells and FoxP3-positive cells. In an embodiment, the cell population of the present disclosure can have proportions of the CTLA4-positive cells and FoxP3-positive cells of about 60% or more, about 65% or more, about 70% or more, about 75% or more, about 80% or more, about 85% or more, about 90% or more, about 95% or more, about 97% or more, or about 99% or more.

In an embodiment of the present disclosure, in the cell population of the present disclosure, proportions of at least CD172g-positive cells and/or CD26-positive cells can each be about 50% or more. In an embodiment, the cell population of the present disclosure can have proportions of the CD172g-positive cells and/or CD26-positive cells of about 60% or more, about 65% or more, about 70% or more, about 75% or more, about 80% or more, about 85% or more, about 90% or more, about 95% or more, about 97% or more, or about 99% or more.

In an embodiment of the present disclosure, in the cell population of the present disclosure, a proportion of FoxP3 strong-positive cells can be about 50% or more. In an embodiment, the cell population of the present disclosure can have a proportion of the FoxP3 strong-positive cells of about 60% or more, about 65% or more, about 70% or more, about 75% or more, about 80% or more, about 85% or more, about 90% or more, about 95% or more, about 97% or more, or about 99% or more. Although not intended to be limiting, in such a cell population, measurement of the expression intensity of cell surface markers can be performed by measuring the positivity rate using flow cytometry as described above.

In another aspect of the present disclosure, there is provided a cell population containing inducible regulatory human T cells, in which the cell population has a proportion of about 50% or more of FoxP3-positive cells. In another aspect of the present disclosure, there is provided a pharmaceutical composition for treating or preventing a T cell-related disease, the pharmaceutical composition containing, as an active ingredient, a cell population containing inducible regulatory human T cells, in which the cell population has a proportion of about 50% or more of FoxP3-positive cells. Although not intended to be limiting, in an embodiment, such a cell population can contain FoxP3-positive inducible regulatory human T cells in about 50% or more, about 60% or more, about 65% or more, about 70% or more, about 75% or more, about 80% or more, about 85% or more, about 90% or more, about 95% or more, about 97% or more, or about 99% or more on a cell count basis.

In another aspect of the present disclosure, there is provided a cell population containing inducible regulatory human T cells, in which the cell population has a proportion of about 50% or more of CTLA4-positive cells. In another aspect of the present disclosure, there is provided a pharmaceutical composition for treating or preventing a T cell-related disease, the pharmaceutical composition containing, as an active ingredient, a cell population containing inducible regulatory human T cells, in which the cell population has a proportion of about 50% or more of CTLA4-positive cells. Although not intended to be limiting, in an embodiment, such a cell population can contain CTLA4-positive inducible regulatory human T cells in about 50% or more, about 60% or more, about 65% or more, about 70% or more, about 75% or more, about 80% or more, about 85% or more, about 90% or more, about 95% or more, about 97% or more, or about 99% or more on a cell count basis.

In another aspect of the present disclosure, there is provided a cell population containing inducible regulatory human T cells, in which the cell population has a proportion of about 50% or more of NT5E-positive cells. In another aspect of the present disclosure, there is provided a pharmaceutical composition for treating or preventing a T cell-related disease, the pharmaceutical composition containing, as an active ingredient, a cell population containing inducible regulatory human T cells, in which the cell population has a proportion of about 50% or more of NT5E-positive cells. Although not intended to be limiting, in an embodiment, such a cell population can contain NT5E-positive inducible regulatory human T cells in about 50% or more, about 60% or more, about 65% or more, about 70% or more, about 75% or more, about 80% or more, about 85% or more, about 90% or more, about 95% or more, about 97% or more, or about 99% or more on a cell count basis.

In another aspect of the present disclosure, there is provided a cell population containing inducible regulatory human T cells, in which the cell population has a proportion of about 50% or more of ITGAE (CD103)-positive cells. In another aspect of the present disclosure, there is provided a pharmaceutical composition for treating or preventing a T cell-related disease, the pharmaceutical composition containing, as an active ingredient, a cell population containing inducible regulatory human T cells, in which the cell population has a proportion of about 50% or more of ITGAE (CD103)-positive cells. Although not intended to be limiting, in an embodiment, such a cell population can contain ITGAE (CD103)-positive inducible regulatory human T cells in about 50% or more, about 60% or more, about 65% or more, about 70% or more, about 75% or more, about 80% or more, about 85% or more, about 90% or more, about 95% or more, about 97% or more, or about 99% or more on a cell count basis.

In another aspect of the present disclosure, there is provided a cell population containing inducible regulatory human T cells, in which the cell population has a proportion of about 50% or more of AREG-positive cells. In another aspect of the present disclosure, there is provided a pharmaceutical composition for treating or preventing a T cell-related disease, the pharmaceutical composition containing, as an active ingredient, a cell population containing inducible regulatory human T cells, in which the cell population has a proportion of about 50% or more of AREG-positive cells. Although not intended to be limiting, in an embodiment, such a cell population can contain AREG-positive inducible regulatory human T cells in about 50% or more, about 60% or more, about 65% or more, about 70% or more, about 75% or more, about 80% or more, about 85% or more, about 90% or more, about 95% or more, about 97% or more, or about 99% or more on a cell count basis.

In another aspect of the present disclosure, there is provided a cell population containing inducible regulatory human T cells, in which the cell population has a proportion of about 50% or more of CD172g-positive cells. In another aspect of the present disclosure, there is provided a pharmaceutical composition for treating or preventing a T cell-related disease, the pharmaceutical composition containing, as an active ingredient, a cell population containing inducible regulatory human T cells, in which the cell population has a proportion of about 50% or more of CD172g-positive cells. Although not intended to be limiting, in an embodiment, such a cell population can contain CD172g-positive inducible regulatory human T cells in about 50% or more, about 60% or more, about 65% or more, about 70% or more, about 75% or more, about 80% or more, about 85% or more, about 90% or more, about 95% or more, about 97% or more, or about 99% or more on a cell count basis.

In another aspect of the present disclosure, there is provided a cell population containing inducible regulatory human T cells, in which the cell population has a proportion of about 50% or more of CD26-positive cells. In another aspect of the present disclosure, there is provided a pharmaceutical composition for treating or preventing a T cell-related disease, the pharmaceutical composition containing, as an active ingredient, a cell population containing inducible regulatory human T cells, in which the cell population has a proportion of about 50% or more of CD26-positive cells. Although not intended to be limiting, in an embodiment, such a cell population can contain CD26-positive inducible regulatory human T cells in about 50% or more, about 60% or more, about 65% or more, about 70% or more, about 75% or more, about 80% or more, about 85% or more, about 90% or more, about 95% or more, about 97% or more, or about 99% or more on a cell count basis.

As described above, the properties of human cells and mouse cells are clearly different from each other, and therefore, even under the same induction conditions, the proportion of induction of specific cells differs between mouse cells and human cells. In the present disclosure, when induction is performed by a predetermined induction method, it is possible to obtain a cell population in which a proportion of positive cells for a predetermined cell surface marker is about 50% or more, and preferably to obtain a cell population in which "proportions of CTLA4-positive cells and FoxP3-positive cells are each about 50% or more".

CTLA4 is a molecule localized within a cell, and is not at a level that can be used, at least, as an index for enrichment or purification. In addition, since FoxP3 is a transcription factor and is entirely an intracellular molecule, FoxP3 cannot be detected on the surface at all and cannot be used for purification. That is, since CTLA4 and FoxP3 are markers expressed "within" T cells, it is not possible to obtain a cell population in which CTLA4-positive cells and/or FoxP3-positive cells account for about 50% or more by sorting and enriching using CTLA4 and/or FoxP3 as indices. Therefore, it is also not possible, according to conventional methods, to obtain a cell population in which proportions of CTLA4-positive cells and FoxP3-positive cells are each less than about 50%, and then, using such a cell population as starting material, to enrich by sorting with CTLA4 and/or FoxP3 as indices to obtain a cell population in which these cells each account for about 50% or more.

In an embodiment of the present disclosure, the inducible regulatory human T cells of the present invention mainly serve to exhibit the immunosuppressive function of Tregs, and when these cells account for more than half in a certain cell population, a medically effective immunosuppressive effect can be stably achieved, which is also important for technical and medical effects. That is, by containing 50% or more of T cells that exhibit a medically effective immunosuppressive effect, a stable cell preparation can be provided. Therefore, this effect is an extremely important point in medical practice, and is important not only as a difference in amount or numerical values but also as a quality issue regarding whether the preparation can be properly established.

In an embodiment of the present disclosure, in the cell population of the present disclosure, the inducible regulatory human T cells can further have characteristics of being ITGAE (CD103)-positive, NT5E-positive, and/or AREG-positive. These cell markers are genes that play an important role in immunosuppressive function, and since these markers are highly expressed, it is possible to maintain greater functional stability after administration of cells compared to conventional inducible regulatory T cells. For example, CD103 is important for migration of regulatory T cells to an inflammation site, NT5E (CD73) is important for production of adenosine having immunosuppressive ability, and AREG is important for tissue regeneration by regulatory T cells. The cell population of the present disclosure can achieve the effect of maintaining functional stability due to the high expression of these markers.

In an embodiment of the present disclosure, the T cells in the cell population of the present disclosure can be regulatory T cells. In this case, in the regulatory T cells of the cell population of the present disclosure, about 60% or more, about 65% or more, about 70% or more, about 75% or more, about 80% or more, about 85% or more, about 90% or more, about 95% or more, about 97% or more, or about 99% or more can be inducible regulatory T cells as described elsewhere herein.

In an embodiment of the present disclosure, the cell population of the present disclosure may contain cells other than T cells, but preferably about 60% or more, about 65% or more, about 70% or more, about 75% or more, about 80% or more, about 85% or more, about 90% or more, about 95% or more, about 97% or more, or about 99% or more of the cell population of the present disclosure are T cells, and more preferably about 90% or more are T cells.

### (Stability)

In an embodiment of the present disclosure, the inducible regulatory T cells in the cell population of the present disclosure are capable of stably expressing FOXP3 even with continued long-term culture. The inducible regulatory T cells in the cell population of the present disclosure are capable of expressing FOXP3 in at least about 50% or more of the cells in the cell population at any time (including after re-stimulation and freeze-thawing) under normal culture conditions, for example, for at least about 48 hours or longer, about 60 hours or longer, about 72 hours or longer, about 84 hours or longer, about 96 hours or longer, about 108 hours or longer, about 120 hours or longer, about 132 hours or longer, about 144 hours or longer, about 156 hours or longer, about 168 hours or longer, or about 180 hours or longer. In another embodiment, inducible regulatory T cells in a cell population of the present disclosure are capable of expressing FOXP3 at least from day 7 of culture. For example, the inducible regulatory T cells in the cell population of the present disclosure can stably express FOXP3 in cells of an intermediate product on day 3 of culture, day 4 of culture, day 5 of culture, day 6 of culture, day 7 of culture, day 8 of culture, day 9 of culture, day 10 of culture, and the like as an in-process control test. In addition, in an embodiment, the inducible regulatory T cells in the cell population of the present disclosure are stably FOXP3-positive cells, and can stably express FOXP3 even when thawed after cryopreservation, and can stably express FOXP3 even when cells that can be the final product (for example, cells at day 13 of culture) are freeze-thawed (see, for example, FIG. 18). In addition, in an embodiment of the present disclosure, the inducible regulatory T cells in the cell population of the present disclosure can stably express FOXP3 even when the cells that can be the final product (for example, cells at day 13 of culture) with or without freeze-thawing are re-stimulated and cultured (including the Th1 environment), and for example, after the cells that can be the final product (for example, cells at day 13 of culture) are re-stimulated, the inducible regulatory T cells can stably express FOXP3 also in cells on day 14 of culture (day 1 of stability test), day 15 of culture (day 2 of stability test), day 16 of culture (day 3 of stability test), day 17 of culture (day 4 of stability test), day 18 of culture (day 5 of stability test), and the like (see, for example, FIG. 18). In another embodiment, the inducible regulatory T cells in the cell population of the present disclosure can express FOXP3 for at least 3, 4, 5, 6, 7, 8, 9, 10, 11, 12, 13, 14, 15, 16, 16, 17, 18, 19, 20 days of culture. In addition, in still another embodiment, the inducible regulatory T cells in the cell population of the present disclosure are also capable of expressing FOXP3 when freeze-thawed for at least about 1 month, about 2 months, about 3 months, about 4 months, about 5 months, or about 6 months. Since the cells are stably FOXP3-positive cells, the cells can be stably used as a cell preparation.

In a preferred embodiment, the cell population or inducible regulatory T cells of the present disclosure can stably express at least one marker selected from the group consisting of CTLA4, NT5E, ITGAE (CD103), AREG, CD172g, and CD26 in addition to FoxP3, and can preferably be stably CTLA4 positive cells.

### (Pharmaceutical composition)

In an aspect of the present disclosure, a pharmaceutical composition containing the inducible regulatory T cells or the cell population of the present disclosure is provided. In addition, in another aspect, a regenerative medical material or product containing the inducible regulatory T cells or the cell population of the present disclosure is provided. This pharmaceutical composition or regenerative medical material or product can be used in autoimmune diseases, inflammatory diseases, and allergies, which can be treated by the immunosuppressive action. These medicaments and regenerative medical materials or products can be used together with media or any other additive used in the art. As such a medium, a medium obtained by adding necessary factors to a basal medium for animal cell culture used for culturing cells can be used. Examples of such media are described in detail elsewhere herein. Examples of components added to the medium are also described in detail elsewhere herein. When provided as such a product, DMSO or the like may be included.

In an embodiment, the pharmaceutical composition of the present disclosure can be used for treating or preventing a T cell-related disease, the T cell-related disease is not particularly limited as long as it is a disease that can be treated by an immunosuppressive action, and examples of the T cell-related disease include an autoimmune disease, an infectious disease, cancer, an allergy, an inflammatory disease, and ALS. In addition, in an embodiment, examples of the autoimmune disease includes, but are not limited to, Addison's disease, alopecia areata, ankylosing spondylitis, autoimmune parotitis, Crohn's disease, diabetes (Type I), dystrophic epidermolysis bullosa, epididymitis, glomerulonephritis, Graves' disease, Guillain-Barré syndrome, Hashimoto's disease, hemolytic anemia, systemic lupus erythematosus, multiple sclerosis, myasthenia gravis, psoriasis, rheumatic fever, rheumatoid arthritis, sarcoidosis, scleroderma, Sjogren's syndrome, spondyloarthritis, thyroiditis, vasculitis, vitiligo, myxedema, pernicious anemia, cardiomyopathy, dilated cardiomyopathy (DCM), peripartum cardiomyopathy (PPCM), idiopathic cardiomyopathy, Chagas cardiomyopathy, Chagas megacolon, Chagas megaesophagus, Chagas neuropathy, benign prostatic hyperplasia, scleroderma, Raynaud's syndrome, preeclampsia, myocarditis, glaucoma, hypertension, pulmonary hypertension, malignant hypertension, pemphigus, Alzheimer's disease, systemic sclerosis, polymyositis, mixed connective tissue disease, antiphospholipid antibody syndrome, microscopic polyangiitis, granulomatosis with polyangiitis, eosinophilic granulomatosis with polyangiitis, crescentic glomerulonephritis, organ-specific autoimmune disease, Basedow's disease, autoimmune liver disease, primary sclerosing cholangitis (PSC), autoimmune hepatitis (AIH), primary biliary cholangitis, autoimmune pancreatitis, Goodpasture's syndrome, autoimmune hemolytic anemia, megaloblastic anemia, idiopathic thrombocytopenic purpura, primary sclerosing cholangitis, polyarteritis nodosa, Takayasu's arteritis, giant cell arteritis, polymyalgia rheumatica, adult-onset Still's disease, Behçet's disease, and ulcerative colitis. The autoimmune disease is preferably pemphigus, autoimmune liver disease (primary sclerosing cholangitis (PSC), autoimmune hepatitis (AIH), or the like), primary biliary cholangitis, or primary sclerosing cholangitis, more preferably pemphigus, or autoimmune hepatitis, and still more preferably autoimmune hepatitis.

In an embodiment, the pharmaceutical composition of the present disclosure can be administered by various routes of administration and dosages, but is preferably administered by injection. In an embodiment, the pharmaceutical composition of the present disclosure can contain inducible regulatory T cells as described elsewhere As used herein, such that the inducible regulatory T cells are administered at about 10⁸ to about 10⁹ cells per dose or about 10⁷ cells/kg.

In an embodiment, when the pharmaceutical composition of the present disclosure is administered to a patient and is ineffective or insufficient, the pharmaceutical composition is additionally administered to the patient. Whether the effect is absent or insufficient can be determined, for example, by using the degree of suppression of inflammation in the target disease as an index. In an embodiment, in the case of additional administration, it can be administered at least about one week, about 2 weeks, about 3 weeks, or about 4 weeks after the initial administration.

### (Production method)

The present disclosure provides a method for producing inducible regulatory T cells of the present disclosure. The method of the present disclosure is a novel method capable of producing highly functional and stable inducible regulatory T cells, and is described in detail below herein.

In an aspect of the present disclosure, there is provided a method for producing inducible regulatory T cells, the method including: a step (a) of stimulating CD4-positive T cells or CD8-positive T cells in human peripheral blood in a first basal medium for about 1 to about 5 days; a step (b) of subjecting the cells obtained in the step (a) to a resting culture in a medium containing IL-2 for at least about 1 to about 3 days; a step (c) of stimulating the cells obtained in the step (b) in a second basal medium for about 1 to about 5 days; and a step (d) of subjecting the cells obtained in the step (c) to a resting culture in a medium containing IL-2 for at least about 1 to about 3 days. In the present disclosure, the inducible regulatory T cells of the present disclosure can be produced using any of CD4-positive T cells and CD8-positive T cells as starting material, and in an embodiment, the inducible regulatory T cells of the present disclosure can also be produced using mixed cells of CD4-positive T cells and CD8-positive T cells as starting material.

In an embodiment, the method of the present disclosure can also be a method for producing regulatory T cells from human peripheral T cells (including CD4-positive T cells or CD8-positive T cells), the method including: a step of culturing human peripheral T cells in a medium containing TGFβ and IL-2 in the presence of anti-CD3 antibody stimulation; a step of culturing the cells in a medium containing IL-2 in the absence of anti-CD3 antibody; and a step of culturing the cells again in a medium containing TGFβ and IL-2 in the presence of anti-CD3 antibody stimulation.

In an embodiment, the stimulation of T cells refers to the stimulation for obtaining Tregs, and is not particularly limited as long as it is stimulation via the TCR. For example, stimulation of T cells can include stimulation using an anti-CD3 antibody and a complex containing the same, stimulation using a peptide that binds to a TCR and a complex containing the peptide, stimulation using an antigen-presenting cell, stimulation using an anti-CD3 antibody and an antigen-presenting cell simultaneously, stimulation using a peptide or a protein and an antigen-presenting cell simultaneously, and the like, but the medium and conditions are not particularly limited as long as Tregs can be obtained.

In an embodiment, the medium and conditions for the resting culture are not particularly limited as long as the cells are cultured in a state without stimulation as described above.

As used herein, the term "anti-CD3 antibody stimulation" means that stimulation specific to a CD3 receptor on a cell is provided. Examples of the CD3 stimulation include an anti-CD3 agonist antibody. The anti-CD3 agonist antibody may be prepared by using a commercially available product as a research reagent or may be prepared by a conventional method. As the anti-CD3 antibody, for example, an antibody derived from an animal such as a mouse, a rabbit, a goat, or a cow, and an antibody derived from a human can be used.

In an embodiment, in the method of the present disclosure, once iTregs are induced (T cells are stimulated to induce demethylation) (step a), the cells are recovered by replacing the medium and performing a resting culture for 1 to 3 days (step b), and then, the T cells are stimulated again to induce demethylation (step c), thereby obtaining inducible regulatory human T cells having functionality and stability. It is common technical knowledge that T cells exhibit apoptosis when T cells are generally stimulated twice; however, in the method of the present disclosure, by performing a "resting culture" and a "second stimulation", it has been found that inducible regulatory human T cells having functionality and stability can be obtained without exhibiting apoptosis.

That is, in the method of the present disclosure, since the inducible regulatory human T cells having functionality and stability can be obtained by performing T cell stimulation, a resting culture, and subsequent re-stimulation, the desired effect of the present disclosure can be achieved as long as the inducible regulatory human T cells obtained in this manner or a cell population containing such inducible regulatory human T cells is used. Therefore, in an embodiment of the present disclosure, the medium for culture and the type of stimulation are not particularly limited, and by using a medium having any composition and any type of stimulation, it is possible to obtain inducible regulatory human T cells having functionality and stability.

In an embodiment, the medium used in each step may further contain retinoic acid and/or ascorbic acid. Preferably, the medium can contain ascorbic acid. In an embodiment, the medium may further contain a CDK8 inhibitor, a CDK19 inhibitor, and/or a CDK8/19 inhibitor. Preferably, the medium can contain a CDK8 inhibitor, a CDK19 inhibitor, and/or a CDK8/19 inhibitor. Since the CDK8 inhibitor, the CDK19 inhibitor, and the CDK8/19 inhibitor may have overlapping functions, in the present disclosure, the description of the CDK8 inhibitor, the CDK19 inhibitor, or the CDK8/19 inhibitor may be used, and in such a case, at least one selected from the group consisting of a CDK8 inhibitor, a CDK19 inhibitor, and a CDK8/19 inhibitor is selected.

In an embodiment of the present disclosure, the first basal medium and the second basal medium can each independently contain at least one, at least two, at least three, at least four, at least five, or all of the factors selected from the group consisting of an anti-CD3 antibody, TGF-β (for example, TGF-β1, TGF-β2, TGFβ-3, or the like), IL-2, retinoic acid, a CDK8 inhibitor, a CDK19 inhibitor, or a CDK8/19 inhibitor (at least one selected from the group consisting of a CDK8 inhibitor, a CDK19 inhibitor, and a CDK8/19 inhibitor), and ascorbic acid. In addition, in another embodiment, the first basal medium and the second basal medium can each independently contain an anti-CD3 antibody, TGF-β1, IL-2, retinoic acid, a CDK8 inhibitor, a CDK19 inhibitor, or a CDK8/19 inhibitor (at least one selected from the group consisting of a CDK8 inhibitor, a CDK19 inhibitor, and a CDK8/19 inhibitor), and ascorbic acid. The concentration of these respective components can be normal concentrations used in the art. In an embodiment, the concentrations of the CDK8 inhibitor, the CDK19 inhibitor, and/or the CDK8/19 inhibitor that may be used may be any suitable concentrations that may be used in the art, and for example, when Senexin A is used, the concentration may be about 0.1 µM or more, about 0.5 µM or more, about 1 µM or more, about 2 µM or more, about 3 µM or more, about 4 µM or more, about 5 µM or more, about 6 µM or more, about 7 µM or more, about 8 µM or more, about 9 µM or more, about 10 µM or more, about 12 µM or more, about 14 µM or more, about 16 µM or more, about 18 µM or more, about 20 µM or more, and the like, but is not limited to these concentrations, and can be appropriately changed by those skilled in the art depending on other medium compositions.

In an embodiment of the disclosure, the first basal medium used in the present disclosure contains at least one selected from the group consisting of an anti-CD3 antibody, TGF-β, IL-2, retinoic acid, and a CDK8 inhibitor, a CDK19 inhibitor, and a CDK8/19 inhibitor, and/or the second basal medium contains at least one selected from the group consisting of an anti-CD3 antibody, TGF-β, IL-2, retinoic acid, ascorbic acid, and a CDK8 inhibitor, a CDK19 inhibitor, and a CDK8/19 inhibitor.

In a preferred embodiment, at least one selected from the group consisting of a CDK8 inhibitor, a CDK19 inhibitor, and a CDK8/19 inhibitor contained in the first basal medium includes Senexin A or AS2863619. In another preferred embodiment, at least one selected from the group consisting of a CDK8 inhibitor, a CDK19 inhibitor, and a CDK8/19 inhibitor contained in the second basal medium includes Senexin A or AS2863619.

In another preferred embodiment, TGF-β contained in the first basal medium includes TGF-β1 and/or TGF-β3. In another preferred embodiment, TGF-β contained in the second basal medium includes TGF-β1 and/or TGF-β3.

In another preferred embodiment, TGF-β contained in the first basal medium includes TGF-β1.

In another preferred embodiment, TGF-β contained in the second basal medium includes TGF-β1.

In another preferred embodiment, the first basal medium contains an anti-CD3 antibody, TGF-β1, IL-2, retinoic acid, and Selexin A.

In another preferred embodiment, the second basal medium contains an anti-CD3 antibody, TGF-β1, IL-2, retinoic acid, Selexin A, and ascorbic acid.

In another preferred embodiment, the first basal medium contains an anti-CD3 antibody, TGF-β3, IL-2, retinoic acid, and Selexin A.

In another preferred embodiment, the second basal medium contains an anti-CD3 antibody, TGF-β3, IL-2, retinoic acid, Selexin A, and ascorbic acid.

In an embodiment, the method of the present disclosure produces regulatory T cells from human peripheral T cells. The peripheral T cells include naive regulatory T cells, CD4-positive T cells, CD8-positive T cells, and the like. The regulatory T cells may be induced from a culture containing a plurality of types of T cells, or the regulatory T cells may be induced after specific cells such as CD4-positive T cells and CD8-positive T cells are isolated from these cells. In addition, regulatory T cells may be induced after T cells specific to a specific antigen are isolated. Thus, inducible regulatory T cells of the present disclosure include CD4-positive cells and CD8-positive cells. Note that, As used herein, the term "CD4-positive" or "CD4+" refers to a CD4-positive CD8-negative single-positive cell, unless otherwise specified. In addition, in the production method in the present disclosure, either CD4-positive or CD8-positive T cells can be used as starting material, and even after generation of inducible regulatory T cells, the CD4-positive or CD8-positive characteristics can be maintained unless special manipulation is performed.

In an embodiment, in the method of the present disclosure, the antibody may be added to the medium, or may be immobilized on an inner wall of a culture vessel or a surface of an insoluble carrier. The insoluble carrier can be a member capable of physically or chemically binding an anti-CD3 antibody, and can be insoluble in an aqueous solution. Examples of a material capable of physically adsorbing the anti-CD3 antibody include synthetic resins such as polystyrene, polyethylene terephthalate, polycarbonate, and polypropylene, and glass. The shape of the insoluble carrier is not particularly limited, and for example, a plate shape, a bead shape, a container shape, or the like can be adopted. The amount of the anti-CD3 antibody may vary depending on the titer or origin of the antibody used, but may be appropriately set so as to provide sufficient stimulation for induction of regulatory T cells.

In an embodiment, in the method of the present disclosure, a medium obtained by adding necessary factors to a basal medium for animal cell culture can be used for culturing the cells. Examples of the basal medium for animal cell culture that can be used in the method of the present disclosure include Iscove's modified Eagle's Medium, Ham's F12 medium, MEM Zinc Option medium, IMEM Zinc Option medium, IMDM medium, Medium 199, Eagle's Minimum Essential Medium (EMEM), αMEM medium, Dulbecco's modified Eagle's Medium (DMEM), RPMI 1640 medium, Fischer's medium, and a mixed medium or a medium partially modified in composition thereof.

The basal medium may contain serum (for example, fetal bovine serum (FBS)) or may be serum-free. The serum-free medium may optionally contain one or more serum substitutes, such as albumin, bovine serum albumin (BSA), transferrin, apotransferrin, KnockOut Serum Replacement (KSR) (a serum substitute for ES cell culture) (Thermo Fisher Scientific), N2 supplement (Thermo Fisher Scientific), B27 supplement (Thermo Fisher Scientific), fatty acids, insulin, collagen precursors, trace elements, 2-mercaptoethanol, 3'-thiolglycerol, and monothioglycerol. The basal medium may also contain one or more substances such as lipids (for example, chemically defined lipid concentrate), amino acids, L-glutamine, GlutaMAX (Thermo Fisher Scientific), non-essential amino acids (NEAA), vitamins (for example, nicotinamide, ascorbic acid), growth factors, antibiotics (for example, penicillin and streptomycin), antioxidants, pyruvate, buffers, inorganic salts, and equivalents thereof.

In one embodiment, as an example of the basal medium, RPMI 1640 medium containing serum and HEPES (4-(2-hydroxyethyl)-1-piperazine ethanesulfonic acid) may be mentioned.

In the method of the present disclosure, the cells may be cultured under general animal cell culture conditions. A culture temperature is, but is not limited to, about 30 to 40°C, and is preferably about 37°C. The culture is preferably performed in an atmosphere of air containing CO₂, and the CO₂ concentration is preferably about 2 to 5%.

In the method of the present disclosure, the anti-CD3 antibody may be directly added to the medium, or an antibody immobilized on an inner wall of a culture vessel or on a surface of an insoluble carrier may be used. The amount of the anti-CD3 antibody may vary depending on the titer or origin of the antibody used, but may be appropriately set so as to provide sufficient stimulation for induction of regulatory T cells.

Examples of TGFβ used include TGFβ1, TGFβ2, and TGFβ3, and, for example, TGFβ1 may be used. The concentration of TGFβ may be appropriately determined by those skilled in the art, and is not particularly limited. When TGFβ1 or TGFβ3 is used as TGFβ, the concentration in the medium is not particularly limited, and may be 0.25 to 25 ng/mL, for example, about 10 ng/mL.

The concentration of IL-2 in the medium used is not limited, and can be about 5 U/mL to about 500 U/mL, for example, about 100 U/mL.

Retinoic acid and/or ascorbic acid may be further added to the medium used in the present disclosure. Preferably, the medium contains ascorbic acid. The concentration of ascorbic acid is not limited, and is about 1 to about 100 µg/mL, for example, about 10 µg/mL.

A CDK8 inhibitor, a CDK19 inhibitor, and/or a CDK8/19 inhibitor may be further added to the medium used in the present disclosure. Any CDK8 inhibitor, CDK19 inhibitor, and/or CDK8/19 inhibitor can be used in the present disclosure, and examples thereof include 4-[1-(2-methyl-1H-benzimidazol-5-yl)-1H-imidazo[4,5-c]pyridin-2-yl]-1,2,5-oxadiazol-3-amine, 3-{1-[1-(4-methoxyphenyl)piperidin-4-yl]-4-methyl-1H-imidazo[4,5-c]pyridin-2-yl}pyrazin-2-amine, or salts, hydrates, solvates thereof, and compounds described in US 8,598,344, WO 2013/001310 A, WO 2013/040153 A, WO 2013/116786 A, WO 2014/029726 A, WO 2014/063778 A, WO 2014/072435 A, WO 2014/090692 A, WO 2014/106606 A, WO 2014/123900 A, WO 2014/154723 A, WO 2014/194245 A, WO 2015/049325 A, WO 2015/100420 A, WO 2015/144290 A, WO 2015/159937 A, WO 2015/159938 A, WO 2016/009076 A, or WO 2018/139660 A. One example includes Senexin A or AS2863619, but the present disclosure is not limited thereto. The concentration of the CDK8 inhibitor, the CDK19 inhibitor, and/or the CDK8/19 inhibitor that may be used may be any suitable concentration that may be used in the art, and may be any suitable concentration described in the above literature, and can be appropriately changed by those skilled in the art according to other medium compositions.

In the method of the present disclosure, regulatory T cells can be induced by stimulating human peripheral T cells with an anti-CD3 antibody in a medium containing TGFβ and IL-2, and regulatory T cells having a high immunosuppressive function can be induced by stimulating the cells again with an anti-CD3 antibody after a resting culture in an IL-2-containing medium that does not contain an anti-CD3 antibody. It can be confirmed that the inducible regulatory T cells obtained have a high immunosuppressive function, for example, by comprehensive gene expression analysis using RNA sequencing, or by an in vitro cell proliferation inhibition assay.

In an embodiment of the present disclosure, the number of culture days in the step (a) of stimulating CD4-positive T cells or CD8-positive T cells in peripheral blood in a first basal medium for about 1 to about 5 days can be appropriately set by those skilled in the art, and is not particularly limited, but can be, for example, about 3 days.

In addition, in an embodiment, the number of culture days in the resting culture step in the step (b) can be appropriately set by those skilled in the art, and is not particularly limited, but can be, for example, about 2 days.

In addition, in an embodiment, the number of culture days in the culture step in the second basal medium in the step (c) can be appropriately set by those skilled in the art, and is not particularly limited, but can be, for example, about 3 days.

In addition, in an embodiment, the number of culture days in the resting culture step in step (d) can be appropriately set by those skilled in the art, and is not particularly limited, but can be, for example, about 2 days. In an embodiment, regulatory T cells having an inducible regulatory T cell specific demethylation state can be expanded by culturing in an unstimulated state in the presence of IL-2. The medium may further contain ascorbic acid, and by culturing in a medium further containing IL-2, a stable regulatory T cell culture of the inducible regulatory T cells can be obtained.

In order to isolate the regulatory T cells from the obtained cell culture containing the regulatory T cells, the cells may be isolated by a conventional method based on cell surface markers specific to the regulatory T cells, and for example, a FoxP3-positive fraction may be collected using a cell sorter. In addition, regulatory T cells having specific antigenic properties may also be isolated as desired.

The inducible regulatory T cells obtained by the method of the present disclosure are expected to be used for the treatment of human inflammatory diseases, for example, autoimmune diseases and allergies.

### (Measurement of positivity rate by flow cytometry)

In an embodiment, a positivity rate measurement by flow cytometry can be performed as follows.

### Preparation

· Fixation/Permeabilization Concentrate(Hereinafter, Buffer) (eBio Science, 00-5123-43)
· Fixation/Permeabilization Diluent(Hereinafter, Diluent) (eBio Science, 00-5223-56)
Permeabilization Buffer (10×) (eBio Science, 00-833-56)
· FOXP3 Monoclonal Antibody (236A/E7), PE (hereinafter, anti-FOXP3 antibody) (eBio Science, 12-4777-42)
· Mouse IgG1 kappa Isotype Control (P3.6.2.8.1), PE (hereinafter, PE control) (eBio Science)
· BV421, Mouse, Anti-Human, CD152(Hereinafter, anti-CTLA4 antibody) (BD)
· BV421 Mouse IgG2a, k Isotype Control (Hereinafter, BV421 control) (BD)
· CD4 Monoclonal Antibody (RPA-T4), APC (hereinafter, anti-CD4 antibody) (eBio Science)
· Mouse IgG1 kappa Isotype Control (P3.6.2.8.1), APC (hereinafter, APC control) (eBio Science)
· D-PBS(NACALAI TESQUE, INC., 14249-95)
· FBS (HyClone, SH30084.03)
· 0.5 mol/l EDTA solution(pH 8.0) (NACALAI TESQUE, INC., 06894-14)
· MilliQ water
· Centrifuge
· Safety cabinet
· Micropipette(P200, P1000)
5 mL polystyrene round tube (hereinafter, dedicated tube) (Falcon, 352008)
· Nylon mesh(65 µm) (Kyoshin Riko, PP-65N)

### Reagent preparation

* Fixation Buffer (use 100 µL per sample)
   Buffer and Diluent are mixed at a ratio of 1 : 3.
* Perm Buffer
   Permeabilization Buffer (10×) is diluted 10-fold with MilliQ water.
* FACS Bufer (in the case of preparing 500 mL)
   D-PBS 489 mL
   FBS 10 mL (final concentration 2%)
   0.5 mol/l EDTA solution 1 mL (final concentration 1 mM)

The above reagents are stored at 4°C or on ice after preparation.

### Method

(1) Add 500 µL of FACS Bufer to a 1.5 mL tube.
(2) Add 1 × 10⁶ cells of the final product to the tube of (1), and gently suspend using a micropipette.
(3) Centrifuge at 500 × g and 4°C for 5 minutes.
(4) After removing the supernatant with an aspirator, add 100 µL of Fixation Buffer and gently pipette. → Take care to avoid foaming.
(5) Fix by standing for 30 minutes or longer with light shielding on ice. → 45 minutes may also be acceptable.
(6) After fixation, add 1 mL of Perm Buffer to the tube and gently pipette.
(7) Prepare new 1.5 mL tubes according to the number of samples.
(8) Dispense 500 µL of each sample for control and for antibody staining.
(9) Centrifuge at 500 × g and 4°C for 5 minutes.
(10) During centrifugation, prepare an antibody preparation solution by diluting an anti-FOXP3 antibody (stock concentration = 0.05 mg/ml), an anti-CTLA4 antibody (Lot: 0030269, stock concentration = 0.2 mg/ml), and an anti-CD4 antibody (stock concentration = 0.1 mg/ml) 100-fold with Perm Buffer (hereinafter, referred to as the antibody preparation solution). For the control samples, prepare PE control (stock concentration = 0.1 mg/ml), BV421 control (stock concentration = 0.2 mg/ml), and APC control (stock concentration = 0.1 mg/ml) at the same concentration as the antibodies with the corresponding dyes (hereinafter, referred to as the control solution). * In the in-process control test, staining for CTLA4 is not performed.
(11) After removing the supernatant with an aspirator, add 100 µL of the antibody preparation solution to the antibody-staining sample, and 100 µL of the control solution to the control sample, and gently pipette. → Take care to avoid foaming.
(12) Fix by standing for 60 minutes with light shielding on ice.
(13) Start up the measurement equipment during staining.
(14) After staining, add 1 mL of FACS Bufer and gently pipette.
(15) Centrifuge at 500 × g and 4°C for 5 minutes. → After removing the supernatant with an aspirator, the steps (14) and (15) are repeated once more for washing.
(16) After removing the supernatant with an aspirator, add 500 µL of FACS Bufer and gently pipette.
(17) Place a nylon mesh on a dedicated tube using tweezers, and filter the suspension.
(18) Analyze using any flow cytometer. The number of cells collected for data acquisition should be 10,000 or more. In calculation of the positivity rate for the target antigen, the baseline is set so that the positivity rate of the control sample is 5% or less, and the proportion of cells showing an antigen expression level above the baseline is defined as the positivity rate.

### Remarks

· The reagents used for fixation and staining can also be purchased as a set of three under the name "Foxp3/Transcription Factor Staining Buffer Set" (Cat.: 00-5523).
· As long as the settings of the equipment and the like are not so deviated that it can be clearly determined from a general or scientific viewpoint that normal measurement cannot be performed, any settings may be used. A clear deviation is a case where various signals of the target cell population fall below the set fluorescence threshold value, a case where various signal values of the control sample or the measurement target sample fall below or exceed a limit value that can be normally measured by the equipment, or the like.

### (Measurement of immunosuppressive activity)

The cells of the present disclosure can have immunosuppressive activity. The immunosuppressive activity can be measured by various methods.

In an embodiment, as described in Examples, the suppression can be determined by measuring cell proliferation caused by the immune response of responder T cells. In addition, various kits can be used, and a Treg immunosuppressive assay (horizon discovery assay; https://www.horizondiscoverykk.com/products/research-services/immune-cell-based-assay/immune-suppression-assays/#Treg) can be used, but is not limited thereto. The immunosuppressive activity of the cells of the present disclosure may be enhanced compared to conventional iTregs. Preferably, in the present disclosure, it is desirable to demonstrate this using an in vivo model. For example, as described in Examples, in the case of a colitis model mouse, the activity can be measured by administering the inducible regulatory T cells of the present disclosure to a colitis model mouse, collecting tissue after a certain period following administration, and determining whether immunosuppression is observed in the tissue. Confirmation of immunosuppression in the tissue can be achieved by various methods, and for example, the presence or absence of immunosuppression can be confirmed by histological staining analysis of the tissue.

### (General technique)

The molecular biological technique, the biochemical technique, and the microbiological technique used herein are well known and commonly used in the art, and are described in, for example, Sambrook J. et al. (1989). Molecular Cloning: A Laboratory Manual, Cold Spring Harbor and its 3rd Ed. (2001); Ausubel, F.M. (1987). Current Protocols in Molecular Biology, Greene Pub. Associates and Wiley-Interscience; Ausubel, F.M. (1989). Short Protocols in Molecular Biology: A Compendium of Methods from Current Protocols in Molecular Biology, Greene Pub. Associates and Wiley-Interscience; Innis, M.A. (1990). PCR Protocols: A Guide to Methods and Applications, Academic Press; Ausubel, F.M. (1992). Short Protocols in Molecular Biology: A Compendium of Methods from Current Protocols in Molecular Biology, Greene Pub. Associates; Ausubel, F.M. (1995). Short Protocols in Molecular Biology: A Compendium of Methods from Current Protocols in Molecular Biology, Greene Pub. Associates; Innis, M.A. et al. (1995). PCR Strategies, Academic Press; Ausubel, F.M. (1999). Short Protocols in Molecular Biology: A Compendium of Methods from Current Protocols in Molecular Biology, Wiley, and annual updates; Sninsky, J.J. et al. (1999). PCR Applications: Protocols for Functional Genomics, Academic Press, the special volume of Experimental Medicine, "Experimental Methods for Gene Introduction & Expression Analysis", YODOSHA CO., LTD., 1997, and the like, and relevant parts of which are incorporated herein by reference.

For DNA synthesis techniques and nucleic acid chemistry for producing artificially synthesized genes, gene synthesis or fragment synthesis services such as GeneArt, GenScript, and Integrated DNA Technologies (IDT) can also be used, in addition, the DNA synthesis techniques and nucleic acid chemistry for producing artificially synthesized genes are described in, for example, Gait, M.J. (1985). Oligonucleotide Synthesis: A Practical Approach, IRL Press; Gait, M.J. (1990). Oligonucleotide Synthesis: A Practical Approach, IRL Press; Eckstein, F. (1991). Oligonucleotides and Analogues: A Practical Approach, IRL Press; Adams, R.L. et al. (1992). The Biochemistry of the Nucleic Acids, Chapman & Hall; Shabarova, Z. et al. (1994). Advanced Organic Chemistry of Nucleic Acids, Weinheim; Blackburn, G.M. et al. (1996). Nucleic Acids in Chemistry and Biology, Oxford University Press; Hermanson, G.T. (I996). Bioconjugate Techniques, Academic Press, and the like, and relevant parts (which may be all) of which are incorporated herein by reference.

As used herein, "or" is used when "at least one or more" of the items listed in the text can be employed. The same applies to "or". When explicitly described herein as "within the range" of "two values", the range also includes the two values themselves. Reference literatures such as scientific literatures, patents, and patent applications cited herein are incorporated herein by reference to the same extent that the entirety of each document is specifically described.

The present disclosure has been described above with reference to preferred embodiments for easy understanding. Hereinafter, the present disclosure will be described based on examples, but the above description and the following examples are provided for the purpose of illustration only and not for the purpose of limiting the present disclosure. Accordingly, the scope of the present disclosure is not limited to the embodiments or examples specifically described As used herein, but is limited only by the claims.

### Examples

In the following Examples, glutamine-free RPMI1640 (containing 10% FCS (v/v), 60 µg/mL penicillin G, 100 µg/mL streptomycin, and 10 mM HEPES) was used as a basal medium. If necessary, L-glutamine was added to the medium at 30 to 300 mg/L and used.

### Bisulfite sequence

Genomic DNA was obtained from the induced cells, and after treatment using MethylEasy Xceed Rapid DNA Bisulphite Modification Kit (Human Genetic Signatures), the demethylation of genes characteristic of regulatory T cells was examined. The analysis of demethylation of each gene was performed using known methods and primers (Floess et al. (2007) PloS Biology Volume 5, Issue 2, e38, and Ohkura et al. (2012) Immunity 37(5) 785-799).

In addition, in the analysis of the human Foxp3 CNS2 region, 5'-TTGGGTTAAGTTTGTTGTAGGATAG-3' (SEQ ID NO: 1) was used on the forward side, and 5'-ATCTAAACCCTATTATCACAACCCC-3' (SEQ ID NO: 2) was used on the reverse side.

Acquisition of each T cell fraction Human CD4⁺ T cells were isolated from PBMCs of healthy individuals. The concentrated PBMCs were purified using CliniMACS CD4 GMP MicroBeads according to the product protocol.

Acquisition of mouse T cell fraction Foxp3-eGFP (eFox) reporter mice (Ito et al. (2014) Science 346, 363-368) were used. Lymph node cells of Foxp3-eGFP (eFox) reporter mice were obtained and an endogenous regulatory T cell (nTreg) fraction was obtained by sorting CD4⁺GFP⁺ cells by FACSAriaII (BD). CD4⁺GFP⁻CD44^{low}CD62L^{high} cells were sorted to obtain a naive T cell fraction. Furthermore, a CD4⁺GFP⁻CD44^{high}CD62L^{low} cell fraction was obtained and used as effector/memory T cells.

### (Example 1: Production of regulatory T cells from CD4-positive human peripheral T cells)

The CD4-positive T cells were stimulated for 3 days in a basal medium containing an anti-CD3 antibody (the antibody at a concentration of 10 µg/ml was added in an amount of 100 µL per well and allowed to stand at room temperature for 60 minutes to immobilize the antibody on the container), hIL-2 (100 U/ml), hTGFβ1 (10 ng/ml), retinoic acid (1 µM), and Senexin A (5 µM). Thereafter, a resting culture was performed for 2 days in a basal medium containing hIL-2 (100 U/ml). After 2 days, the medium was similarly replaced, and a resting culture was further performed for another 2 days. After 2 days, the cells were again stimulated for 2 days in a basal medium containing an anti-CD3 antibody (the antibody at a concentration of 10 µg/ml was added in an amount of 100 µL per well and allowed to stand at room temperature for 60 minutes to immobilize the antibody on the container), hIL-2 (100 U/ml), h TGFβ1 (10 ng/ml), retinoic acid (1 µM), Senexin A (5 µM), and ascorbic acid (10 µg/ml). Thereafter, a resting culture was performed for 2 days in a basal medium containing hIL-2 (100 U/ml). After 2 days, the medium was similarly replaced, and a resting culture was further performed for another 2 days. The expression of FoxP3, CTLA4, and Helios of the regulatory T cells obtained after 2 days was analyzed by flow cytometry (FIG. 1).

Conventional inducible regulatory T cells (conventional iTregs) have insufficient expression of functional molecules (for example, in this case, CTLA4) and low FoxP3 induction efficiency. On the other hand, CTLA4 and FoxP3 are expressed in endogenous regulatory T cells (nTregs), and Helios, which is a representative nTreg marker molecule, is expressed. The highly functional inducible regulatory T cells obtained by the production method of the present application are Helios-negative, and can be confirmed to be a population containing many cells expressing FoxP3 and CTLA4. Helios is a marker of regulatory T cells that are endogenously present in the body, and Helios negativity indicates that the cell is an inducible regulatory T cell. Since the inducible regulatory T cells obtained by the conventional method are negative for CTLA4 as an example, it can be seen that the obtained inducible regulatory T cells are inducible regulatory T cells newly induced in vitro at least in terms of positive for CTLA4.

In addition, the demethylation state of cells cultured in the same experimental system was confirmed by bisulfite sequencing (FIG. 2). The inducible regulatory T cells obtained by the production method of the present disclosure show a demethylation state similar to that of nTregs.

It is shown that the inducible regulatory T cells obtained from the above Examples are, unlike endogenous nTregs, regulatory T cells newly induced in vitro, but have characteristics that are not present in existing inducible regulatory T cells having high induction efficiency, high CTLA4 expression, and demethylation of the FOXP3 CNS2 region.

### (Example 2: In vitro suppressive activity of regulatory T cells)

Regulatory T cells were obtained by the same experimental system as in Example 1. The responder T cells (5 x 10⁴) labeled with Cell Trace Violet and the produced regulatory T cells (5 x 10⁴) were co-cultured for 3 days in the presence of Treg Suppression Inspector (Miltenyi Biotec) (5 µL/well), and the Cell Trace Violet intensity was analyzed by flow cytometry. When the responder T cells cause an immune response, the Cell Trace Violet intensity shows multiple weak peaks due to cell proliferation; however, when the immune response is suppressed, the Cell Trace Violet intensity remains as a single strong peak. It was confirmed that the regulatory T cells obtained by the production method of the present disclosure strongly suppress the immune response of the responder T cells. Therefore, it was confirmed that the obtained regulatory T cells have a higher suppressive function than the existing regulatory T cell population (FIG. 3).

### (Example 3: Comprehensive gene expression analysis of regulatory T cells)

The gene expression pattern of the regulatory T cells produced by the method of Example 1 was confirmed by RNA sequencing (FIG. 4). RNA sequencing was performed using known methods (Kitagawa et al. (2017) Nat. Immunol. 18, 173-183). The genes of FOXP3, IL-2RA (CD25), and major suppressive molecules of Tregs (such as CTLA4) were confirmed to be highly expressed in the inducible regulatory T cells obtained by the production method of the present disclosure and in nTreg cells, compared to existing inducible regulatory T cells and activated Tconv cells. In addition, it was confirmed that expression of IKZF2 as a marker of nTreg cells was low in inducible regulatory T cells. It can be confirmed that the inducible regulatory T cells obtained by the production method of the present disclosure have higher expression of some suppressive molecules such as ENTPD1, NT5E, and AREG, compared to other regulatory T cell populations. In addition, it can be confirmed that expression of adhesion molecules such as ITGAE and chemokine receptors such as CCR4 is high (FIG. 4).

As an example, when the expression of the CD103 molecule encoded by ITGAE is analyzed by flow cytometry, it can be confirmed that the inducible regulatory T cells obtained by the production method of the present application have higher CD103 expression compared to other regulatory T cell populations (FIG. 5). As a comprehensive consideration, it can be expected that the inducible regulatory T cells obtained by the production method of the present application exhibit a higher suppressive ability compared to existing regulatory T cells.

### (Example 4: Production of regulatory T cells from CD8-positive human peripheral T cells)

The CD8-positive T cells were stimulated for 3 days in a basal medium containing an anti-CD3 antibody (the antibody at a concentration of 10 µg/ml was added in an amount of 100 µL per well and allowed to stand at room temperature for 60 minutes to immobilize the antibody on the container), hIL-2 (100 U/ml), hTGFβ1 (10 ng/ml), retinoic acid (1 µM), and Senexin A (5 µM). Thereafter, a resting culture was performed for 2 days in a basal medium containing hIL-2 (100 U/ml). After 2 days, the medium was similarly replaced, and a resting culture was further performed for another 2 days. After 2 days, the cells were again stimulated for 2 days in a basal medium containing an anti-CD3 antibody (the antibody at a concentration of 10 µg/ml was added in an amount of 100 µL per well and allowed to stand at room temperature for 60 minutes to immobilize the antibody on the container), hIL-2 (100 U/ml), hTGFβ1 (10 ng/ml), retinoic acid (1 µM), Senexin A (5 µM), and ascorbic acid (10 µg/ml). Thereafter, a resting culture was performed for 2 days in a basal medium containing hIL-2 (100 U/ml). After 2 days, the medium was similarly replaced, and a resting culture was further performed for another 2 days. The expression of FoxP3, CTLA4, and Helios of the regulatory T cells obtained after 2 days was analyzed by flow cytometry (FIG. 6).

It was also confirmed that the inducible regulatory T cells of the present disclosure produced from CD8-positive T cells were Helios-negative and constituted a population containing many cells expressing FoxP3 and CTLA4.

### (Example 5: Production of inducible regulatory T cells from human peripheral T cells)

Inducible regulatory T cells or a cell population thereof are obtained in the same manner as in Example 1 using human peripheral T cells. A medicament containing the inducible regulatory T cells or the cell population thereof and a carrier is produced. The medicament contains about 2 x 10⁵ cells as inducible regulatory T cells or a cell population thereof, and the effectiveness can be confirmed from the degree of inflammation in tissues collected 1 month after administration to a patient with an inflammatory disease.

### (Example 6: Formulation example)

Inducible regulatory T cells or a cell population thereof are obtained in the same manner as in Example 1. A medicament containing the inducible regulatory T cells or the cell population thereof and a carrier is produced.

### (Example 7: Expression intensity analysis)

The expression intensity of each of CD25 and FOXP3 in the cell population of the inducible regulatory T cells produced in Example 1 was analyzed by flow cytometry using a BD FACSLyric flow cytometer. The results are shown in FIG. 7. In all the tested cells, it was confirmed that a cell population of FOXP3^{Hi} inducible regulatory T cells was present at a high proportion.

### (Example 8: Antibody panel analysis of inducible regulatory T cells)

In order to comprehensively analyze cell surface markers in the inducible regulatory T cells produced in Example 1, antibody panel analysis was performed. The inducible regulatory T cells produced in Example 1 were stained using the LEGENDScreen Human PE Kit from BioLegend, and antibodies reacting with the inducible regulatory T cells of the present disclosure were analyzed by flow cytometry. A portion of the results (those for which clear and meaningful expression was confirmed) is shown in Tables 1 to 4. When stained with an antibody panel containing slightly fewer than 400 types, it was possible to confirm the positive/negative characteristics of surface markers in the inducible regulatory T cells of the present disclosure using at least 185 surface marker molecules.

**[Table 1]**

| Antigen | Phenotype | Antigen | Phenotype |
|---|---|---|---|
| ICOS | + | CD137 | - |
| IFN *γ* R*β* | - | CD138 | - |
| CD1a | - | CD141 | - |
| CD2 | + | CD142 | - |
| beta2microgroblin | + | CD143 | - |
| Cadherin11 | - | CD15 | - |
| CD10 | - | CD156c | + |
| CD100 | + | CD158e1 | - |
| CD103 | + | CD162 | + |
| CD106 | - | CD163 | - |
| CD107a | + | CD164 | + |
| CD107b | + | CD169 | - |
| CD116 | - | CD172g | + |
| CD117 | - | CD18 | + |
| CD11a | + | CD183 | + |
| CD122 | - | CD194 | + |
| CD123 | + | CD202b | - |
| CD126 | - | CD206 | - |
| CD127 | - | CD207 | - |
| CD135 | - | CD21 | - |
| | | CD213 *α* 2 | + |
| | | CD229 | - |
| | | CD23 | - |
| | | CD231 | - |

**[Table 2]**

| Antigen | Phenotype | Antigen | Phenotype |
|---|---|---|---|
| CD25 | + | CD35 | - |
| CD27 | + | CD354 | - |
| CD271 | - | CD365 | - |
| CD275 | - | CD367 | - |
| CD147 | + | CD38 | + |
| CD28 | + | CD39 | + |
| CD29 | + | CD4 | + |
| CD290 | - | CD40 | - |
| CD298 | + | CD41 | - |
| CD3 | + | CD42b | - |
| CD300c | + | CD43 | + |
| CD309 | - | CD44 | + |
| CD317 | + | CD45 | + |
| CD324 | - | CD47 | + |
| CD328 | - | CD49d | + |
| CD334 | - | CD5 | + |
| CD335 | - | CD50 | + |
| CD336 | - | CD54 | + |
| CD34 | - | CD55 | + |
| CD340 | - | CD58 | + |
| | | CD6 | + |
| | | CD61 | - |
| | | CD62E | - |
| | | CD62P | - |
| | | CD63 | + |
| | | CD64 | - |
| | | CD81 | + |
| | | CD85g | - |
| | | CD85k | - |
| | | CD89 | - |
| | | CD95 | + |
| | | CD97 | + |
| | | CD99 | + |
| | | CXCL16 | - |

**[Table 3]**

| Antigen | Phenotype | Antigen | Phenotype | Antigen | Phenotype |
|---|---|---|---|---|---|
| DLL1 | | SUSD2 | - | CD303 | - |
| DLL4 | | Tim-4 | - | CD307 | - |
| EGFR | | TM4SF3 | - | CD323 | - |
| GPR19 | | TSLPR | - | | |
| HVEM | + | VEGFR3 | - | CD36 | - |
| IgM | | CD133 | - | CD369 | - |
| Ksp37 | | APCDD1 | - | CD370 | - |
| LY6G6D | | BTLA | + | CD371 | - |
| MERTK | | CCR8 | + | CD45RO | + |
| MUC-13 | | CD102 | + | CD51 | - |
| NKp80 | | CD104 | - | CD59 | + |
| Notch1 | + | CD130 | - | | |
| Notch3 | | CD144 | - | CD7 | + |
| Notch4 | | CD158b | - | CD71 | + |
| PSMA | | CXCR4 | + | CD88 | - |
| Siglec10 | | CD192 | + | CRTAM | - |
| Siglec7 | | CD209 | - | FPR3 | - |
| Siglec8 | | CD230 | + | | |
| Siglec9 | | CD24 | - | | |
| SSEA-5 | | CD245 | - | | |
| | | CD26 | + | | |
| | | CD269 | - | | |
| | | CD282 | - | | |

**[Table 4]**

| Antigen | Phenotype | Antigen | Phenotype |
|---|---|---|---|
| IL28RA | - | CD120b | + |
| LOX-1 | - | CD210 | + |
| MICA | - | CD267 | - |
| Notch2 | + | | |
| TACSTD2 | - | CD294 | - |
| CD129 | + | CD49f | - |
| CXCR1 | + | CD85d | + |
| CD1d | - | Integrin beta7 | + |
| CD20 | + | Podoplanin | - |
| CD220 | - | CD132 | + |
| CD32 | - | | |
| CD368 | - | | |
| CD52 | + | | |
| CD66 | + | | |
| CD85h | - | | |
| CD85j | - | | |
| EphA2 | - | | |
| FceRIa | - | | |
| CD255 | - | | |
| CD201 | - | | |

### (Example 9: Analysis of cell surface markers in inducible regulatory T cells)

Characteristic results from the antibody screening performed in Example 8 are shown in FIG. 8. In the inducible regulatory T cells of the present disclosure, specific cell surface markers such as CD172g and CD26 were found to be positive.

### (Example 10: Method for producing mouse highly functional and stable regulatory T cells)

The mouse CD4-positive T cells were stimulated for 3 days in a basal medium containing an anti-CD3 antibody (the antibody at a concentration of 10 µg/ml was added in an amount of 100 µL per well and allowed to stand at room temperature for 60 minutes to immobilize the antibody on the container), hIL-2 (100 U/ml), hTGFβ1 (2.5 ng/ml), retinoic acid (1 µM), and Senexin A (5 µM). Thereafter, a resting culture was performed for 2 days in a basal medium containing hIL-2 (100 U/ml). After 2 days, the medium was similarly replaced, and a resting culture was further performed for another 2 days. After 2 days, the cells were again stimulated for 2 days in a basal medium containing an anti-CD3 antibody (the antibody at a concentration of 10 ug/ml was added in an amount of 100 µL per well and allowed to stand at room temperature for 60 minutes to immobilize the antibody on the container), hIL-2 (100 U/ml), hTGFβ1 (2.5 ng/ml), retinoic acid (1 µM), Senexin A (5 µM), and ascorbic acid (10 µg/ml). Thereafter, a resting culture was performed for 2 days in a basal medium containing hIL-2 (100 U/ml). After 2 days, the medium was similarly replaced, and a resting culture was further performed for another 2 days. The expression of FoxP3 and CD25 of the regulatory T cells obtained after 2 days was analyzed by flow cytometry (FIG. 9), the demethylation state of the Treg-specific demethylated region was analyzed by the bisulfite method (FIG. 10), and the comprehensive gene expression pattern was analyzed by an RNA-seq method (FIG. 11), respectively.

Although conventional inducible regulatory T cells (conventional iTregs) have insufficient expression of functional molecules (in this case, for example, CTLA4, Entpd1, Nt5e, Itgae, and the like are shown in FIG. 3) and low FoxP3 induction efficiency, it was confirmed that the highly functional inducible regulatory T cells obtained by the production method of the present application exhibit high expression of FoxP3 and functional genes (in this case, for example, CTLA4, Entpd1, Nt5e, and Itgae as shown in FIG. 11), and have a demethylation state characteristic of Tregs (FIGS. 9 to 11).

### (Example 11: In vitro suppressive activity of regulatory T cells)

Regulatory T cells were obtained by the same experimental system as in Example 1. The responder T cells (5 × 10⁴) labeled with Cell Trace Violet and the produced regulatory T cells (5 × 10⁴) were co-cultured for 3 days in the presence of either anti-CD3 antibody (1µg/mL) + antigenpresenting cells (MHC class II-positive cells: 1 × 10⁴), or Dynabeads T-activator (Veritas) (5 µL/well), and the Cell Trace Violet intensity was analyzed by flow cytometry. When the responder T cells cause an immune response, the Cell Trace Violet intensity shows multiple weak peaks due to cell proliferation; however, when the immune response is suppressed, the Cell Trace Violet intensity remains as a single strong peak. It was confirmed that the regulatory T cells obtained by the production method of the present application strongly suppress the immune response of the responder T cells. Therefore, it was confirmed that the obtained regulatory T cells have a higher suppressive function than the existing regulatory T cell population (FIG. 12).

### (Example 12: In vivo stability of regulatory T cells)

Regulatory T cells were produced from Thy1.2/eFox reporter mice by the method of Example 1, Foxp3 positive cells (2 × 10⁵) were separated by FACS, and Thy1.1/wild-type mice were tail-vein administered. The Thy1.2 cells transferred after 2 weeks were collected from the lymph nodes, and Foxp3 expression was analyzed (FIG. 13). It was confirmed that the regulatory T cells induced without CD28 stimulation and subjected to a stabilization culture stably expressed Foxp3 even after 2 weeks in vivo.

### (Example 13: Suppressive effect on colitis model)

2 x 10⁵ wild-type mouse-derived CD45RB-highly expressing CD4 positive naive T cells were administered to a RAG-deficient mouse to prepare a colitis model. After 1 week, 2 x 10⁵ highly functional and stable iTreg cells produced by the method of Example 1 were administered, and the change in body weight was measured over time (FIG. 14A). Thereafter, 1 month after the administration, the colon tissue and lymph nodes were collected, and HE staining analysis of the colon tissue (FIG. 14B) and CD69 expression analysis in lymph node T cells (FIG. 14C: flow cytometry) were performed. As a result, it was confirmed that the administration of the highly functional and stable iTregs suppressed colitis. In addition, the therapeutic effect was confirmed at a dose of 2 × 10⁵ cells per mouse (about 20 g), and the efficacy at 1 × 10⁷ cells/kg was confirmed.

### (Example 14: Production of regulatory T cells from human peripheral T cells)

The CD4-positive T cells derived from a patient with Crohn's disease were stimulated for 3 days in a basal medium containing an anti-CD3 antibody (the antibody at a concentration of 10 µg/ml was added in an amount of 100 µL per well and allowed to stand at room temperature for 60 minutes to immobilize the antibody on the container), hIL-2 (100 U/ml), hTGFβ1 (10 ng/ml), retinoic acid (1 µM), and Senexin A (5 µM). Thereafter, a resting culture was performed for 2 days in a basal medium containing hIL-2 (100 U/ml). After 2 days, the medium was similarly replaced, and a resting culture was further performed for another 2 days. After 2 days, the cells were again stimulated for 2 days in a basal medium containing an anti-CD3 antibody (the antibody at a concentration of 10 ug/ml was added in an amount of 100 µL per well and allowed to stand at room temperature for 60 minutes to immobilize the antibody on the container), hIL-2 (100 U/ml), hTGFβ1 (10 ng/ml), retinoic acid (1 µM), Senexin A (5 µM), and ascorbic acid (10 µg/ml). Thereafter, a resting culture was performed for 2 days in a basal medium containing hIL-2 (100 U/ml). After 2 days, the medium was similarly replaced, and a resting culture was further performed for another 2 days. The expression of FoxP3 and CTLA4 of the regulatory T cells obtained after 2 days was analyzed by flow cytometry (FIG. 15A).

Although conventional inducible regulatory T cells (conventional iTregs) exhibit insufficient expression of functional molecules (in this case, CTLA4) and low FoxP3 induction efficiency, it can be confirmed that the highly functional inducible regulatory T cells obtained by the production method of the present application constitute a population containing many cells expressing FoxP3 and CTLA4.

### (Example 15: In vitro suppressive activity of regulatory T cells)

Regulatory T cells were obtained by the same experimental system as in Example 10. The responder T cells (5 × 10⁴) labeled with Cell Trace Violet and the produced regulatory T cells (5 × 10⁴) were co-cultured for 3 days in the presence of Treg Suppression Inspector (Miltenyi Biotec) (5 µL/well), and the Cell Trace Violet intensity was analyzed by flow cytometry. When the responder T cells cause an immune response, the Cell Trace Violet intensity shows multiple weak peaks due to cell proliferation; however, when the immune response is suppressed, the Cell Trace Violet intensity remains as a single strong peak. It was confirmed that the regulatory T cells obtained by the production method of the present application strongly suppress the immune response of the responder T cells. Therefore, it was confirmed that the obtained regulatory T cells have a higher suppressive function than the existing regulatory T cell population.

### (Example 16: Production of regulatory T cells from T cells derived from systemic lupus erythematosus (SLE) patient)

Using CD4-positive T cells derived from an SLE patient, regulatory T cells were obtained by the same experimental system as in Example 1. The expression of FoxP3, CD4, and CTLA4 of the obtained regulatory T cells was analyzed by flow cytometry (FIG. 16).

Although conventional inducible regulatory T cells (conventional iTregs) exhibit insufficient expression of functional molecules (in this case, CTLA4) and low FoxP3 induction efficiency, it can be confirmed that the highly functional inducible regulatory T cells obtained by the production method of the present disclosure constitute a population containing many cells expressing FoxP3 and CTLA4.

### (Example 17: Production of regulatory T cells from T cells derived from rheumatoid arthritis (RA) patient)

Using CD4-positive T cells derived from an RA patient, regulatory T cells were obtained by the same experimental system as in Example 1. The expression of FoxP3, CD4, and CTLA4 of the obtained regulatory T cells was analyzed by flow cytometry (FIG. 17).

Although conventional inducible regulatory T cells (conventional iTregs) exhibit insufficient expression of functional molecules (in this case, CTLA4) and low FoxP3 induction efficiency, it can be confirmed that the highly functional inducible regulatory T cells obtained by the production method of the present application constitute a population containing many cells expressing FoxP3 and CTLA4.

### (Example 18: FOXP3 stability evaluation)

In the present example, it was confirmed that the inducible regulatory T cells in the cell population of the present disclosure were stably FOXP3-positive cells. As shown in FIG. 18, FOXP3 was stably expressed consistently from day 7 of culture (day7) in the in-process control test through day 13 (final product), day 15 (day 2 of the stability test), and day 17 (day 4 of the stability test).

In addition, with respect to the medium conditions during the production of the inducible regulatory human T cells in the cell population of the present disclosure, the stability of FOXP3 expression was analyzed on day 9 (after the second stimulation), day 13 (final product), and day 15 (re-stimulation of the final product; example of a stability test) when RPMI was used, when glucose was reduced to half in RPMI, and when glutamine was reduced to 10 to 50% in RPMI (FIG. 19).

As shown in FIG. 19, it can be seen that in these medium compositions, the inducible regulatory T cells of the present disclosure can be produced at a certain proportion, and that the inducible regulatory T cells exhibited consistently stable regulatory T cell characteristics from the point of phenotypic confirmation (day 9).

Furthermore, the stability of FOXP3 expression in the inducible regulatory T cells of the present disclosure was analyzed after re-stimulation of the final product of the inducible regulatory T cells of the present disclosure when re-stimulated under a conventional re-stimulation condition (CD3/CD28 stimulation in RPMI + IL2), or when re-stimulated with the addition of Th1 cytokines (IL-12 and IFN-y), Th2 cytokines (IL-4 and IL-5), or Th17 cytokines (TGF-β, IL-6, IL-1β, and IL-23) (FIG. 20).

As shown in FIG. 20, it was confirmed that FOXP3 expression remained stable not only when re-stimulated under a conventional re-stimulation condition (CD3/CD28 stimulation with RPMI + IL2), but also when re-stimulated with the addition of Th1 cytokines (IL-12 and IFN-y), Th2 cytokines (IL-4 and IL-5), or Th17 cytokines (TGF-β, IL-6, IL-1β, and IL-23), and that stable regulatory T cell characteristics were exhibited.

### (Example 19: Evaluation of FOXP3 stability after freeze-thawing)

It was confirmed that the inducible regulatory T cells in the cell population of the present disclosure were stably FOXP3-positive cells even after freeze-thawing. The inducible regulatory T cells in the cell population of the present disclosure were frozen and then thawed 7.5 months later (freezing date: October 16, 2021; thawing date: June 6, 2022), and allowed to stand for a certain period of time before performing FACS analysis. As a freezing solution, the Kymriah composition (1 × 10⁸ cells/freezing bag) was used. As a thawing solution, 40 mL of dextran injection was used, the solution was allowed to stand at 4°C or at room temperature, and cells were sampled and analyzed at 0 hours, 1 hour, 2 hours, and 3 hours after standing. The results are shown in Table 5.

**[Table 5]**

| **RD110** | Viability | %FOXP3+CD4+ | %CTLA4+,FOXP3+ | FOXP3 Mean | CTLA4 Mean |
|---|---|---|---|---|---|
| 0 h | 93.86% | 93.00% | 80.50% | 3787 | 1125 |
| RT, 1 h | 94.74% | 92.20% | 76.30% | 3375 | 938 |
| RT, 2 h | 90.57% | 92.20% | 76.10% | 3276 | 933 |
| RT, 3 h | 87.08% | 88.90% | 71.00% | 2744 | 755 |
| | | | | | |
| 0 h | 93.86% | 93.00% | 80.50% | 3787 | 1125 |
| 4°C, 1 h | 95.29% | 91.90% | 78.00% | 4304 | 1083 |
| 4°C, 2 h | 89.62% | 89.90% | 74.80% | 3856 | 997 |
| 4°C, 3 h | 91.96% | 90.90% | 73.90% | 3444 | 916 |

The inducible regulatory T cells in the cell population of the present disclosure stably express FOXP3 even after being frozen and stored for 6 months or longer, indicating that the cells can be preserved for 6 months or longer. In addition, after thawing, the state of the cells was good when stored at 4°C for up to 2 hours. Even at room temperature, no significant change was observed during storage for up to 2 hours.

### (Example 20: Production of regulatory T cells from femalederived T cells and evaluation of FOXP3 stability)

Using CD4-positive T cells derived from a female, regulatory T cells were obtained by the same experimental system as in Example 1, and with respect to the final product of the inducible regulatory T cells of the present disclosure, the expression of FoxP3 and CTLA4 in the obtained regulatory T cells was analyzed by flow cytometry before re-stimulation, or after re-stimulation under a conventional re-stimulation condition (CD3/CD28 stimulation in RPMI + IL-2) with the addition of Th1 cytokines (IL-12 and IFN-y), Th2 cytokines (IL-4 and IL-5), Th17 cytokines (TGF-β, IL-6, IL-1β, and IL-23), and TNF-α (FIG. 21). In addition, each demethylation state was confirmed by bisulfite sequencing (FIG. 21).

As shown in the left diagram of FIG. 21, it was confirmed that the CTLA4 was further increased when the cells were re-stimulated under the conventional re-stimulation condition (CD3/CD28 stimulation in RPMI + IL-2) with the addition of Th1 cytokines (IL-12 and IFN-γ), Th2 cytokines (IL-4 and IL-5), Th17 cytokines (TGF-β, IL-6, IL-1β, and IL-23), and TNF-α compared to the cells before re-stimulation. In addition, as shown in the right diagram of FIG. 21, the inducible regulatory T cells obtained by the production method of the present disclosure showed a demethylation state similar to that of nTregs.

Thus, it was found that the regulatory T cells of the present disclosure are stable even in harsh cytokine conditions.

In addition, with respect to the final product of the inducible regulatory T cells of the present disclosure, the stability of FOXP3 expression in the obtained inducible regulatory T cells was analyzed before re-stimulation, after re-stimulation under a conventional re-stimulation condition (CD3/CD28 stimulation in RPMI + IL-2), and after re-stimulation with the addition of Th1 cytokines (IL-12 and IFN-γ), Th2 cytokines (IL-4 and IL-5), Th17 cytokines (TGF-β, IL-6, IL-1β, and IL-23), and TNF-α (FIG. 21).

As shown in the right diagram of FIG. 21, it was confirmed that FOXP3 expression remained stable not only in the cells before re-stimulation, but also in the cells re-stimulated under a conventional re-stimulation condition (CD3/CD28 stimulation with RPMI + IL2) with the addition of Th1 cytokines (IL-12 and IFN-γ), Th2 cytokines (IL-4 and IL-5), Th17 cytokines (TGF-β, IL-6, IL-1β, and IL-23), and TNF-α, and that stable regulatory T cell characteristics were exhibited. Thus, it was found that the regulatory T cells of the present disclosure are stable even in harsh cytokine conditions.

### (Example 21: Demonstration of efficacy against autoimmune liver disease)

In the present example, demonstration of efficacy against autoimmune liver diseases such as primary sclerosing cholangitis (PSC) and autoimmune hepatitis (AIH) is conducted.

The present disclosure is a cell preparation that is expected to suppress immune responses in general, particularly those centered on T cells. Accordingly, in the transcription instruction, an experiment targeting autoimmune liver disease (AILD) is conducted for the purpose of confirming the efficacy against a broad range of T cell autoimmune diseases as development targets.

### (Details)

AILD mainly includes primary sclerosing cholangitis (PSC) and autoimmune hepatitis (AIH), and in PSC, comorbidity with inflammatory bowel diseases such as ulcerative colitis is common. Therefore, comprehensive control of T cell autoimmune responses is considered important. Accordingly, it is considered that regulation of T cell autoimmune responses by regulatory T cells is highly likely to be effective in controlling the pathology of the disease, and non-clinical development is being conducted.

At present in the present disclosure, a preliminary test is performed. Specifically, the non-clinical tests are conducted at the stage of practical development toward clinical trials, and the items thereof are as follows.

**[Table 6]**

| **Test item** | **Overview** |
|---|---|
| Residual substance test | Measurement of residual raw materials in final product |
| Proliferation property analysis test | Analysis of characteristics of cells cultured over time |
| Analysis of produced humoral factor | Measurement of amount of produced cytokines by ELISA |
| General toxicity test | Testing of administration of cells to immunodeficient rats |
| Stability test | Testing of storage stability and transport stability |
| In vivo efficacy test | Administration of mouse cells to mouse disease model |
| In vitro efficacy test | Analysis of in vitro suppressive ability using human cells |

In particular, general safety is evaluated by a general toxicity test for overall safety and by a proliferation property analysis test for tumorigenicity risk. With respect to efficacy, an in vitro proliferation inhibition assay using human T cells is conducted, and an in vivo allogeneic transplantation test using mouse cells is performed.

### <Residual substance test>

The estimated residual levels of the reagents used in the production of the preparation of the present disclosure are sufficiently close to zero, and there is no safety concern for humans. However, residual amounts of FBS (a xenogeneic component), mouse-derived antibody raw materials (xenogeneic components), and AS2863619 (a novel compound with no history of clinical use) are measured in a reference test for the purpose of evaluating their residual levels and safety.

According to the results of the preliminary test, residual FBS was observed at 8.36 ± 3.55 ng/mL (n = 4 lots), measured as BSA. This value is not higher than that of existing cell preparations using FBS, and it is not considered to be a particular safety concern by sufficiently considering the risk for bovine allergy. With respect to the other mouse-derived antibodies and AS2863619, residual levels were confirmed to be below the measurement limit.

### <Proliferation property analysis test>

A proliferation property analysis test was conducted on the preparation of the present disclosure, and it was confirmed that the preparation did not exhibit uncontrolled proliferative properties during long-term culture, that is, it did not exhibit tumorigenicity. In the preliminary test, extended culture was performed using two lots of the final product, and the number of days until proliferation ceased was 26 and 22 days, respectively, with stimulation, and 16 and 20 days, respectively, without stimulation. It was shown that even under conditions with stimulation that promotes T cell proliferation, proliferation ceased within 30 days.

It is expected that any of the cells constituting the preparation used in the present example will disappear even after administration into the body, and it can be determined that there is no risk of tumorigenicity.

### <Analysis of humoral factor>

At the final stage of culture of the S/F-iTregs of the present disclosure, the following humoral factors contained in the culture supernatant were measured by ELISA. This is attributable to the low production of inflammatory cytokines. The efficacy and safety of the present product are expected, and since a high level of anti-inflammatory cytokine production was observed, the suppressive activity of the present product against responder cells was suggested.

Inflammatory cytokines: IL-4, IL-6, IL-17, IFNγ, TNFα
Anti-inflammatory cytokines: IL-10

**[Table 7]**

| Measurement item | Lot1 | Lot2 | Lot3 | Lot4 |
|---|---|---|---|---|
| IL-10 | 115pg/ml | 70pg/ml | 75pg/ml | 50pg/ml |
| TNF- *α* | <4pg/ml | <4pg/ml | <4pg/ml | <4pg/ml |
| IL-17A | 55pg/ml | 12pg/ml | <2pg/ml | <2pg/ml |
| IFN- *γ* | 32pg/ml | 16pg/ml | 17pg/ml | <4pg/ml |
| IL-4 | <2pg/ml | <2pg/ml | <2pg/ml | <2pg/ml |
| IL-6 | 3pg/ml | 2pg/ml | 2pg/ml | <2pg/ml |

### <Single-dose general toxicity test (preliminary test)>

A single intravenous administration of S/F-iTregs or raw material cells (peripheral blood mononuclear cells: PBMCs) at a dose of 5 × 10⁷ cells/kg (equivalent to 2.5 × 10⁹ cells/50 kg; set to 10 times or more the trial estimated dose of 1 × 10⁸ cells) was performed via the tail vein in immunodeficient male and female rats (F344/NJcl-rnu/rnu), and the presence or absence of toxicity and behavioral changes was observed for 3 weeks after administration. Note that a human serum albumin injection was used as a control. Five male and five female animals were used in each group for the test.

As a result, no toxicological changes attributable to S/F-iTregs at the present dose were observed in general condition, body weight, food consumption, blood chemistry tests, organ weights, necropsy, or histopathological examination.

### <Stability test>

### (1) Storage temperature -80°C

· Storage period: 0.6 months
· Test items: Cell count, viability, proportions of CD4-positive and FOXP3-positive cells, and proportion of CTLA4-positive cells
· Test results: Even after storage for 6 months or longer, no significant changes were observed in the test results of each test item.

### (2) Storage temperature 4°C

· Storage period: 0, 1, 2, and 3 hours
· Test items: Cell count, viability, proportions of CD4-positive and FOXP3-positive cells, and proportion of CTLA4-positive cells
· Test results: No significant change was observed in the test results of each test item at any point up to 2 hours. At the 3-hour time point, a slight decrease in viability was observed.

### (3) Storage temperature 25°C

· Storage period: 0, 1, 2, and 3 hours
· Test items: Cell count, viability, proportions of CD4-positive and FOXP3-positive cells, and proportion of CTLA4-positive cells
· Test results: No significant change was observed in the test results of each test item at any point up to 2 hours. At the 3-hour time point, a slight decrease in viability was observed.

The S/F-iTregs used for non-clinical studies were stored at -80°C for about 6 months or longer, during which the provisional specifications for cell count, viability, proportions of CD4-positive and FOXP3-positive cells, and a proportion of CTLA4-positive cells were maintained, and no difference was observed in each result compared to that at month 0.

### <Non-clinical pharmacological efficacy test (in vitro test)>

### · T cell proliferation inhibition assay

Using a general cell proliferation assay to evaluate the suppressive capacity of S/F-iTregs derived from healthy individuals, it was confirmed that the product exhibited greater suppression of autologous T cell proliferation compared to the control (FIG. 3B). In the present example, further, the effect in the target disease is confirmed using T cells derived from an AILD patient.

### <Non-clinical pharmacological efficacy test (in vivo test)>

Since TCR-dependent T cell preparations, including the present product, exert efficacy in response to antigen stimulation via allogeneic MHC molecules, it is theoretically impossible to conduct in vivo pharmacological efficacy tests using human cells. Accordingly, an allogeneic pharmacological efficacy test using mouse cells in a mouse disease model was conducted as a reference test.

### · Mouse model of autoimmune hepatitis

It was confirmed that the autoimmune hepatitis disease state induced by experimental Treg depletion (diphtheria toxin DTX administration model in Foxp3-DTR mice) was ameliorated by administration of S/F-iTregs.

### · Mouse model of colitis

Since some of the target autoimmune liver diseases are accompanied by inflammatory bowel disease symptoms, POC is being obtained using a colitis model induced by transfer of CD45RB-positive naive T cells derived from BALB/c mice into RAG2-deficient mice, in consideration of the potential to include comprehensive improvement of autoimmune symptoms as an evaluation item. The suppressive effect of S/F-iTreg cells in the colitis model has been confirmed so far.

### (Note)

Although the present disclosure has been illustrated using preferred embodiments of the present disclosure as described above, it is understood that the scope of the present disclosure should be interpreted only by the claims. It is understood that the patents, patent applications, and other documents cited herein are to be incorporated by reference herein in the same manner as the contents are specifically described herein. The present application claims priority to Japanese Patent Application No. 2023-53538, filed on March 29, 2023 to the Japan Patent Office, the entire contents of which are incorporated herein by reference.

### Industrial Applicability

The inducible regulatory T cells and/or cell population of the present disclosure can be used for the treatment or prevention of various immune diseases, autoimmune diseases, and other inflammatory disorders. In addition, by the method for producing inducible regulatory T cells and/or a cell population of the present disclosure, regulatory T cells having high functionality can be stably induced from peripheral T cells, and application to the medical field can be expected.

### [Sequence Listing Free Text]

SEQ ID NO: 1: Forward primer used in Examples
SEQ ID NO: 2: Reverse primer used in Examples

[Sequence Listing]

## Claims

1. An inducible regulatory human T cell having at least one characteristic selected from the group consisting of FoxP3-positive, CTLA4-positive, NT5E-positive, ITGAE (CD103)-positive, AREG-positive, CD172g-positive, and CD26-positive.

2. An NT5E-positive inducible regulatory human T cell.

3. An ITGAE (CD103)-positive inducible regulatory human T cell.

4. An AREG-positive inducible regulatory human T cell.

5. A CD172g-positive inducible regulatory human T cell.

6. A CD26-positive inducible regulatory human T cell.

7. A CTLA4-positive inducible regulatory human T cell.

8. A stably FoxP3-positive inducible regulatory human T cell.

9. The inducible regulatory human T cell according to any one of claims 1 to 8, wherein the inducible regulatory human T cell is at least CTLA4-positive and FoxP3-positive.

10. The inducible regulatory human T cell according to claim 9, wherein the FoxP3 is stably expressed.

11. The inducible regulatory human T cell according to claim 9, wherein the FoxP3 is continuously expressed for at least about 48 hours or longer.

12. The inducible regulatory human T cell according to claim 9, wherein the FoxP3 is stably expressed at least from day 7 of culture.

13. The inducible regulatory human T cell according to claim 9, wherein the FoxP3 is expressed even after freeze-thawing of the cell.

14. The inducible regulatory human T cell according to any one of claims 1 to 13, wherein the inducible regulatory human T cell is at least CD172g-positive and/or CD26-positive.

15. The inducible regulatory human T cell according to any one of claims 1 to 14, wherein the CNS2 region of the FOXP3 gene is demethylated.

16. The inducible regulatory human T cell according to any one of claims 1 to 15, wherein the inducible regulatory human T cell is CD4-positive or CD8-positive.

17. The inducible regulatory human T cell according to any one of claims 1 to 16, wherein the inducible regulatory human T cell is obtained from or derived from a human peripheral blood T cell or a human tissue-derived T cell.

18. An inducible regulatory human T cell obtained by a method including:
a step (a) of stimulating CD4-positive T cells or CD8-positive T cells in human peripheral blood in a first basal medium for about 1 to about 5 days;
a step (b) of subjecting the cells obtained in the step (a) to a resting culture in a medium containing IL-2 for at least about 1 to about 3 days;
a step (c) of stimulating the cells obtained in the step (b) in a second basal medium for about 1 to about 5 days; and
a step (d) of subjecting the cells obtained in the step (c) to a resting culture in a medium containing IL-2 for at least about 1 to about 3 days.

19. The inducible regulatory human T cell according to claim 18, wherein the inducible regulatory human T cell is stably FoxP3-positive.

20. A cell population comprising the inducible regulatory human T cell according to any one of claims 1 to 18, wherein the cell population has a proportion of about 50% or more of cells having at least one characteristic selected from the group consisting of FoxP3-positive, CTLA4-positive, NT5E-positive, ITGAE (CD103)-positive, AREG-positive, CD172g-positive, and CD26-positive.

21. A cell population comprising inducible regulatory human T cells, wherein the cell population has a proportion of about 50% or more of NT5E-positive cells.

22. A cell population comprising inducible regulatory human T cells, wherein the cell population has a proportion of about 50% or more of ITGAE (CD103)-positive cells.

23. A cell population comprising inducible regulatory human T cells, wherein the cell population has a proportion of about 50% or more of AREG-positive cells.

24. A cell population comprising inducible regulatory human T cells, wherein the cell population has a proportion of about 50% or more of CD172g-positive cells.

25. A cell population comprising inducible regulatory human T cells, wherein the cell population has a proportion of about 50% or more of CD26-positive cells.

26. A cell population comprising inducible regulatory human T cells, wherein the cell population has a proportion of about 50% or more of CTLA4-positive cells.

27. A cell population comprising inducible regulatory human T cells, wherein the cell population has a proportion of about 50% or more of stably FoxP3-positive cells.

28. The cell population according to any one of claims 20 to 27, wherein proportions of at least the CTLA4-positive cells and FoxP3-positive cells are each about 50% or more.

29. The cell population according to claim 28, wherein the FoxP3 is stably expressed.

30. The cell population according to claim 28, wherein the FoxP3 is continuously expressed for at least about 48 hours or longer.

31. The cell population according to claim 28, wherein the FoxP3 is stably expressed at least from day 7 of culture.

32. The cell population according to claim 28, wherein the FoxP3 is expressed even after freeze-thawing of the cell.

33. The cell population according to any one of claims 20 to 32, wherein proportions of at least the CD172g-positive cells and/or CD26-positive cells are each about 50% or more.

34. The cell population according to any one of claims 20 to 33, wherein a proportion of cells having the at least one characteristic in the cell population is about 60% or more.

35. The cell population according to any one of claims 20 to 34, wherein a proportion of cells having the at least one characteristic in the cell population is about 80% or more.

36. The cell population according to any one of claims 20 to 35, wherein a proportion of FoxP3 strong-positive cells is about 50% or more.

37. The cell population according to claim 36, wherein a proportion of stably FoxP3 strong-positive cells is about 50% or more.

38. The cell population according to any one of claims 20 to 37, wherein the cell population contains about 90% or more T cells.

39. A pharmaceutical composition comprising the inducible regulatory human T cell according to any one of claims 1 to 13 or the cell population according to any one of claims 20 to 38.

40. A regenerative medical material or product comprising the inducible regulatory human T cell according to any one of claims 1 to 13 or the cell population according to any one of claims 20 to 38.

41. A method for producing inducible regulatory human T cells, the method comprising:
a step (a) of stimulating CD4-positive T cells or CD8-positive T cells in human peripheral blood in a first basal medium for about 1 to about 5 days;
a step (b) of subjecting the cells obtained in the step (a) to a resting culture in a medium containing IL-2 for at least about 1 to about 3 days;
a step (c) of stimulating the cells obtained in the step (b) in a second basal medium for about 1 to about 5 days; and
a step (d) of subjecting the cells obtained in the step (c) to a resting culture in a medium containing IL-2 for at least about 1 to about 3 days.

42. The method according to claim 41, wherein the first basal medium contains an anti-CD3 antibody, TGF-β, IL-2, retinoic acid, and at least one selected from the group consisting of a CDK8 inhibitor, a CDK19 inhibitor, and a CDK8/19 inhibitor.

43. The method according to claim 41 or 42, wherein the second basal medium contains an anti-CD3 antibody, TGF-β, IL-2, retinoic acid, ascorbic acid, and at least one selected from the group consisting of a CDK8 inhibitor, a CDK19 inhibitor, and a CDK8/19 inhibitor.

44. The method according to any one of claims 41 to 43, wherein the first basal medium contains an anti-CD3 antibody, TGF-β, IL-2, retinoic acid, and a CDK8 inhibitor, a CDK19 inhibitor, or a CDK8/19 inhibitor.

45. The method according to any one of claims 41 to 44, wherein the second basal medium contains an anti-CD3 antibody, TGF-β, IL-2, retinoic acid, ascorbic acid, and a CDK8 inhibitor, a CDK19 inhibitor, or a CDK8/19 inhibitor.

46. The method according to any one of claims 41 to 45, wherein the step (a) includes stimulating the CD4-positive T cells or CD8-positive T cells in the first basal medium for about 3 days.

47. The method according to any one of claims 41 to 46, wherein the step (b) includes subjecting the cells obtained in the step (a) to a resting culture in a medium containing the IL-2 for at least about 2 days.

48. The method according to any one of claims 41 to 47, wherein the step (c) includes stimulating the cells obtained in the step (b) in the second basal medium for about 3 days.

49. The method according to any one of claims 41 to 48, wherein the step (d) includes subjecting the cells obtained in the step (c) to a resting culture in a medium containing the IL-2 for at least about 2 days.

50. An inducible regulatory human T cell or a cell population comprising the inducible regulatory human T cell generated by the method according to any one of claims 41 to 49.

51. A pharmaceutical composition for treating or preventing a T cell-related disease, the pharmaceutical composition comprising, as an active ingredient, an inducible regulatory human T cell having at least one characteristic selected from the group consisting of FoxP3-positive, CTLA4-positive, NT5E-positive, ITGAE (CD103)-positive, AREG-positive, CD172g-positive, and CD26-positive.

52. A pharmaceutical composition for treating or preventing a T cell-related disease, the pharmaceutical composition comprising, as an active ingredient, an NT5E-positive inducible regulatory human T cell.

53. A pharmaceutical composition for treating or preventing a T cell-related disease, the pharmaceutical composition comprising, as an active ingredient, an ITGAE (CD103)-positive inducible regulatory human T cell.

54. A pharmaceutical composition for treating or preventing a T cell-related disease, the pharmaceutical composition comprising, as an active ingredient, an AREG-positive inducible regulatory human T cell.

55. A pharmaceutical composition for treating or preventing a T cell-related disease, the pharmaceutical composition comprising, as an active ingredient, a CD172g-positive inducible regulatory human T cell.

56. A pharmaceutical composition for treating or preventing a T cell-related disease, the pharmaceutical composition comprising, as an active ingredient, a CD26-positive inducible regulatory human T cell.

57. A pharmaceutical composition for treating or preventing a T cell-related disease, the pharmaceutical composition comprising, as an active ingredient, a CTLA4-positive inducible regulatory human T cell.

58. A pharmaceutical composition for treating or preventing a T cell-related disease, the pharmaceutical composition comprising, as an active ingredient, a stably FoxP3-positive inducible regulatory human T cell.

59. The pharmaceutical composition according to any one of claims 51 to 58, wherein the inducible regulatory human T cell is at least CTLA4-positive and FoxP3-positive.

60. The pharmaceutical composition according to claim 59, wherein the FoxP3 is stably expressed.

61. The pharmaceutical composition according to claim 59, wherein the FoxP3 is continuously expressed for at least about 48 hours or longer.

62. The pharmaceutical composition according to claim 59, wherein the FoxP3 is stably expressed at least from day 7 of culture.

63. The pharmaceutical composition according to claim 59, wherein the FoxP3 is expressed even after freeze-thawing of the cell.

64. The pharmaceutical composition according to any one of claims 51 to 63, wherein the inducible regulatory human T cell is at least CD172g-positive and/or CD26-positive.

65. The pharmaceutical composition according to any one of claims 51 to 64, wherein the CNS2 region of the FOXP3 gene of the inducible regulatory human T cell is demethylated.

66. The pharmaceutical composition according to any one of claims 51 to 65, wherein the inducible regulatory human T cell is CD4-positive or CD8-positive.

67. The pharmaceutical composition according to any one of claims 51 to 66, wherein the inducible regulatory human T cell is obtained from or derived from a human peripheral blood T cell or a human tissue-derived T cell.

68. The pharmaceutical composition according to any one of claims 51 to 67, wherein the inducible regulatory human T cell is obtained by a method including:
a step (a) of stimulating CD4-positive T cells or CD8-positive T cells in human peripheral blood in a first basal medium for about 1 to about 5 days;
a step (b) of subjecting the cells obtained in the step (a) to a resting culture in a medium containing IL-2 for at least about 1 to about 3 days;
a step (c) of stimulating the cells obtained in the step (b) in a second basal medium for about 1 to about 5 days; and
a step (d) of subjecting the cells obtained in the step (c) to a resting culture in a medium containing IL-2 for at least about 1 to about 3 days.

69. The pharmaceutical composition according to any one of claims 51 to 68, wherein the pharmaceutical composition contains, as an active ingredient, a cell population containing the inducible regulatory human T cells, and the cell population has a proportion of about 50% or more of cells each having at least one characteristic selected from the group consisting of FoxP3-positive, CTLA4-positive, NT5E-positive, ITGAE (CD103)-positive, AREG-positive, CD172g-positive, and CD26-positive.

70. A pharmaceutical composition for treating or preventing a T cell-related disease, the pharmaceutical composition comprising, as an active ingredient, a cell population containing inducible regulatory human T cells, wherein the cell population has a proportion of about 50% or more of NT5E-positive cells.

71. A pharmaceutical composition for treating or preventing a T cell-related disease, the pharmaceutical composition comprising, as an active ingredient, a cell population containing inducible regulatory human T cells, wherein the cell population has a proportion of about 50% or more of ITGAE (CD103)-positive cells.

72. A pharmaceutical composition for treating or preventing a T cell-related disease, the pharmaceutical composition comprising, as an active ingredient, a cell population containing inducible regulatory human T cells, wherein the cell population has a proportion of about 50% or more of AREG-positive cells.

73. A pharmaceutical composition for treating or preventing a T cell-related disease, the pharmaceutical composition comprising, as an active ingredient, a cell population containing inducible regulatory human T cells, wherein the cell population has a proportion of about 50% or more of CD172g-positive cells.

74. A pharmaceutical composition for treating or preventing a T cell-related disease, the pharmaceutical composition comprising, as an active ingredient, a cell population containing inducible regulatory human T cells, wherein the cell population has a proportion of about 50% or more of CD26-positive cells.

75. A pharmaceutical composition for treating or preventing a T cell-related disease, the pharmaceutical composition comprising, as an active ingredient, a cell population containing inducible regulatory human T cells, wherein the cell population has a proportion of about 50% or more of CTLA4-positive cells.

76. A pharmaceutical composition for treating or preventing a T cell-related disease, the pharmaceutical composition comprising, as an active ingredient, a cell population containing inducible regulatory human T cells, wherein the cell population has a proportion of about 50% or more of stably FoxP3-positive cells.

77. The pharmaceutical composition according to any one of claims 69 to 76, wherein proportions of at least the CTLA4-positive cells and FoxP3-positive cells in the cell population are each about 50% or more.

78. The pharmaceutical composition according to claim 77, wherein the FoxP3 is stably expressed.

79. The pharmaceutical composition according to claim 77, wherein the FoxP3 is continuously expressed for at least about 48 hours or longer.

80. The pharmaceutical composition according to claim 77, wherein the FoxP3 is stably expressed at least from day 7 of culture.

81. The pharmaceutical composition according to claim 77, wherein the FoxP3 is expressed even after freeze-thawing of the cell.

82. The pharmaceutical composition according to any one of claims 69 to 80, wherein proportions of at least the CD172g-positive cells and/or CD26-positive cells in the cell population are each about 50% or more.

83. The pharmaceutical composition according to any one of claims 69 to 82, wherein a proportion of cells having the at least one characteristic in the cell population is about 60% or more.

84. The pharmaceutical composition according to any one of claims 69 to 83, wherein a proportion of cells having the at least one characteristic in the cell population is about 80% or more.

85. The pharmaceutical composition according to any one of claims 69 to 84, wherein a proportion of FoxP3 strong-positive cells in the cell population is about 50% or more.

86. The pharmaceutical composition according to claim 85, wherein a proportion of stably FoxP3 strong-positive cells in the cell population is about 50% or more.

87. The pharmaceutical composition according to any one of claims 51 to 86, wherein the cell population contains about 90% or more T cells.

88. The pharmaceutical composition according to any one of claims 51 to 87, wherein the inducible regulatory human T cells are contained so as to be administered at about 10⁸ to about 10⁹ cells per dose or about 10⁷ cells/kg.

89. The pharmaceutical composition according to any one of claims 51 to 88, wherein the T cell-related disease includes an autoimmune disease, an infectious disease, cancer, an allergy, an inflammatory disease, and ALS, which are treatable by an immunosuppressive action.

90. The pharmaceutical composition according to claim 89, wherein the autoimmune disease is selected from the group consisting of Addison's disease, alopecia areata, ankylosing spondylitis, autoimmune liver disease, autoimmune parotitis, Crohn's disease, diabetes (Type I), dystrophic epidermolysis bullosa, epididymitis, glomerulonephritis, Graves' disease, Guillain-Barré syndrome, Hashimoto's disease, hemolytic anemia, systemic lupus erythematosus, multiple sclerosis, myasthenia gravis, psoriasis, rheumatic fever, rheumatoid arthritis, sarcoidosis, scleroderma, Sjogren's syndrome, spondyloarthritis, thyroiditis, vasculitis, vitiligo, myxedema, pernicious anemia, cardiomyopathy, dilated cardiomyopathy (DCM), peripartum cardiomyopathy (PPCM), idiopathic cardiomyopathy, Chagas cardiomyopathy, Chagas megacolon, Chagas megaesophagus, Chagas neuropathy, benign prostatic hyperplasia, scleroderma, Raynaud's syndrome, preeclampsia, myocarditis, glaucoma, hypertension, pulmonary hypertension, malignant hypertension, pemphigus, Alzheimer's disease, systemic sclerosis, polymyositis, mixed connective tissue disease, antiphospholipid antibody syndrome, microscopic polyangiitis, granulomatosis with polyangiitis, eosinophilic granulomatosis with polyangiitis, crescentic glomerulonephritis, organ-specific autoimmune diseases, Basedow's disease, autoimmune hepatitis, primary biliary cholangitis, autoimmune pancreatitis, Goodpasture's syndrome, autoimmune hemolytic anemia, megaloblastic anemia, idiopathic thrombocytopenic purpura, primary sclerosing cholangitis, polyarteritis nodosa, Takayasu's arteritis, giant cell arteritis, polymyalgia rheumatica, adult-onset Still's disease, Behçet's disease, and ulcerative colitis.

91. The pharmaceutical composition according to any one of claims 51 to 90, wherein the pharmaceutical composition is administered by injection.

92. The pharmaceutical composition according to any one of claims 51 to 91, wherein when the pharmaceutical composition is administered to a patient and is ineffective or insufficient, the pharmaceutical composition is additionally administered to the patient.

93. The pharmaceutical composition according to claim 92, wherein the pharmaceutical composition is additionally administered at least about 2 weeks after the initial administration.
